# EUROPEAN PATENT SPECIFICATION

(11) **EP 2 352 521 B1**
(45) Date of publication and mention of the grant of the patent: **16.09.2020**
(21) Application number: 09793631.4
(22) Date of filing: 13.10.2009
(51) Int. Cl.: A61K 39/395, C07K 16/22, C07K 16/32

(54) **IMMUNOGLOBULIN VARIANTS AND USES THEREOF**
IMMUNGLOBULINVARIANTEN UND IHRE VERWENDUNG
VARIANTS D'IMMUNOGLOBULINE ET LEURS UTILISATIONS

(30) Priority: 14.10.2008 US 105086 P; 12.02.2009 US 152131 P; 22.04.2009 US 171768 P; 25.06.2009 US 220514 P
(43) Date of publication of application: 10.08.2011
(60) Divisional application: 18212252.3
(73) Proprietor: Genentech, Inc., South San Francisco, CA 94080 (US); F. Hoffmann-La Roche AG, 4070 Basel (CH)
(72) Inventor: DAMICO, Lisa A., San Carlos, California 94070 (US); FERRARA, Napoleone, San Francisco, California 94109 (US); LOWMAN, Henry B., El Granada, California 94018 (US); MENG, Yu-Ju G., Albany, California 94706 (US); YEUNG, Yik Andy, Pittsburg, California 94565 (US)
(74) Representative: Mewburn Ellis LLP
(86) International application number: PCT/US2009/060443
(87) International publication number: WO 2010/045193

(56) References cited:
- WO-A1-97/34631
- WO-A1-2009/086320
- WO-A2-00/42072
- WO-A2-02/060919
- WO-A2-2006/031370
- WO-A2-2006/053301
- WO-A2-2009/058492
- DALL' ACQUA WILLIAM F ET AL: "Increasing the affinity of a human IgG1 for the neonatal Fc receptor: Biological consequences" JOURNAL OF IMMUNOLOGY, AMERICAN ASSOCIATION OF IMMUNOLOGISTS, US, vol. 169, no. 9, 1 November 2002 (2002-11-01), pages 5171-5180, XP002268422 ISSN: 0022-1767
- YEUNG YIK ANDY ET AL: "Engineering Human IgG1 Affinity to Human Neonatal Fc Receptor: Impact of Affinity Improvement on Pharmacokinetics in Primates" JOURNAL OF IMMUNOLOGY, vol. 182, no. 12, June 2009 (2009-06), pages 7663-7671, XP002566420 ISSN: 0022-1767

## Description

### RELATED APPLICATIONS

This application is a non-provisional application filed under 37 CFR 1.53(b)(1), claiming priority under 35 USC 119(e) to provisional application number 61/105,086 filed October 14, 2008, provisional application number 61/152,131 filed February 12, 2009, provisional application number 61/171,768 filed April 22, 2009, and provisional application number 61/220,514 filed June 25, 2009.

### FIELD OF THE INVENTION

The present invention relates generally to the field of molecular biology. More specifically, the present invention relates to IgG immunoglobulin variants with altered biological properties and methods of using the same.

### BACKGROUND OF THE INVENTION

Over the years the use of immunoglobulins as therapeutic agents has increased dramatically. Immunoglobulin (Ig) molecules which constitute an important part of the immune system are of great interest because they (1) react with a diverse family of ligands, (2) possess different effector functions and (3) are of great biological importance. Today uses of antibody based drugs include treatment of cancer, autoimmune diseases as well as various systemic and infectious diseases. Also, immunoglobulins are useful as *in vivo* diagnostic tools, for example, in diagnostic imaging procedures.

IgG is the most prevalent immunoglobulin class in humans and other mammals and is utilized in various types of immunotherapies and diagnostic procedures. Human IgGi is the most commonly used antibody for therapeutic purposes. Currently many antibodies in clinical trials are directed against tumor associated antigens. In particular, anti-VEGF neutralizing antibodies have been shown to suppress the growth of a variety of human tumor cell lines in nude mice (Kim et al. Nature 362:841-844 (1993); Warren et al. J. Clin. Invest. 95:1789-1797 (1995); Borgstrom et al. Cancer Res. 56:4032-4039 (1996); and Melnyk et al. Cancer Res. 56:921-924 (1996)) and also inhibit intraocular angiogenesis in models of ischemic retinal disorders (Adamis et al. Arch. Ophthalmol. 114:66-71 (1996)). Indeed, a humanized anti-VEGF antibody, bevacizumab (AVASTIN®, Genentech, South San Francisco, CA) is the first U.S. FDA-approved therapy designed to inhibit angiogenesis.

Despite its potential, one of the problems with immunoglobulin immunotherapy has been the persistence of immunoglobulins in the circulation. The rate of immunoglobulin clearance directly affects the amount and frequency of dosage of the immunoglobulin. Increased dosage and frequency of dosage may cause adverse effects in the patient and also increase medical costs.

The mechanism of IgG catabolism in the circulation has been elucidated through studies related to the transfer of passive immunity from mother to fetus/neonate through the placenta or yolk sac or through colostrum (maternofetal transfer of IgG via transcytosis) in rodents (Brambell, Lancet, ii: 1087-1093, 1966; Rodewald, J. Cell Biol., 71:666-670, 1976; Morris et al., In: Antigen Absorption by the Gut, pp. 3-22, 1978, University Park Press, Baltimore; Jones et al., J. Clin. Invest., 51:2916-2927, 1972). The neonatal Fc receptor (FcRn) plays an important role in the transcytosis and homeostasis of IgG in mammals. FcRn is structurally homologous to major histocompatibility complex (MHC) class I molecules and consists of a transmembrane α chain and β₂-microglobulin (β2m). Previous studies in knockout mice illustrated that the serum half-life of IgG in FcRn- or β2m-deficient mice was greatly reduced (Roopenian et al., J Immunol 170(7), 3528-3533, 2003; Israel et al., Immunology 89(4), 573-578, 1996), demonstrating the protective role of FcRn in regulating the level of circulating IgG. Various site-specific mutagenesis experiments in the Fc region of mouse IgGs have led to identification of certain critical amino acid residues involved in the interaction between IgG and FcRn (Kim et al., Eur. J. Immunol., 24:2429-2434, 1994; Medesan et al., Eur. J. Immunol., 26:2533, 1996; Medesan et al., J. Immunol., 158:2211-2217, 1997). Additionally, various publications describe methods for obtaining physiologically active molecules whose half-lives are modified either by introducing or modifying an FcRn-binding region of the IgGs (WO 97/43316; U.S. Pat. No. 5,869,046; U.S. Pat. No. 5,747,035; WO 96/32478; WO2006053301; U.S. Pat. No. 7,083,784; U.S. Pat. No. 7,371,826).
At the molecular level, FcRn binds the Fc portion of IgG in the C_{H}2-C_{H}3 domain region. The Fc-FcRn interaction is highly pH dependent; IgGs bind FcRn with high affinity at pH 6, but as the pH is raised to 7.4, the binding affinity drops considerably. This pH dependent interaction is responsible for protecting IgG from degradation. Specifically, pinocytosed IgG is captured by FcRn in the acidic endosome, recycled back to the cell surface and then released back into the circulation at a physiological serum pH of 7.4 (Ober et al. Proc Natl Acad Sci U S A 101(30), 11076-11081, 2004; Ober et al. J Immunol 172(4), 2021-2029, 2004; Prabhat et al. Proc Natl Acad Sci U S A 104(14), 5889-5894, 2007). IgG that is not bound by FcRn is targeted to the lysosome and degraded. As FcRn is important in regulating IgG homeostasis, modulating the interaction between Fc and FcRn through protein engineering is one method for improving the pharmacokinetics of therapeutic antibodies (Shields et al. J Biol Chem 276(9), 6591-6604, 2001; Dall'Acqua et al. Nat Biotechnol 15(7), 637-640, 1997; Dall'Acqua et al., J Immunol 169(9), 5171-5180, 2002; Hinton et al. J Biol Chem 279(8), 6213-6216, 2004; Hinton et al. J Immunol 176(1), 346-356, 2006; Datta-Mannan et al. Drug Metab Dispos 35(1), 86-94, 2007; Datta-Mannan et al. J Biol Chem 282(3), 1709-1717, 2007). A number of studies in mice, rhesus and cynomolgus monkeys have demonstrated that increasing the pH-6 binding affinity of IgGs can prolong half-life (Dall'Acqua et al. Nat Biotechnol 15(7), 637-640, 1997; Dall'Acqua et al., J Immunol 169(9), 5171-5180, 2002; Hinton et al. J Biol Chem 279(8), 6213-6216, 2004; Hinton et al. J Immunol 176(1), 346-356, 2006). Furthermore, other studies have also demonstrated that FcRn binding affinity at pH 7.4 is an additional determinant of IgG pharmacokinetics. Specifically, certain variants with increased pH-7.4 binding affinity to mouse FcRn exhibited increased clearance (*i.e.*, decreased half-life) in mice (Dall'Acqua et al., J Immunol 169(9), 5171-5180, 2002). Nevertheless, the detailed relationship between FcRn affinity and half-life has not been elucidated, as all of the previous studies involved a small number of variants, within a limited range of FcRn affinities. The maximal half-life extension achievable through engineering the Fc:FcRn interaction is unclear. WO2006/031370 discloses polypeptides having IgG Fc regions with amino acid modifications that result in the polypeptides exhibiting altered Fc effector functions.

Despite the fact that adherence to (compliance with) drug treatment is important, it is estimated that half of those for whom medicines are prescribed do not take them in the recommended way. For example, a recent research showed that as many as one-third of women taking breast cancer drugs developed in the past 10 years do not complete their recommended five-year course. Some of the causes for poor compliance include forgetfulness, physical difficulty in complying (*e.g.* traveling to or moving away from place of treatment), inconvenience, adverse side effect, complicated regimen and cost of drugs. Poor adherence to drug treatment can lead to achieving less than the full health benefits medicines can provide to patients. For example, not completing the recommended course of cancer treatment could lead to a recurrence of the disease and a reduced chance of survival.

Strategies to improve drug compliance include making it more convenient for patients to finish the recommended course of treatment. One of the ways this may be accomplished for patients under immunotherapy treatment is by increasing the duration of time that immunoglobulins are in circulation. The rate of immunoglobulin clearance directly affects the amount and frequency of dosage of the immunoglobulin. Therefore, developing an immunoglobulin that confers increased *in vivo* half-life may decrease the amount and/or frequency of dosage, thus minimizing the inconvenience as well as any additional medical costs.

Accordingly, it would be highly advantageous to have modified immunoglobulins that confer increased *in vivo* half-life for therapeutic purposes. The present invention addresses these and other needs, as will be apparent upon review of the following disclosure.

### SUMMARY OF THE INVENTION

The invention provides novel IgG variants and uses as described in the appended claims. Additional IgG variants are described herein.. For example, IgG variants comprising a human IgG Fc region as disclosed herein can comprise two or more amino acid substitutions relative to a wild-type human IgG Fc region at two or more of amino acid residues 251, 252, 307, 308, 378, 428, 430, 434, and 436, numbered according to the EU index as in Kabat, wherein the variant IgG has an increased half-life compared to the half-life of an IgG having the wild-type human IgG Fc region, and wherein at least two of the amino acid substitutions are at amino acid residue 251, 252, 307, 308, 378, 428, 430, 434, or 436, and an amino acid substitution at amino acid residue 251 is a substitution with aspartic acid or glutamic acid, an amino acid substitution at amino acid residue 252 is a substitution with tyrosine, an amino acid substitution at amino acid residue 307 is a substitution with glutamine, an amino acid substitution at amino acid residue 308 is a substitution with proline, an amino acid substitution at amino acid residue 378 is a substitution with valine, an amino acid substitution at amino acid residue 428 is a substitution with leucine, an amino acid substitution at amino acid residue 430 is a substitution with alanine or lysine, an amino acid substitution at amino acid residue 434 is a substitution with alanine, serine or tyrosine, and an amino acid substitution at amino acid residue 436 is a substitution with isoleucine.

For example, the human IgG Fc region as disclosed herein can comprise: an amino acid substitution at amino acid 251, wherein the amino acid substitution at amino acid 251 is the substitution with aspartic acid or glutamic acid; an amino acid substitution at amino acid 307, wherein the amino acid substitution at amino acid 307 is the substitution with glutamine; an amino acid substitution at amino acid 308, wherein the amino acid substitution at amino acid 308 is the substitution withproline, an amino acid substitution at amino acid 378, wherein the amino acid substitution at amino acid 378 is the substitution valine or an amino acid substitution at amino acid 436, wherein the amino acid substitution at amino acid 436 is the substitution with isoleucine. The variant IgG can have a higher binding affinity for FcRn than the IgG having the wild-type human IgG Fc region.

Alternatively, the human IgG Fc region as disclosed herein can comprise: the amino acid substitution at amino acid 307 with glutamine and the amino acid substitution at amino acid 434 with alanine; the amino acid substitution at amino acid 307 with glutamine and the amino acid substitution at amino acid 434 with serine; the amino acid substitution at amino acid 308 with proline and the amino acid substitution at amino acid 434 with alanine; the amino acid substitution at amino acid 252 with tyrosine and the amino acid substitution at amino acid 434 with alanine; the amino acid substitution at amino acid 378 with valine and the amino acid substitution at amino acid 434 with alanine; the amino acid substitution at amino acid 428 with leucine and the amino acid substitution at amino acid 434 withalanine; the amino acid substitution at amino acid 434 with alanine and the amino acid substitution at amino acid 436 with isoleucine, the amino acid substitution at amino acid 308 with proline and the amino acid substitution at amino acid 434 with tyrosine or the amino acid substitution at amino acid 307 with glutamine and the amino acid substitution at amino acid 436 with isoleucine.

Further IgG variants as disclosed herein can comprise a human IgG Fc region comprising three or more amino acid substitutions relative to a wild-type human IgG Fc region at three or more of amino acid residues 251, 252, 307, 308, 378, 380, 428, 430, 434, and 436, numbered according to the EU index as in Kabat, wherein the variant IgG has an increased half-life compared to the half-life of an IgG having the wild-type human IgG Fc region, and wherein at least three of the amino acid substitutions are at amino acid residue 251, 252, 307, 308, 378, 380, 428, 430, 434, or 436, and an amino acid substitution at amino acid residue 251 is a substitution with aspartic acid or glutamic acid, an amino acid substitution at amino acid residue 252 is a substitution with tyrosine, an amino acid substitution at amino acid residue 307 is a substitution with glutamine, an amino acid substitution at amino acid residue 308 is a substitution with proline, an amino acid substitution at amino acid residue 378 is a substitution with valine, an amino acid substitution at amino acid residue 380 is a substitution with alanine, an amino acid substitution at amino acid residue 428 is a substitution with leucine, an amino acid substitution at amino acid residue 430 is a substitution with alanine or lysine, an amino acid substitution at amino acid residue 434 is a substitution with alanine, serine, tyrosine or histidine, and an amino acid substitution at amino acid residue 436 is a substitution with isoleucine.

Alternatively, the human IgG Fc region as disclosed herein can comprise: an amino acid substitution at amino acid 251, wherein the amino acid substitution at amino acid 251 is the substitution with aspartic acid or glutamic acid; an amino acid substitution at amino acid 307, wherein the amino acid substitution at amino acid 307 is the substitution with glutamine, an amino acid substitution at amino acid 308, wherein the amino acid substitution at amino acid 308 is the substitution with proline; an amino acid substitution at amino acid 378, wherein the amino acid substitution at amino acid 378 is the substitution with valine or, an amino acid substitution at amino acid 436, wherein the amino acid substitution at amino acid 436 is the substitution with isoleucine. The variant can have a higher binding affinity to FcRn compared to the IgG having the wild-type human IgG Fc region.

As disclosed herein the human IgG Fc region can comprise: the amino acid substitution at amino acid 307 with glutamine, the amino acid substitution at amino acid 380 with alanine and the amino acid substitution at amino acid 434 with serine; the amino acid substitution at amino acid 307 with glutamine, the amino acid substitution at amino acid 380 with alanine and the amino acid substitution at amino acid 434 with analine; the amino acid substitution at amino acid 252 with tyrosine, the amino acid substitution at amino acid 308 with proline and the amino acid substitution at amino acid 434 with tyrosine or the amino acid substitution at amino acid 251 with aspartic acid, the amino acid substitution at amino acid 307 with glutamine and the amino acid substitution at amino acid 434 with histidine.

Alternatively, the variant IgGs or fragments thereof as disclosed herein can further comprise an amino acid substitution at position 297 to alanine.

The variant IgGs as disclosed herein can have a higher binding affinity for FcRn than the IgG having the wild-type human IgG Fc region. For example, a variant IgG can have a higher binding affinity for FcRn at pH 6.0 than at pH 7.4. The variant igG can also be a human or humanized IgG or can be IgG₁, IgG₂, IgG₃ or IgG₄. For example, the IgG Fc region of the variant IgG can be an IgG₁, IgG₂, IgG₃ or IgG₄ Fc region. In certain embodiments, the IgG Fc region of the variant IgG is an IgG₁ Fc region.

In certain embodiments, the variant IgG is an anti-VEGF antibody. In certain embodiments, the variant IgG is a variant of bevacizumab. In certain embodiments, the IgG having the wild-type human IgG Fc region is bevacizumab. In certain embodiments, the wild-type human IgG Fc region is the Fc region of bevacizumab. In certain embodiments, the variant IgG comprises the heavy chain variable domain (SEQ ID NO: 1) and light chain variable domain (SEQ ID NO:2). The variant IgG as disclosed herein can comprise the heavy chain variable domain (SEQ ID NO:3) and light chain variable domain (SEQ ID or alternatively, it can comprise the heavy chain variable domain (SEQ ID NO:7) and light chain variable domain (SEQ ID NO:8).

The present invention further discloses pharmaceutical compositions comprising any of the variant IgGs as claimed and a pharmaceutically acceptable carrier. A kit comprising any of the variant IgGs described herein, in a container, and instructions for use is also provided herein.

As disclosed herein, the half life of the variant IgG is increased by at least 50%, 100%, 150%, 200%, 300% or greater compared to an IgG having the wild-type human IgG Fc region. For example, the half life of the variant IgG is increased by at least 2 fold compared to an IgG having the wild-type human IgG Fc region; by at least 3 fold compared to an IgG having the wild-type human IgG Fc region or by at least 4 fold compared to an IgG having the wild-type human IgG Fc region. The wild-type human IgG Fc region can be bevacizumab and the half life of the variant IgG can be the mean half-life of bevacizumab. For example, the mean half-life of bevacizumab can be about 10 to 12 days as measured in cynomolgus monkeys, or about 3 weeks as measured in humans.

Variant IgGs as set out in the appended claims comprise a human IgG1 Fc region which has the amino acid substitutions relative to a wild-type human IgG Fc region at amino acid residues 307 and 434, numbered according to the EU index as in Kabat, wherein the variant IgG has an increased half-life compared to the half-life of an IgG having the wild-type human IgG Fc region, and wherein the amino acid substitution at amino acid residue 307 is a substitution with glutamine, and the amino acid substitution at amino acid residue 434 is a substitution with alanine. In one embodiment, the variant IgG is variant IgG₁ comprising the heavy chain variable domain (SEQ ID NO: 1) and light chain variable domain (SEQ ID NO:2), and comprising a human IgGi Fc region comprising amino acid substitutions relative to a wild-type human IgG₁ Fc region at amino acid residues 307 and 434, numbered according to the EU index as in Kabat, wherein the variant IgG₁ has an increased half-life compared to the half-life of an IgG₁ having the wild-type human IgG₁ Fc region, and wherein the amino acid substitution at amino acid residue 307 is a substitution with glutamine, and the amino acid substitution at amino acid residue 434 is a substitution with alanine.

In another embodiment, variant IgGs comprising a human IgG Fc region are provided, as set out in the claims wherein the human IgG Fc region comprises amino acid substitutions relative to a wild-type human IgG Fc region at amino acid residues 307, 380 and 434, numbered according to the EU index as in Kabat, wherein the variant IgG has an increased half-life compared to the half-life of an IgG having the wild-type human IgG Fc region, and wherein the amino acid substitution at amino acid residue 307 is a substitution with glutamine, the amino acid substitution at amino acid residue 380 is a substitution with alanine, and the amino acid substitution at amino acid residue 434 is a substitution with serine.

In certain embodiments, the variant IgG comprising a human IgG Fc region described above has a higher binding affinity for FcRn than the IgG having the wild-type human IgG Fc region. In certain embodiments, the variant IgG has a higher binding affinity for FcRn at pH 6.0 than at pH 7.4. In certain embodiments, the variant IgG is a human or humanized IgG. In certain embodiments, the variant IgG is an anti-VEGF antibody. In certain embodiments, the variant IgG is a variant of bevacizumab. In certain embodiments, the IgG having the wild-type human IgG Fc region is bevacizumab. In certain embodiments, the wild-type human IgG Fc region is the Fc region of bevacizumab. In certain embodiments, the variant IgG comprises the heavy chain variable domain (SEQ ID NO:1) and light chain variable domain (SEQ ID NO:2). In certain embodiments, a pharmaceutical composition comprising any of the variant IgGs comprising a human IgG Fc region and a pharmaceutically acceptable carrier are provided herein. A kit comprising any of the variant IgGs comprising a human IgG Fc region, in a container, and instructions for use is also provided herein.

Further variant IgGs disclosed herein comprise human IgG₁ Fc region, wherein the variant IgGs comprise the heavy chain variable domain (SEQ ID NO: 1) and light chain variable domain (SEQ ID NO:2) and wherein the human IgGi Fc region comprises an amino acid substitution relative to a wild-type human IgG₁ Fc region at amino acid residue 434, numbered according to the EU index as in Kabat, wherein the variant IgG has an increased half-life compared to the half-life of an IgG having the wild-type human IgG₁ Fc region, and the variant IgG has a higher binding affinity for FcRn compared to binding affinity for FcRn of the IgG having the wild-type human IgG₁ Fc region, and wherein the amino acid substitution at amino acid residue 434 is a substitution with histidine. The variant IgG as disclosed herein can be variant IgGi.

As disclosed herein, the half like of a variant can be increased by at least 50, 55, 60, 65, 70, 75, 80, 85, 90, 95, or 100% compared to the half life of the IgG having the wild-type human IgG Fc region.

The half life of a variant IgG as disclosed herein can also be at least about 15, 20, 25, 30, 35, or 40 days. The half life of a variant IgG can be the half life as measured inhumans or in cynomolgus monkeys. In certain embodiments, the wild-type IgG or the IgG having the wild-type human IgG Fc region is bevacizumab. The half-life of bevacizumab can be 10 to 12 days as measured in cynomolgus monkeys, or about 20 days as measured in humans.

A number of methods of using IgG variants are provided in the invention. Methods of treating tumor in a subject are provided. For example, methods comprise administering to the subject an effective amount of any variant IgGs described above and herein. In certain embodiments, the variant IgG is an anti-VEGF antibody. In certain embodiments, the variant IgG is a variant of bevacizumab. These methods can further comprise administering to the subject an effective amount of a chemotherapeutic agent.

Methods of inhibiting VEGF activity in a subject are provided in the invention. For example, methods comprise administering to said subject an effective amount of a variant IgG anti-VEGF antibody as described above and herein. In certain embodiments, the VEGF activity is angiogenesis.

Methods of modulating vascular permeability in a subject are also disclosed herein. For example, methods comprise administering to said subject an effective amount of any variant IgGs described above and herein.

Methods of treating a non-neoplastic disorder in a subject are also disclosed herein. For example, methods comprise administering to said subject an effective amount of any variant IgGs described above and herein. For example, the non-neoplastic disorder is an autoimmune disease or is Alzheimer's disease. Alternatively, the subject is diagnosed with age-related macular degeneration.

These methods also include treating a HER expressing tumor in a subject. For example, these methods comprise administering to said subject an effective amount of any variant IgGs described above and herein, such as the variant IgG comprising the heavy chain variable domain (SEQ ID NO:7) and light chain variable domain (SEQ ID NO:8).

Methods of inhibiting or preventing growth of cancer cells in a subject are also disclosed herein. For example, these methods comprise administering to said subject an effective amount of any variant IgGs described above and herein.

Methods of administering to a subject an effective amount of a variant IgG are also disclosed herein. These methods of administration can be used in combination with other methods (*e.g.*, methods of treatments) described herein. For example, methods comprise administering to said subject an effective amount of any variant IgGs described above and herein, and wherein the variant IgG is administered to the subject every 4 weeks or at longer intervals; every 5 weeks or longer; every 6 weeks or longer; every 7 weeks or longer; every 8 weeks or longer; every 9 weeks or longer; every 10 weeks or longer; every 11 weeks or longer; every 12 weeks or longer. Other methods can also comprise administering to said subject an effective amount of any variant IgGs, wherein the variant IgG is administered less frequently than the recommended or prescribed dosage frequency of the IgG having the wild-type human IgG Fc region, the IgG having the wild-type human IgG Fc region can be bevacizumab. The variant IgG, *e.g.*, a variant of bevacizumab, can be administered less frequently than the prescribed dosage frequency of bevacizumab.

The variant IgG can be initially administered every 2 weeks, and later administered every 4 weeks or longer; every 3 weeks, and later administered every 6 weeks or longer; initially administered every 4 weeks, and later administered every 8 weeks or longer; initially administered every 5 weeks, and later administered every 10 weeks or longer; initially administered every 6 weeks, and later administered every 12 weeks or longer or the variant IgG, *e.g.*, a variant of bevacizumab, is initially administered with the prescribed dosage frequency of bevacizumab, and later administered less frequently than the prescribed dosage of bevacizumab.

In accordance with the methods described herein, the variant IgG is an anti-VEGF antibody; an anti-VEGF antibody comprising the heavy chain variable domain (SEQ ID NO:1) and light chain variable domain (SEQ ID NO:2 or the anti-VEGF antibody is a variant of bevacizumab.

The variant IgGs can be administered to the subject intravenously or subcutaneously.

In certain embodiments of the methods described herein, the subject is human. In certain embodiments, the subject is diagnosed with cancer. For example, the cancer is selected from the group consisting of non-small cell lung cancer, renal cell carcinoma, ovarian cancer, glioblastoma, breast cancer, and colorectal cancer.

Also disclosed herein are methods of treating a benign, pre-cancerous or non-metastatic cancer in a subject, which comprise administering to the subject an effective amount of a variant IgG. For example, the administration of the variant IgG can prevent the benign, pre-cancerous, or non-metastatic cancer from becoming an invasive or metastatic cancer. The benign, pre-cancerous or non-metastatic cancer can be a stage 0, stage I, or stage II cancer, and for example, the administration of the variant IgG can prevent the benign, pre-cancerous or non-metastatic cancer from progressing to the next stage(s), *e.g.*, a stage I, a stage II, a stage III or stage IV cancer.

Alternatively, the variant IgG can be administered for a time and in an amount sufficient to treat the benign, pre-cancerous, or non-metastatic tumor in the subject or to prevent the benign, pre-cancerous, or non-metastatic tumor from becoming an invasive or metastatic cancer. Alternatively, administering the variant IgG can reduce tumor size, tumor burden, or the tumor number of the benign, pre-cancerous, or non-metastatic tumor. The variant IgG can also be administered in an amount and for a time to decrease the vascular density in the benign, pre-cancerous, or non-metastatic tumor.

As described herein, the methods disclosed herein can be used to treat, *e.g.*, a stage 0 (*e.g.*, a carcinoma in situ), stage I, or stage II cancer. The methods of neoadjuvant and adjuvant therapy can be used to treat any type of cancer, *e.g.*, benign or malignant. For example, the cancer can be a solid tumor, including, but not limited to, colon cancer, breast cancer, prostate cancer, renal cancer, lung cancer (*e.g.*, non-small cell lung cancer), melanoma, ovarian cancer, pancreatic cancer, gastrointestinal cancer, head and neck cancer, liver cancer and soft tissue cancers (*e.g.*, B cell lymphomas such as NHL and multiple myeloma and leukemias such as chronic lymphocytic leukemia). For example, the benign, pre-cancerous, or non-metastatic tumor is a polyp, adenoma, fibroma, lipoma, gastrinoma, insulinoma, chondroma, osteoma, hemangioma, lymphangioma, meningioma, leiomyoma, rhabdomyoma, squamous cell papilloma, acoustic neuromas, neurofibroma, bile duct cystanoma, leiomyomas, mesotheliomas, teratomas, myxomas, trachomas, granulomas, hamartoma, transitional cell papilloma, pleiomorphic adenoma of the salivary gland, desmoid tumor, dermoid cystpapilloma, cystadenoma, focal nodular hyperplasia, or a nodular regenerative hyperplasia. Alternatively, the method is desirably used to treat an adenoma. Non-limiting examples of adenomas include liver cell adenoma, renal adenoma, metanephric adenoma, bronchial adenoma, alveolar adenoma, adrenal adenoma, pituitary adenoma, parathyroid adenoma, pancreatic adenoma, salivary gland adenoma, hepatocellular adenoma, gastrointestinal adenoma, tubular adenoma, and bile duct adenoma.

The methods disclosed herein comprise administering to a subject an effective amount of a variant IgG to prevent occurrence or recurrence of a benign, pre-cancerous, or non-metastatic cancer in the subject. For example, the subject can be at risk for cancer, polyps, or a cancer syndrome. For example, the subject can have a family history of cancer, polyps, or an inherited cancer syndrome; the subject is at risk of developing a benign, pre-cancerous, or non-metastatic tumor or the method prevents occurrence or recurrence of said benign, pre-cancerous or non-metastatic cancer in a subject who has never had a tumor, a subject who has never had a clinically detectable cancer, or a subject who has only had a benign tumor.

Alternatively, these methods of preventing or reducing the likelihood of recurrence of a cancer in a subject include administering to the subject a variant IgG for a time and in an amount sufficient to prevent or reduce the likelihood of cancer recurrence in the subject. These methods also include a method of preventing the recurrence of a cancer in a subject having a tumor that includes the steps of removing the tumor (*e.g.*, using definitive surgery) and thereafter administering to the subject a variant IgG. Alternatively, they include methods of preventing the regrowth of a tumor in a subject that includes the steps of removing the tumor (*e.g.*, using definitive surgery) and thereafter administering to the subject a variant IgG or they include a method of preventing recurrence of cancer in a subject or reducing the likelihood of cancer recurrence in a subject that optionally includes administering to the subject an effective amount of a variant IgG prior to surgery, performing definitive surgery, and administering an effective amount of a variant IgG following the surgery wherein the administration of the variant IgG after the surgery prevents recurrence of the cancer or reduces the likelihood of cancer recurrence. Another alternative includes a method of preventing recurrence of cancer in a subject or reducing the likelihood of cancer recurrence in a subject that includes administering to the subject an effective amount of a variant IgG in the absence of any additional anti-cancer therapeutic agent, wherein the administering prevents recurrence of cancer in a subject or reduces the likelihood of cancer recurrence in a subject.

For each of the methods described above, the tumor can be any type of tumor including but not limited to the solid tumors, and particularly the tumors and adenomas, described herein. The subject can have a dormant tumor or micrometastases, which may or may not be clinically detectable; the variant IgG is administered for a time and in an amount sufficient to reduce neovascularization of a dormant tumor or micrometastases or the variant IgG can be administered for a time and in an amount sufficient to prevent occurrence of a clinically detectable tumor, or metastasis thereof, or to increase the duration of survival of the subject.

The variant IgG can be a monotherapy. The subject may have been previously treated for the tumor, for example, using an anti-cancer therapy. In one example, the anti-cancer therapy can be surgery. In another example, the subject can be further treated with an additional anti-cancer therapy before, during (*e.g.*, simultaneously), or after administration of the variant IgG. Examples of anti-cancer therapies include, without limitation, surgery, radiation therapy (radiotherapy), biotherapy, immunotherapy, chemotherapy, or a combination of these therapies.

In examples where the subject has undergone definitive surgery, the variant IgG can be generally administered after a period of time in which the subject has recovered from the surgery. This period of time can include the period required for wound healing or healing of the surgical incision, the time period required to reduce the risk of wound dehiscence, or the time period required for the subject to return to a level of health essentially similar to or better than the level of health prior to the surgery. The period between the completion of the definitive surgery and the first administration of the variant IgG can also include the period needed for a drug holiday, wherein the subject requires or requests a period of time between therapeutic regimes. Generally, the time period between completion of definitive surgery and the commencement of the variant IgG therapy can include less than one week, 1 week, 2 weeks, 3 weeks, 4 weeks (28 days), 5 weeks, 6 weeks, 7 weeks, 8 weeks, 3 months, 4 months, 5 months, 6 months, 7 months, 8 months, 9 months, 10 months, 11 months, 1 year, 2 years, 3 years, or more. In one embodiment, the period of time between definitive surgery and administering the variant IgG is greater than 2 weeks and less than 1 year. For example, the period of time between definitive surgery and administering the variant IgG is greater than 4 weeks (28 days).

Each of the above methods can further include monitoring the subject for recurrence of the cancer.

Methods of neoadjuvant therapy prior to the surgical removal of operable cancer in a subject, *e.g.*, a human patient, comprising administering to the patient an effective amount of a variant IgG where the patient has been diagnosed with a tumor or cancer are also disclosed. The variant IgG can be administered alone or in combination with at least one chemotherapeutic agent.

Another method includes a method of treating a subject with operable cancer that includes administering to the subject an effective amount of a variant IgG prior to surgery and thereafter performing surgery whereby the cancer is resected. Alternatively, the method can further include the step of administering to the subject an effective amount of a variant IgG after surgery to prevent recurrence of the cancer or it concerns a method of neoadjuvant therapy comprising administering to a subject with operable cancer an effective amount of a variant IgG and at least one chemotherapeutic agent prior to definitive surgery.

For example, these methods include a method of reducing tumor size in a subject having an unresectable tumor comprising administering to the subject an effective amount of a variant IgG wherein the administering reduces the tumor size thereby allowing complete resection of the tumor, or alternatively, the method further includes administering to the subject an effective amount of a variant IgG after complete resection of the tumor.

Alternatively, these methods concern a method of treating cancer in a subject comprising the following steps: a) a first stage comprising a plurality of treatment cycles wherein each cycle comprises administering to the subject an effective amount of a variant IgG and at least one chemotherapeutic agent at a predetermined interval; b) a definitive surgery whereby the cancer is removed; and c) a second stage comprising a plurality of maintenance cycles wherein each cycle comprises administering to the subject an effective amount of a variant IgG without any chemotherapeutic agent at a predetermined interval. For example, the first stage may a first plurality of treatment cycles wherein a variant IgG and a first chemotherapy regimen are administered followed by a second plurality of treatment cycles wherein a variant IgG and a second chemotherapy regimen are administered.

The disclosure provides methods comprising administering to a subject with metastatic or nonmetastatic cancer, following definitive surgery, an effective amount of a variant IgG. For example, the method may further include the use of at least one chemotherapeutic agent.

For example, the method can comprise the following steps: a) a first stage comprising a plurality of treatment cycles wherein each cycle comprises administering to the subject an effective amount of a variant IgG and at least one chemotherapeutic agent at a predetermined interval; and b) a second stage comprising a plurality of maintenance cycles wherein each cycle comprises administering to the subject an effective amount of a variant IgG without any chemotherapeutic agent at a predetermined interval. Alternatively, the first stage comprises a first plurality of treatment cycles wherein a variant IgG and a first chemotherapy regimen are administered, followed by a second plurality of treatment cycles wherein a variant IgG and a second chemotherapy regimen are administered.

In certain embodiments, the variant IgG is an anti-VEGF antibody that binds to VEGF or reduces VEGF expression or biological activity. The anti-VEGF antibody, or antigen-binding fragment thereof, can be a monoclonal antibody, a chimeric antibody, a fully human antibody, or a humanized antibody. Although the subject can be treated in a number of different ways prior to, during, or after the administration of the variant IgG. The subject may also be treated without surgery or chemotherapy. The treatment with the variant IgG may follow a monotherapy or a monotherapy for the duration of the variant IgG treatment period, as assessed by the clinician or described herein.

Treatment with the variant IgG in combination with an additional anti-cancer therapy, may include but not limited to, surgery, radiation therapy, chemotherapy, differentiating therapy, biotherapy, immune therapy, an angiogenesis inhibitor, and an anti-proliferative compound. Treatment with the variant IgG can also include any combination of the above types of therapeutic regimens. In certain embodiments, cytotoxic agents, anti-angiogenic and anti-proliferative agents can be used in combination with the variant IgG. The anti-cancer therapy can be chemotherapy and the chemotherapeutic agent and the variant IgG can be administered concurrently.

As described herein, the methods disclosed herein may be advantageous in treating and preventing early stage tumors, thereby preventing progression to the more advanced stages resulting in a reduction in the morbidity and mortality associated with advanced cancer. These methods are also advantageous in preventing the recurrence of a tumor or the regrowth of a tumor, for example, a dormant tumor that persists after removal of the primary tumor, or in reducing or preventing the occurrence or proliferation of micrometastases.

For these methods, the cancer may be a solid tumor, *e.g.*, such as, breast cancer, colorectal cancer, rectal cancer, lung cancer, renal cell cancer, a glioma (*e.g.*, anaplastic astrocytoma, anaplastic oligoastrocytoma, anaplastic oligodendroglioma, glioblastoma multiforme), kidney cancer, prostate cancer, liver cancer, pancreatic cancer, soft-tissue sarcoma, carcinoid carcinoma, head and neck cancer, melanoma, and ovarian cancer.

These methods can also include monitoring the subject for recurrence of the cancer or tumor.

Other features and advantages of the invention will be apparent from the following Detailed Description, the drawing. The invention is set out in the claims.

### BRIEF DESCRIPTION OF THE FIGURES

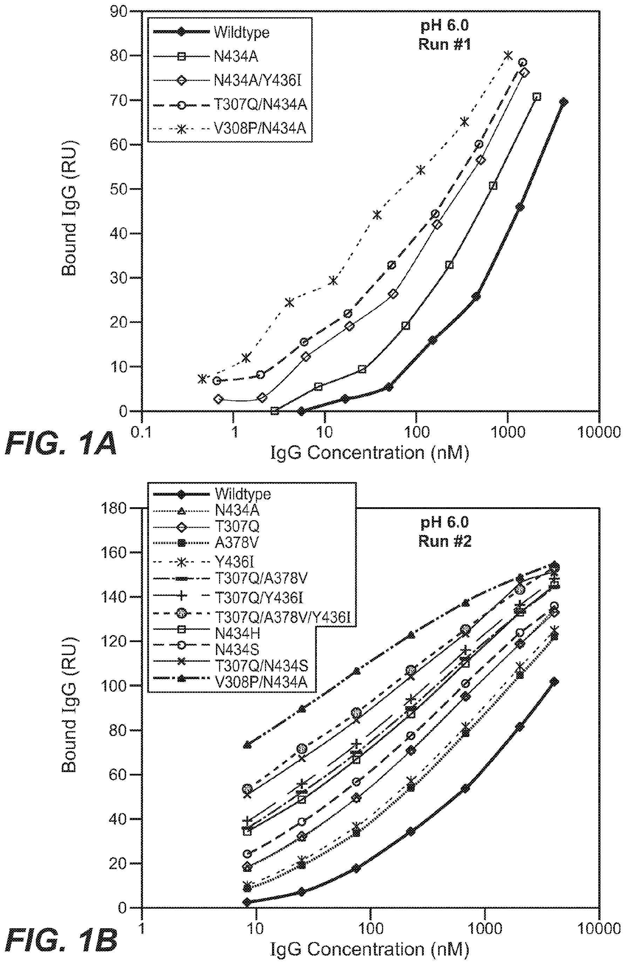

**Fig. 1** Panels A-B: Binding of anti-VEGF wild-type (WT) and anti-VEGF variants to human FcRn at pH 6.0. Two separate experimental runs with different levels of FcRn coupled on the chips were performed. For each run, steady state response unit is plotted as a function of variant concentrations to estimate the dissociation constants.

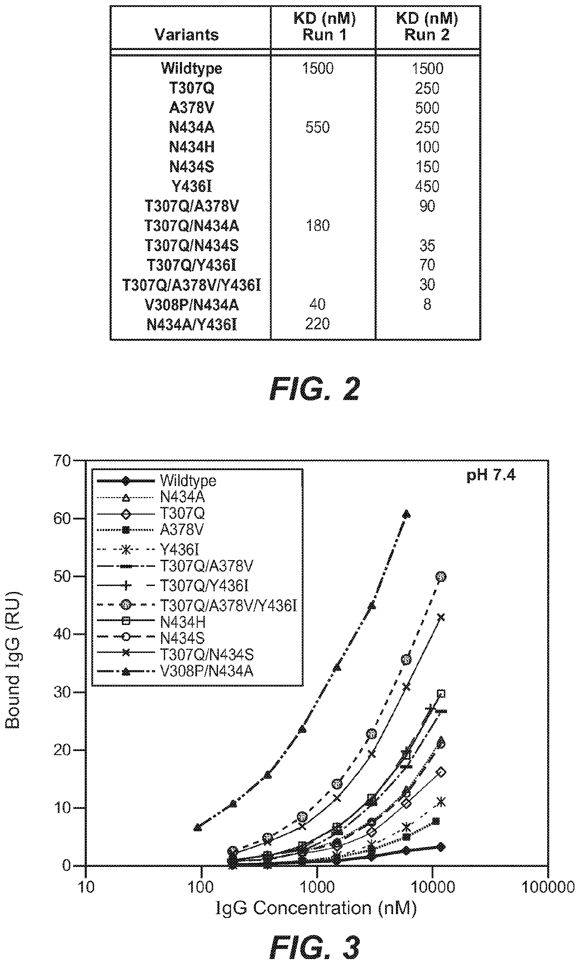

**Fig. 2****:** Dissociation constants of the anti-VEGF wild-type (WT) and anti-VEGF variants against human FcRn at pH 6.0. K_{D} was estimated from the two different runs shown in Figure 1.
**Fig. 3****:** Binding of anti-VEGF wild-type (WT) and anti-VEGF variants to human FcRn at pH 7.4. Steady state response unit is plotted as a function of anti-VEGF variant concentrations.

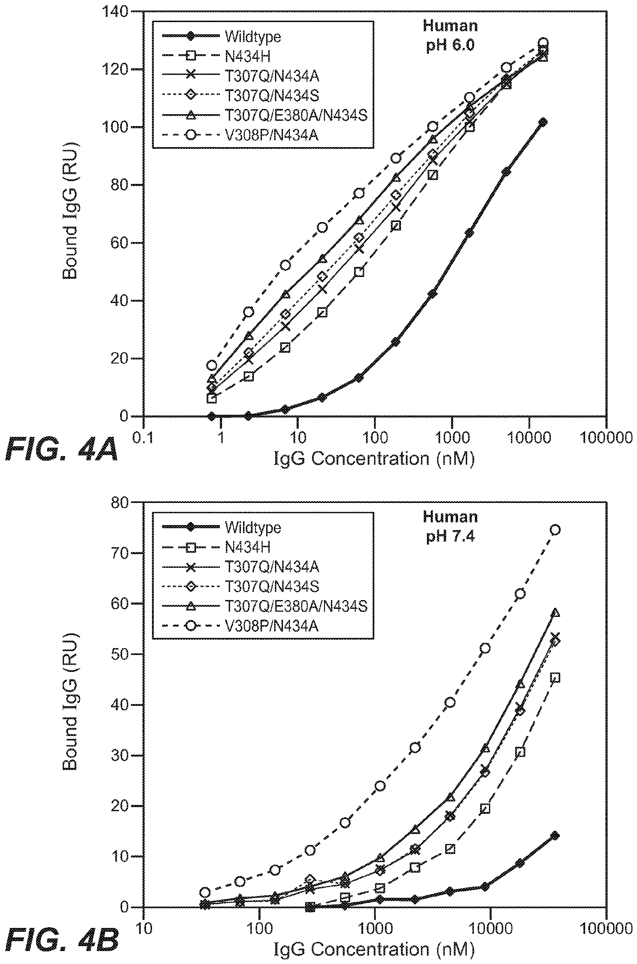

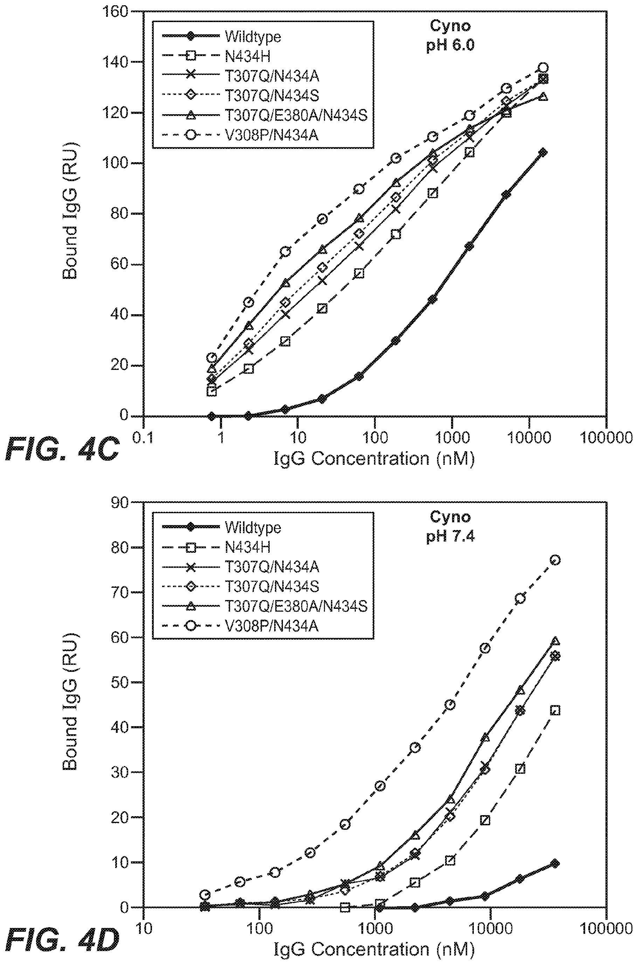

**Fig. 4** Panels A-D: Binding of anti-VEGF wild-type and anti-VEGF variants to (A) human FcRn at pH 6.0, (B) human FcRn at pH 7.4, (C) cyno FcRn at pH 6.0 and (D) cyno FcRn at pH 7.4.

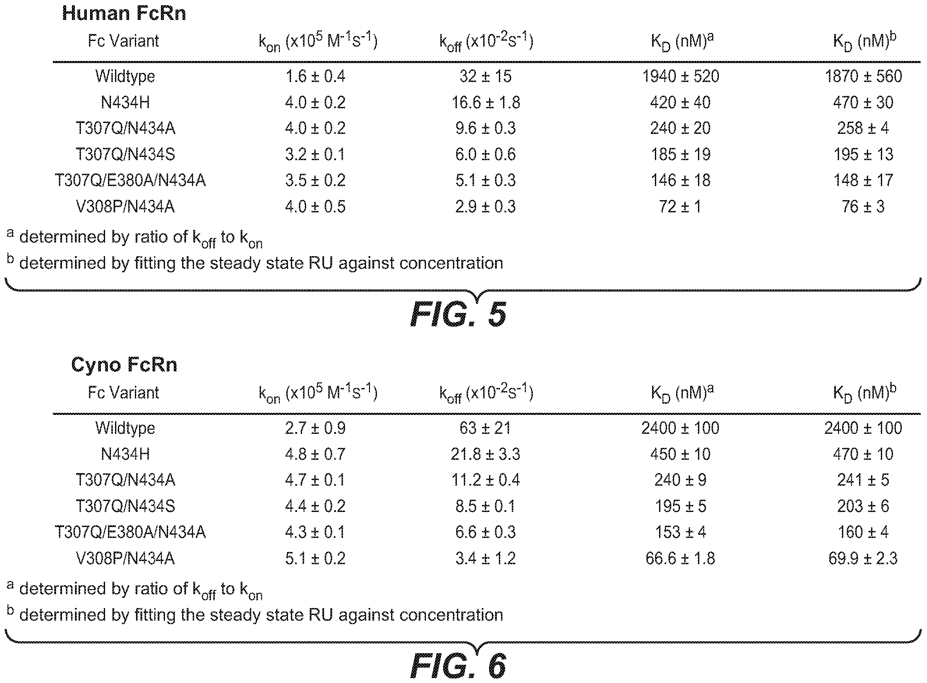

**Fig. 5****:** Kinetics parameters and monovalent dissociation constants (K_{D}) of various anti-VEGF variants against human FcRn at pH 6.0 and 25°C. Results are representative of three independent experiments.
**Fig. 6****:** Kinetics parameters and monovalent dissociation constants (K_{D}) of various anti-VEGF variants against cyno FcRn at pH 6.0 and 25°C. Results are representative of three independent experiments.

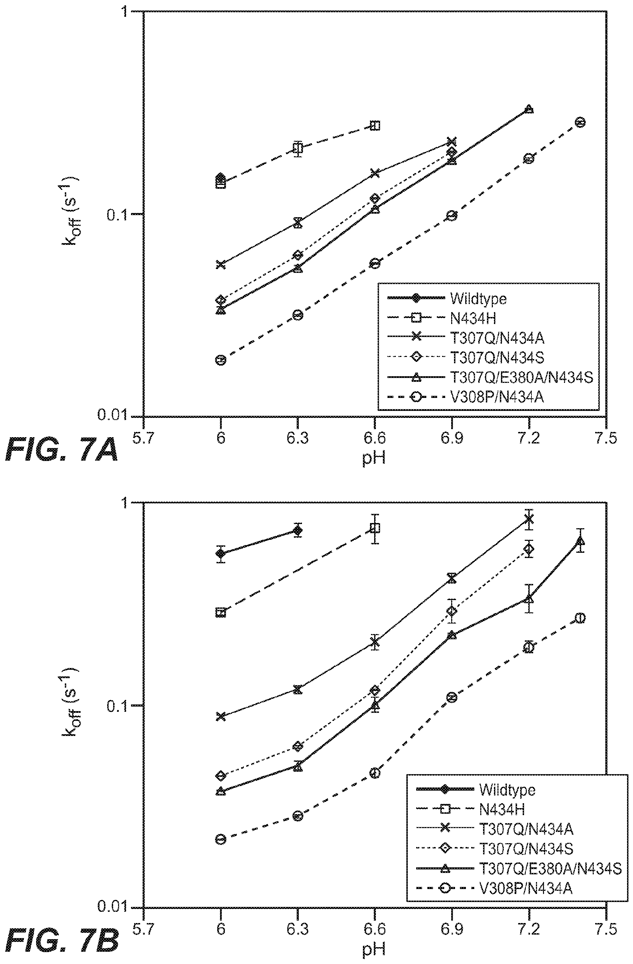

**Fig. 7** Panels A-B: The dissociation rate (k_{off}) of (A) human FcRn and (B) cyno FcRn against different anti-VEGF variants at different pHs.

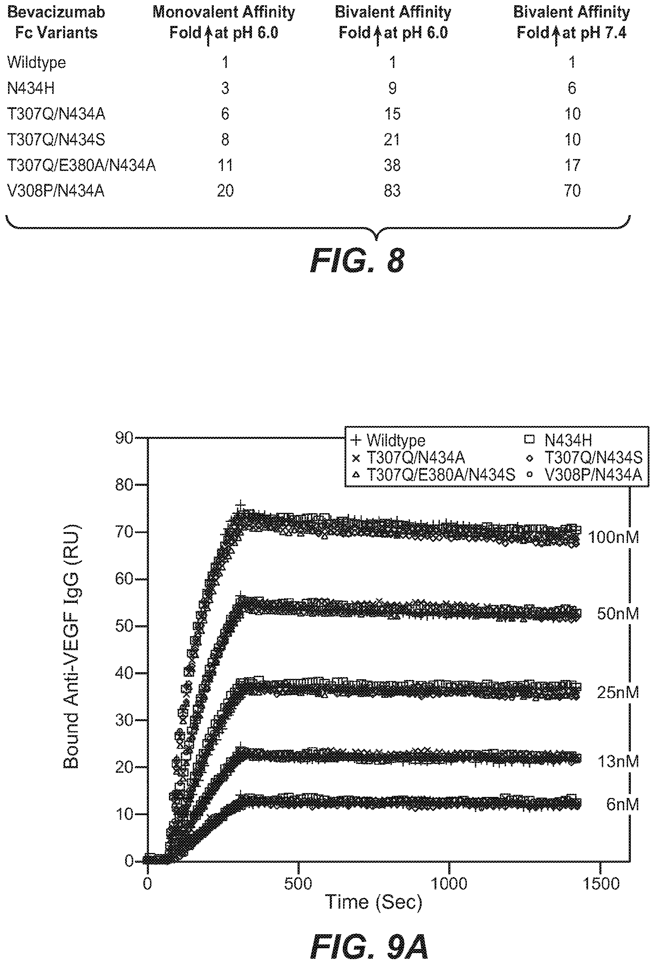

**Fig. 8****:** Summary of the human FcRn affinity improvement of the anti-VEGF variants over anti-VEGF wild-type. Data are summarized from Figures 4 and 5.

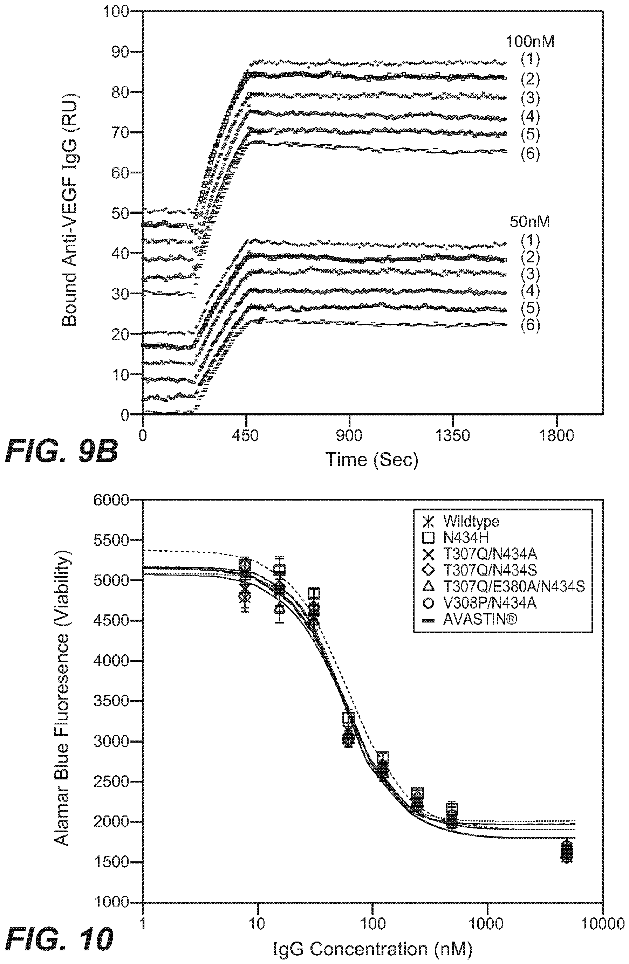

**Fig. 9** Panels A-B: The VEGF binding of (1) anti-VEGF wildtype and anti-VEGF variants (2) N434H, (3) T307Q/N434A, (4) T307Q/N434S, (5) T307Q/E380A/N434S and (6) V308P/N434A. (A) The VEGF binding of the antibodies determined by injecting anti-VEGF wildtype and anti-VEGF variants over a VEGF-A₁₀₉ coated sensor chip at 37°C using BIAcore® 3000. (B) Sensorgrams for the 50nM and 100nM injections. Each sensorgram baseline was offset by 4RU for better viewing.
**Fig. 10****:** The *in-vitro* HUVEC proliferation inhibition of AVASTIN®, anti-VEGF wildtype (bevacizumab) and anti-VEGF variants. Human umbilical vascular endothelial cells (HUVEC) were cultured in the presence of VEGF and various concentrations of anti-VEGF antibodies. Viability after 4 days of culture were assessed.

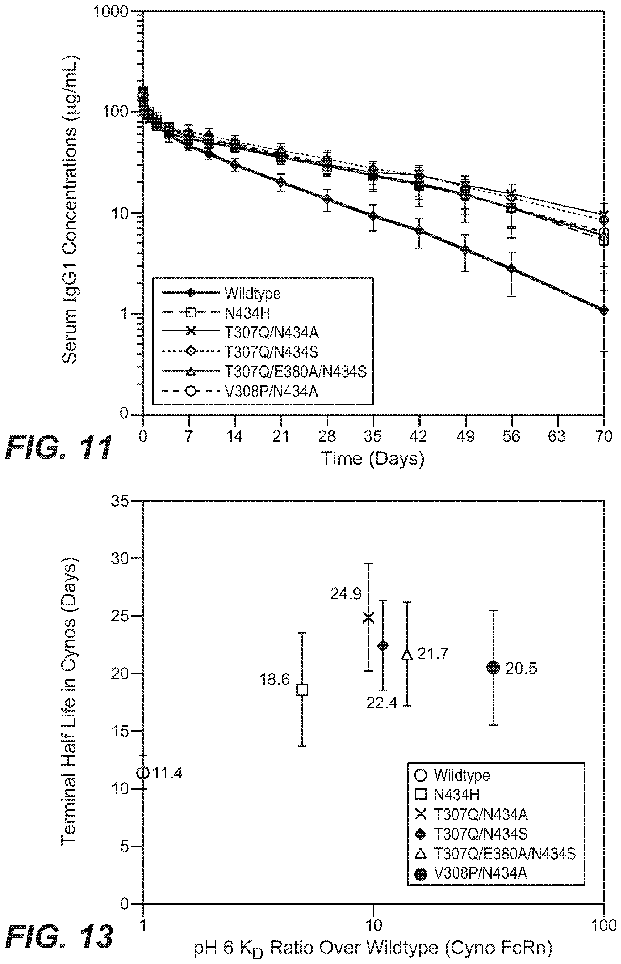

**Fig. 11****:** Pharmacokinetic profiles of the anti-VEGF wild-type and five anti-VEGF variants in cynomolgus monkeys following a single IV dose of 5 mg/kg. Serum concentrations of the antibodies were measured by ELISA. Data are represented as the mean ± standard deviation (n= 12 animals/group except for the V308P/N434A group that has 11 animals).

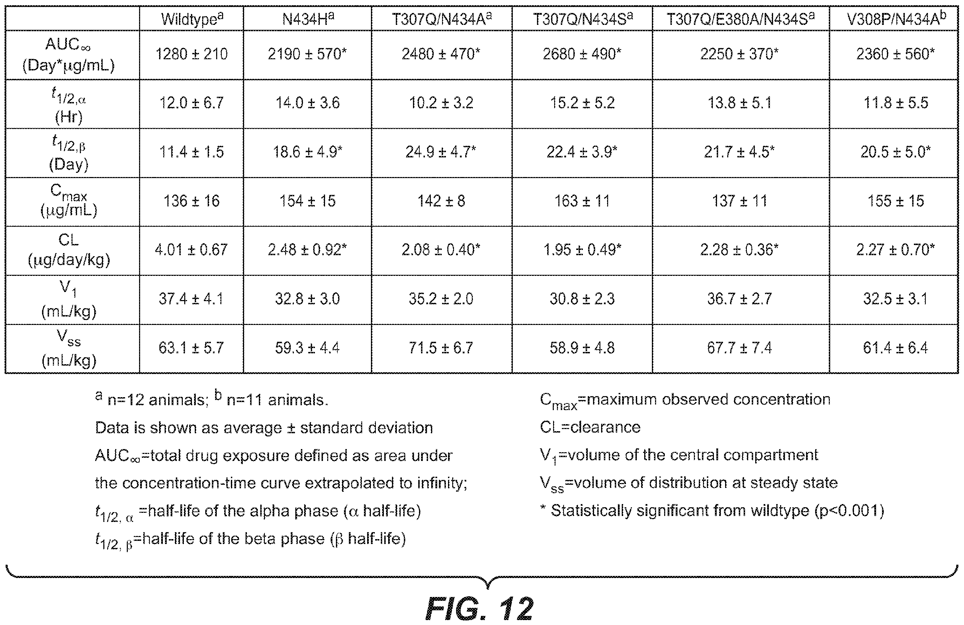

**Fig. 12****:** Pharmacokinetics parameters for the anti-VEGF wild-type and five anti-VEGF variants following a single IV dose of 5 mg/kg to cynomolgus monkeys.
**Fig. 13****:** Graph showing the relationship between terminal half-life in cynomolgus monkeys and pH 6.0 FcRn affinity for the anti-VEGF wild-type and five anti-VEGF variants. Error bars represent standard deviations of 11 or 12 animals per group.

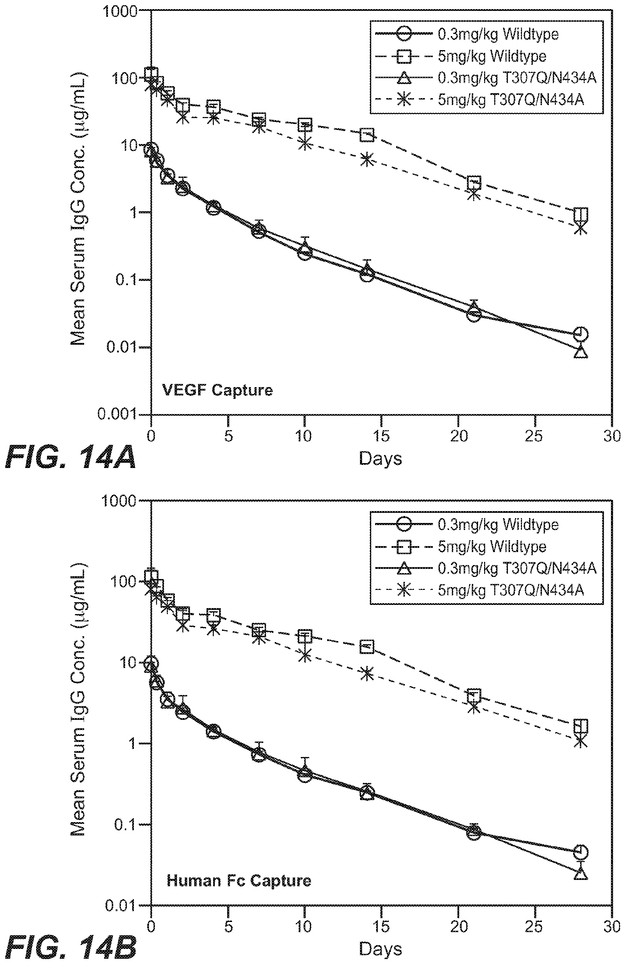

**Fig. 14** Panels A-B: Pharmacokinetic profiles of the anti-VEGF wild-type and anti-VEGF variant T307Q/N434A in humanized VEGF transgenic mice following a single IV dose of 0.3 or 5 mg/kg. Serum concentrations of the antibodies were measured using either (A) VEGF capture ELISA or (B) human Fc capture ELISA. Data are represented as the mean ± standard deviation.

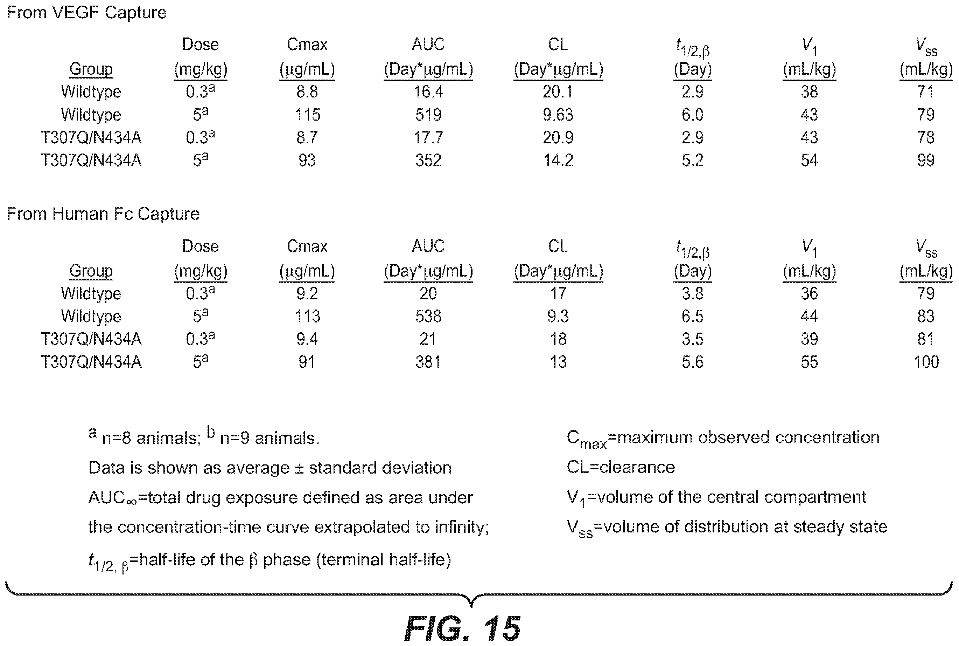

**Fig. 15****:** Pharmacokinetics parameters for the anti-VEGF wild-type and anti-VEGF variant T307Q/N434A following a single intravenous dose of 0.3 or 5 mg/kg to humanized VEGF transgenic mice.

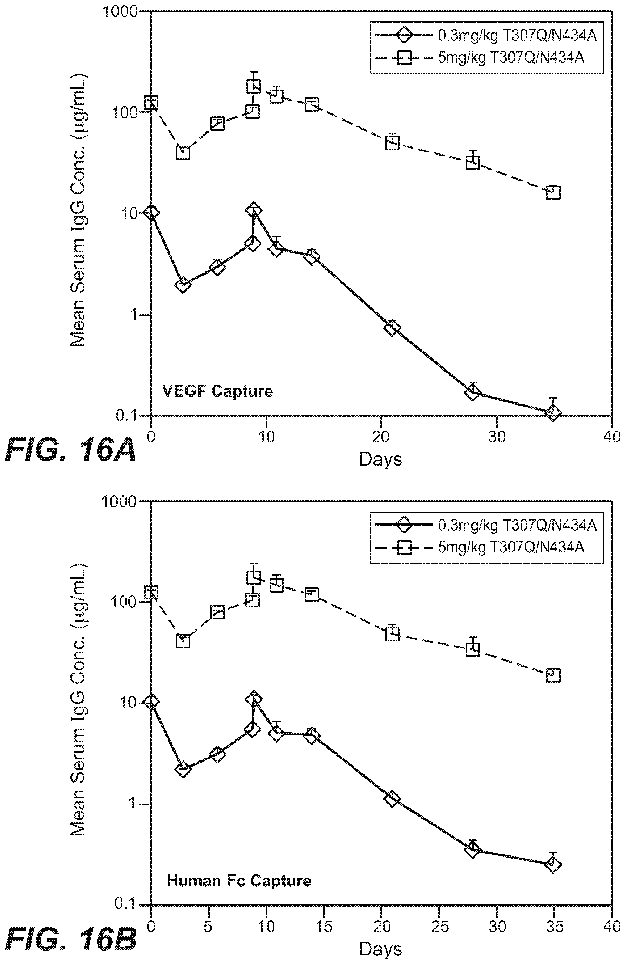

**Fig. 16** Panels A-B: Pharmacokinetic profiles of anti-VEGF variant T307Q/N434A in humanized VEGF transgenic mice following a multi-dose of 0.3 or 5 mg/kg. Antibody was administered at day 0, 3, 6, and 9. Serum concentrations of the antibodies were measured using either (A) VEGF capture ELISA or (B) human Fc capture ELISA. Data are represented as the mean ± standard deviation.

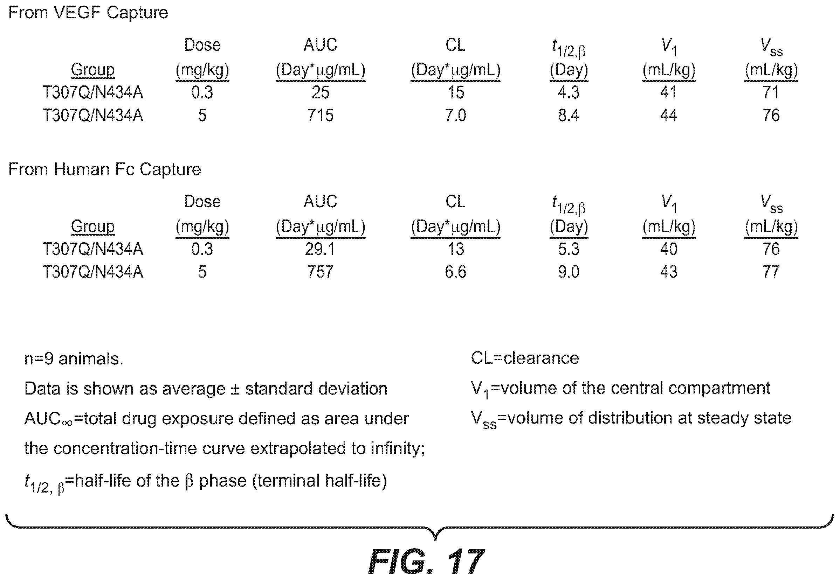

**Fig. 17****:** Pharmacokinetics parameters for the T307Q/N434A following four intravenous doses of 0.3 or 5 mg/kg at day 0, 3, 6, and 9 to humanized VEGF transgenic mice.

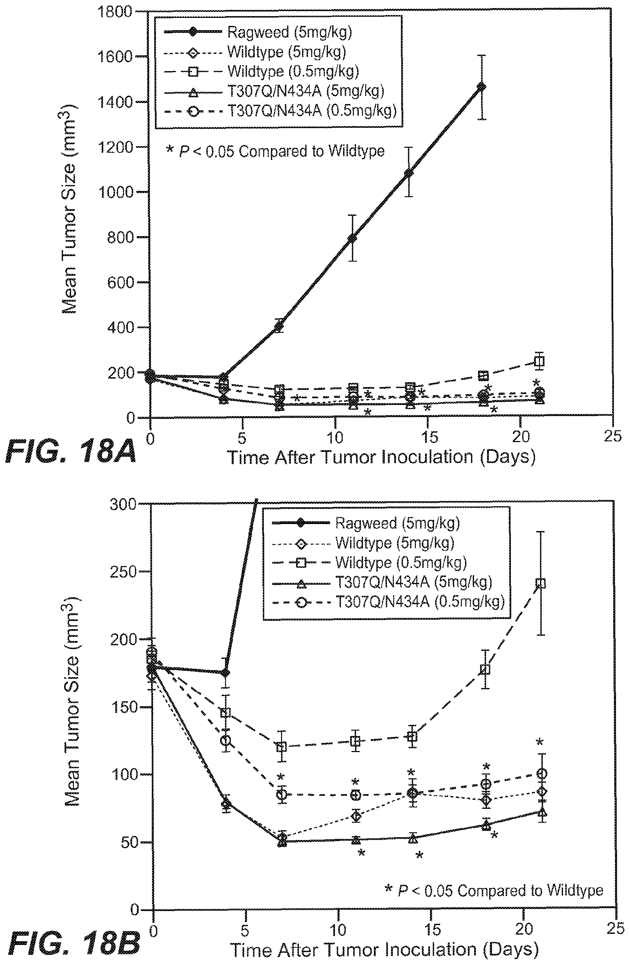

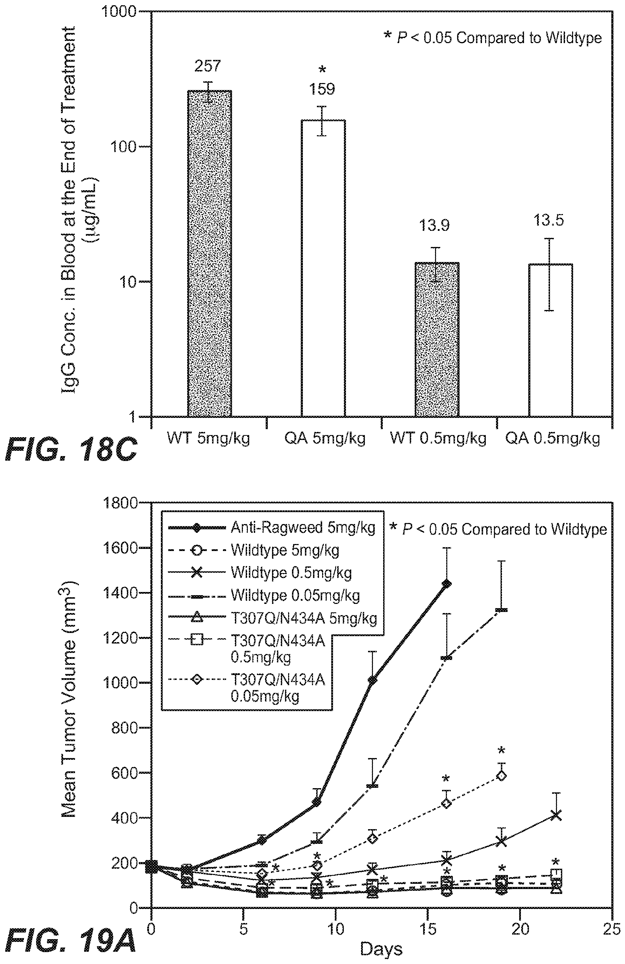

**Fig. 18** Panels A-C: Efficacy of anti-VEGF wildtype and T307Q/N434A (QA) variant in treating HM-7 xenografts implanted s.c. into RAG2 KO; hum-X VEGF KI double-homozygous mice. 5mg/kg and 0.5mg/kg of wildtype and T307Q/N434A variant and 5mg/kg of anti-ragweed control were administered intraperitoneal twice weekly. (A) Growth curves of HM-7 tumors. Data are represented as the mean ± standard error. (B) Growth curves of HM-7 tumors for 0.5 and 0.05mg/kg treatment groups. (C) Serum anti-VEGF antibody concentrations at the end of treatment (day 18 for anti-ragweed and day 21 for the anti-VEGF treatment groups). Concentrations were measured using the VEGF capture ELISA. Data are represented as the mean ± standard deviation.

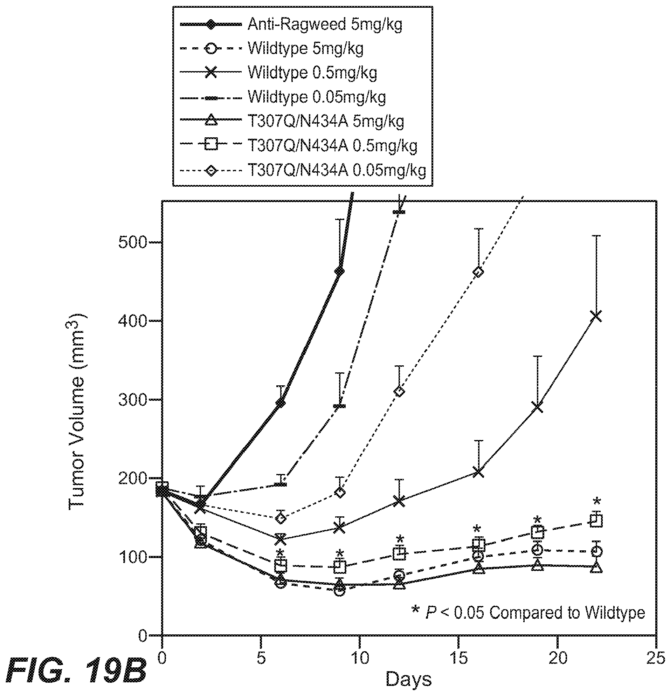

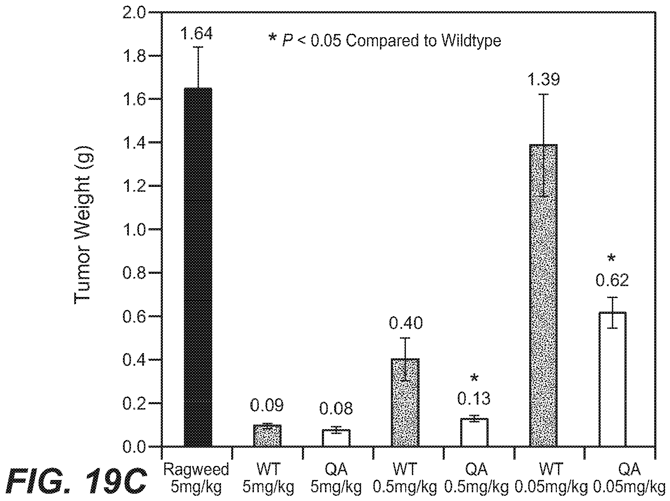

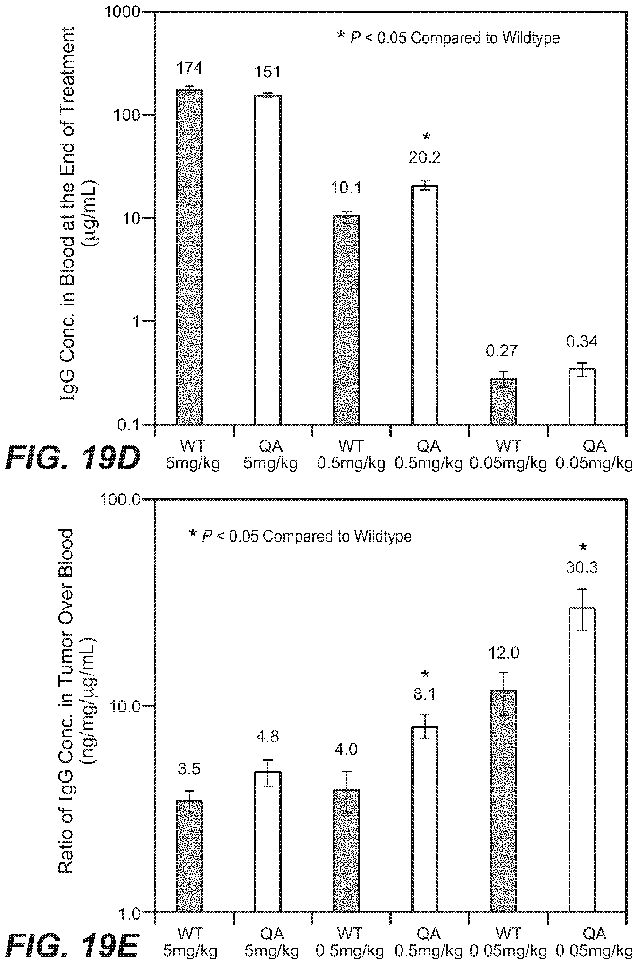

**Fig. 19** Panels A-E: A repeat efficacy study of anti-VEGF wildtype and T307Q/N434A (QA) variant in treating HM-7 xenografts implanted s.c. into RAG2 KO; hum-X VEGF KI double-homozygous mice. 5, 0.5 and 0.05mg/kg of wildtype and T307Q/N434A variant and 5mg/kg of anti-ragweed control were administered intraperitoneal twice weekly. (A) Growth curves of HM-7 tumors. Data are represented as the mean ± standard error. (B) Growth curves of HM-7 tumors for 0.5 and 0.05mg/kg treatment groups. Data are represented as the mean ± standard error. (C) Terminal weights of HM-7 tumors determined at the end of treatment (day 16 for anti-ragweed, day 19 for 0.05mg/kg group, and day 22 of the remaining groups). Data are represented as the mean ± standard error. (D) Serum anti-VEGF antibody concentrations at the end of treatment. Concentrations were measured using the human Fc capture ELISA. Data are represented as the mean ± standard deviation. (E) Ratio of antibody concentration in tumors to that in blood. Tumor antibody concentrations were determined by measuring the total amount of tumor lysates and the amount of anti-VEGF antibody in the tumor lysates. Data are represented as the mean ± standard deviation.

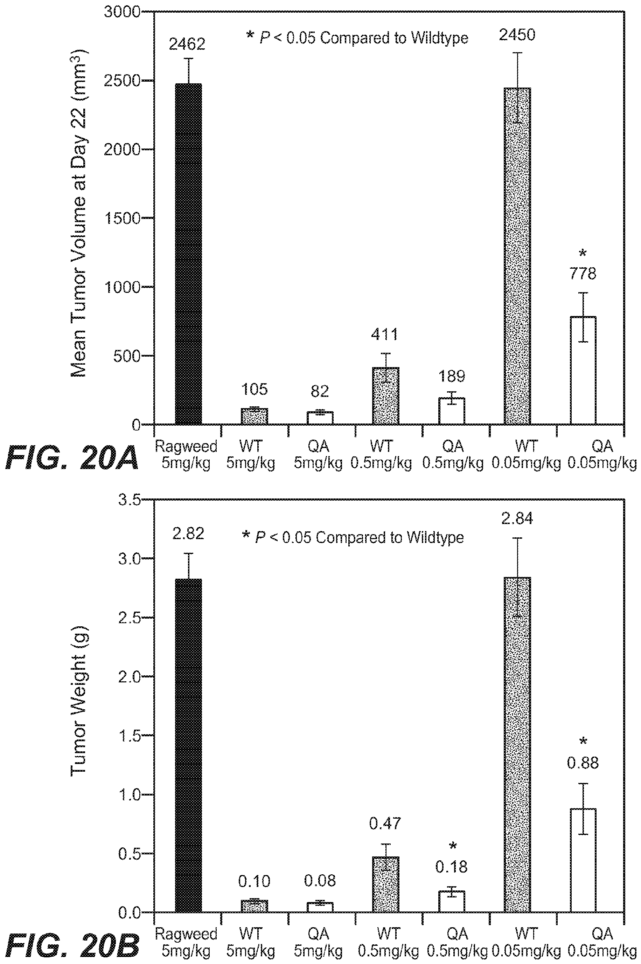

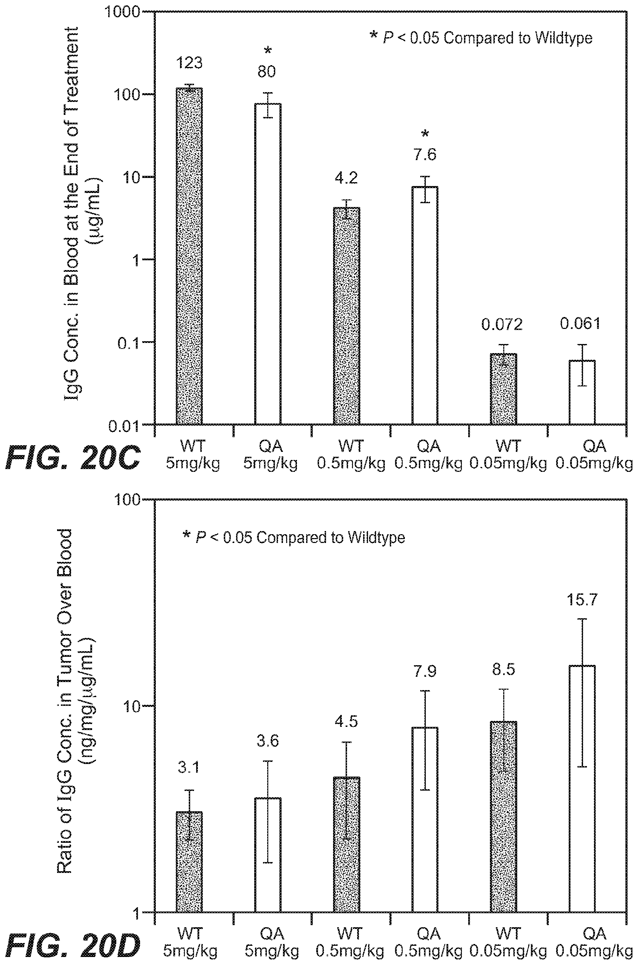

**Fig. 20** Panels A-D: The third efficacy study of anti-VEGF wildtype and T307Q/N434A (QA) variant in treating HM-7 xenografts implanted s.c. into RAG2 KO; hum-X VEGF KI double-homozygous mice. 5, 0.5 and 0.05mg/kg of wildtype and T307Q/N434A and 5mg/kg of anti-ragweed control were administered intraperitoneal twice weekly. (A) Mean tumor volume for each group at the end of treatment (day 22). Data are represented as the mean ± standard error. (B) Terminal weights of HM-7 tumors. Data are represented as the mean ± standard error. (C) Serum anti-VEGF antibody concentrations at the end of treatment. Concentrations were measured using the anti-human Fc capture ELISA. Data are represented as the mean ± standard deviation. (D) Ratio of antibody concentration in tumors to that in blood. Tumor antibody concentrations were determined by measuring the total amount of tumor lysates and the amount of anti-VEGF antibody in the tumor lysates. Data are represented as the mean ± standard deviation.

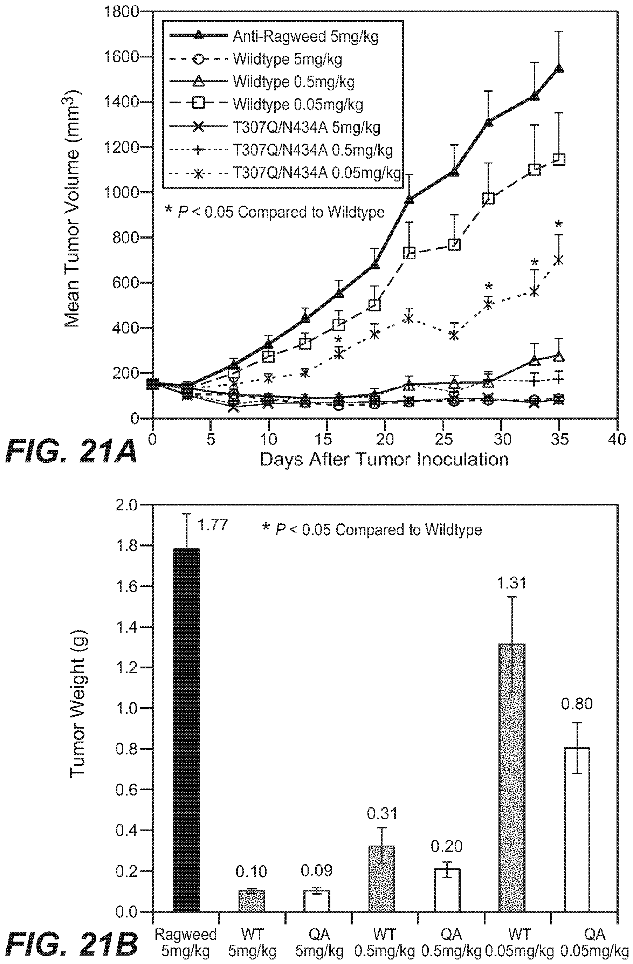

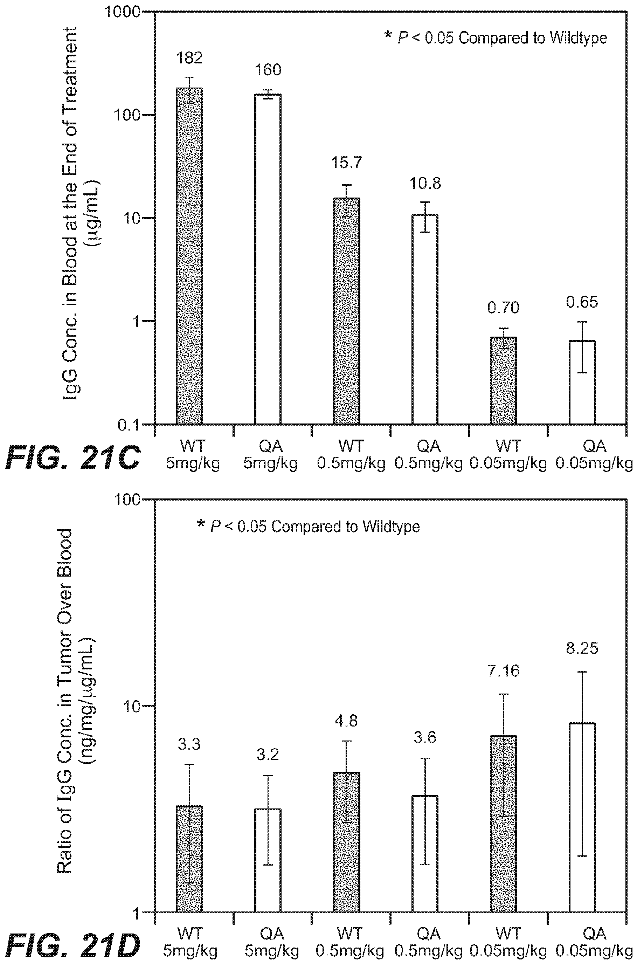

**Fig. 21** Panels A-D: Efficacy of anti-VEGF wildtype and T307Q/N434A (QA) variant in treating HT-55 xenografts implanted s.c. into RAG2 KO; hum-X VEGF KI double-homozygous mice. 5, 0.5 and 0.05mg/kg of wildtype and T307Q/N434A and 5mg/kg of anti-ragweed control were administered intraperitoneal twice weekly. (A) Growth curves of HT-55 tumors. Data are represented as the mean ± standard error. (B) Terminal weights of HT-55 tumors determined at the end of treatment (day 35). Data are represented as the mean ± standard error. (C) Serum anti-VEGF antibody concentrations at the end of treatment. Concentrations were measured using the human Fc capture ELISA. Data are represented as the mean ± standard deviation. (D) Ratio of antibody concentration in tumors to that in blood. Tumor antibody concentrations were determined by measuring the total amount of tumor lysates and the amount of anti-VEGF antibody in the tumor lysates. Data are represented as the mean ± standard deviation.

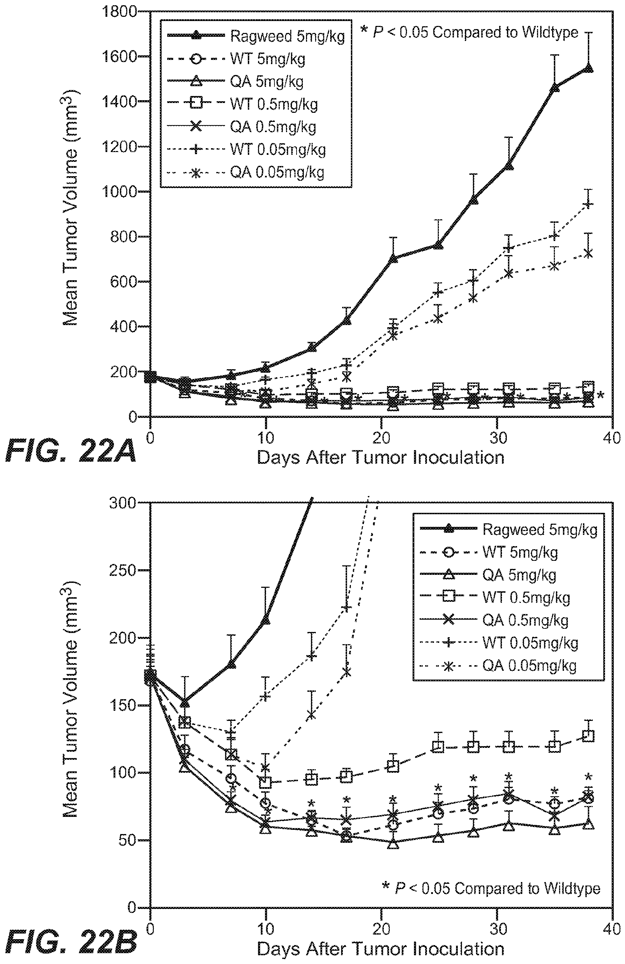

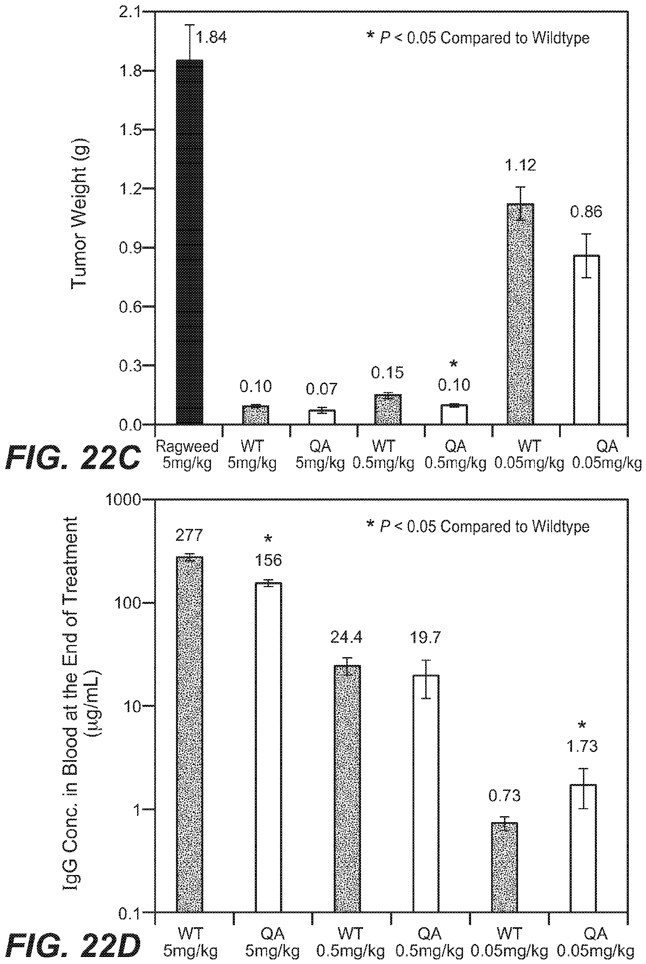

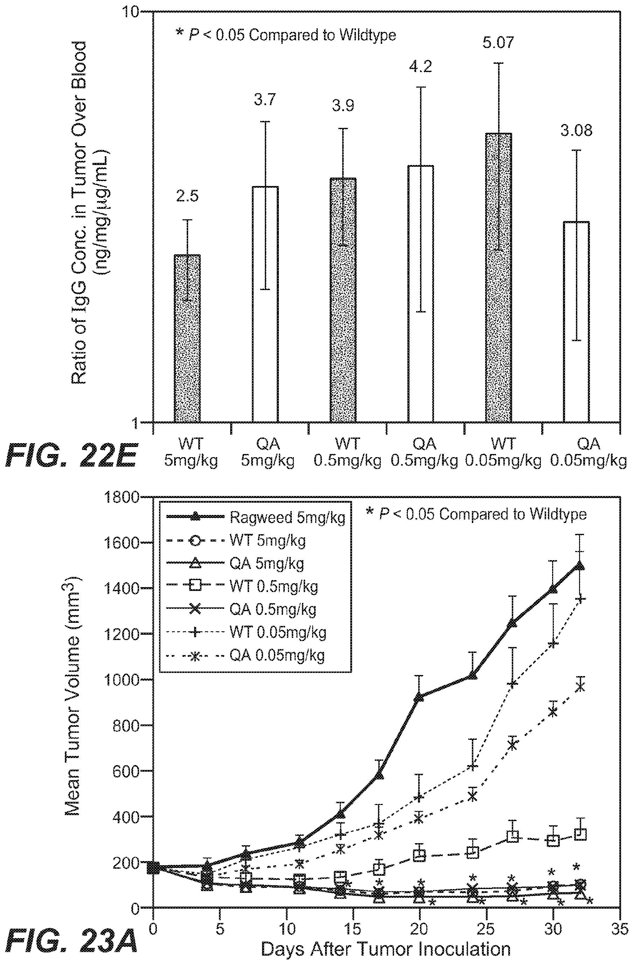

**Fig. 22** Panels A-E: Efficacy of anti-VEGF wildtype and T307Q/N434A (QA) variant in treating Colo-205 xenografts implanted s.c. into RAG2 KO; hum-X VEGF KI double-homozygous mice. 5, 0.5 and 0.05mg/kg of wildtype and T307Q/N434A and 5mg/kg of anti-ragweed control were administered intraperitoneal twice weekly. (A) Growth curves of Colo-205 tumors. Data are represented as the mean ± standard error. (B) Growth curves of Colo-205 tumors at 0.5 and 0.05mg/kg treatment groups. (C) Terminal weights of Colo-205 tumors determined at the end of treatment (day 38). Data are represented as the mean ± standard error. (D) Serum anti-VEGF antibody concentrations at the end of treatment. Concentrations were measured using the human Fc capture ELISA. Data are represented as the mean ± standard deviation. (E) Ratio of antibody concentration in tumors to that in blood. Tumor antibody concentrations were determined by measuring the total amount of tumor lysates and the amount of anti-VEGF antibody in the tumor lysates. Data are represented as the mean ± standard deviation.

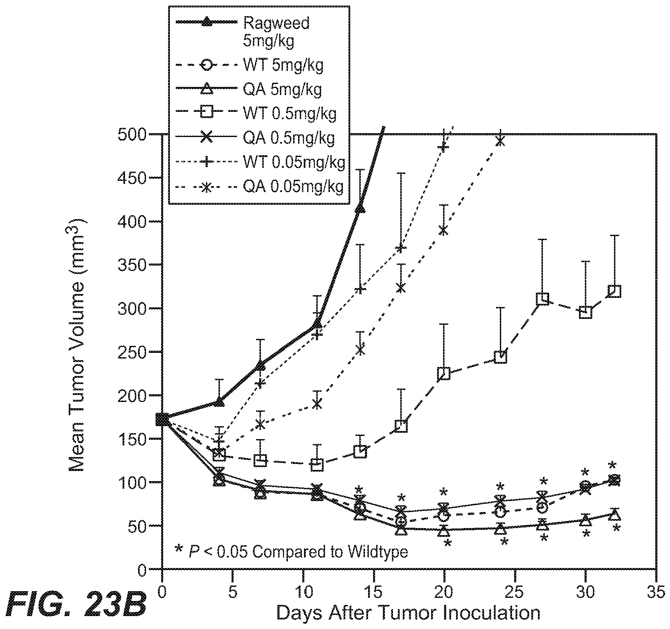

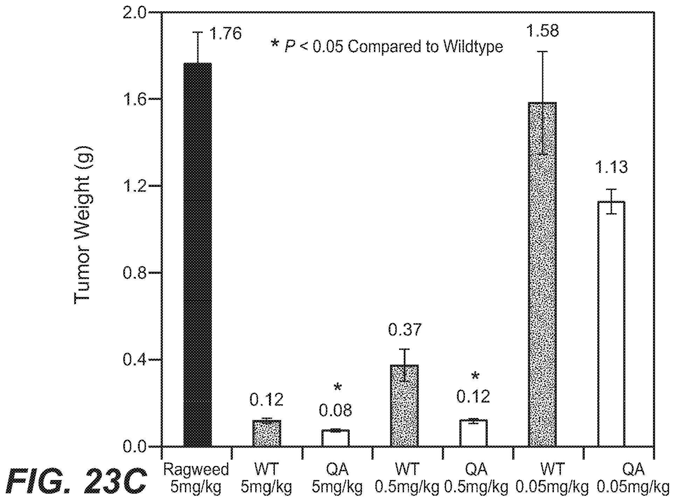

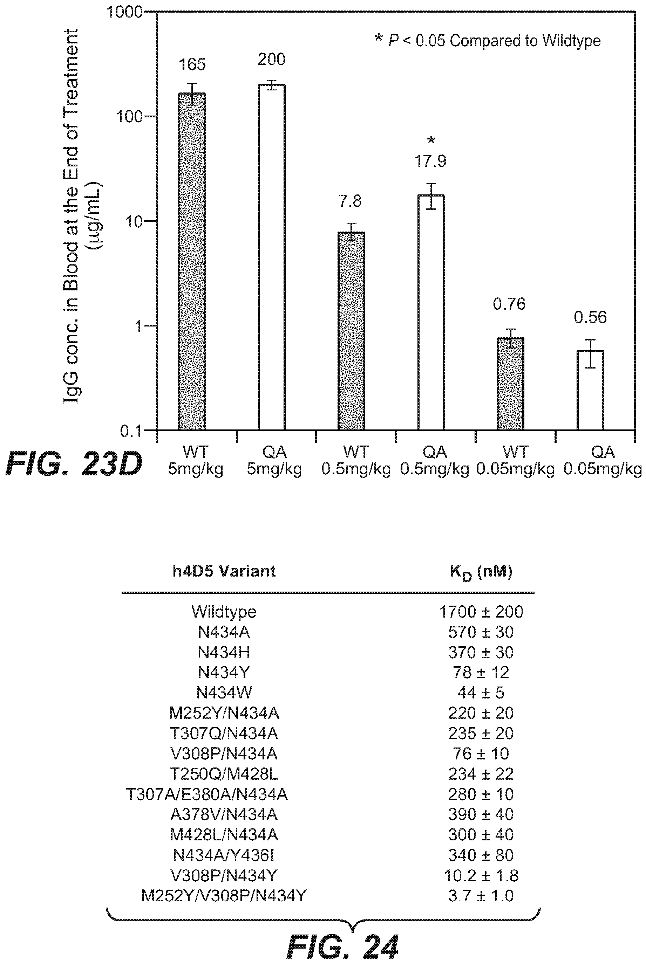

**Fig. 23** Panels A-D: A repeat efficacy study of anti-VEGF wildtype and T307Q/N434A (QA) variant in treating Colo-205 xenografts. 5, 0.5 and 0.05mg/kg of wildtype and T307Q/N434A and 5mg/kg of anti-ragweed control were administered intraperitoneal twice weekly. (A) Growth curves of Colo-205 tumors. Data are represented as the mean ± standard error. (B) Growth curves of Colo-205 tumors at 0.5 and 0.05mg/kg treatment groups. (C) Terminal weights of Colo-205 tumors determined at the end of treatment (day 32). Data are represented as the mean ± standard error. (D) Serum anti-VEGF antibody concentrations at the end of treatment. Concentrations were measured using the human Fc capture ELISA. Data are represented as the mean ± standard deviation.
**Fig. 24****:** Monovalent dissociation constants (K_{D}) of anti-HER2 (traztuzumab) IgG₁ Fc variants to human FcRn at pH 6.0 and 25°C using BIAcore. Results are representative of two independent experiments.

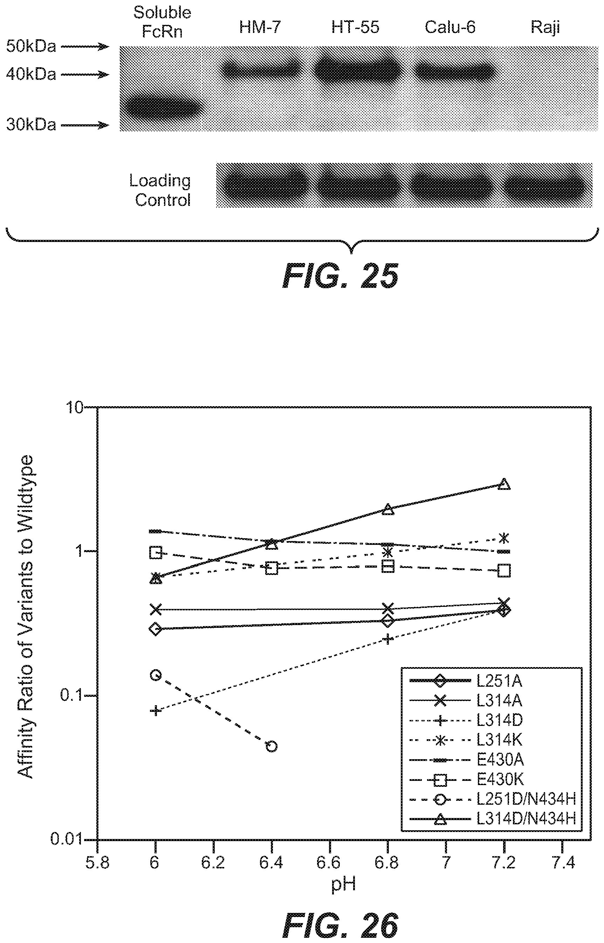

**Fig. 25****:** Expression levels of FcRn in different human tumor cell lines. Five million cells of each cell line were used for the experiment. Raji cells (human B-cell lymphoma) were used as a negative control, while soluble human FcRn protein, which is missing the 7kDa transmembrane and cytoplasmic regions, was blotted as a positive control. Dilutions of soluble FcRn protein were used as the standard to quantify the FcRn expression level. Results shown here are representative of at least three independent experiments.
**Fig. 26****:** pH-dependent binding of anti-HER2 (traztuzumab) IgGi Fc variants to human FcRn. Variants were constructed with mutations at L251, L314, and E430. The binding was measured at pH ranging from 6 to 7.2 using BIAcore at 25°C. The affinity ratios of the variants relative to anti-HER2 IgGi wildtype were determined and plotted as a function of pH.

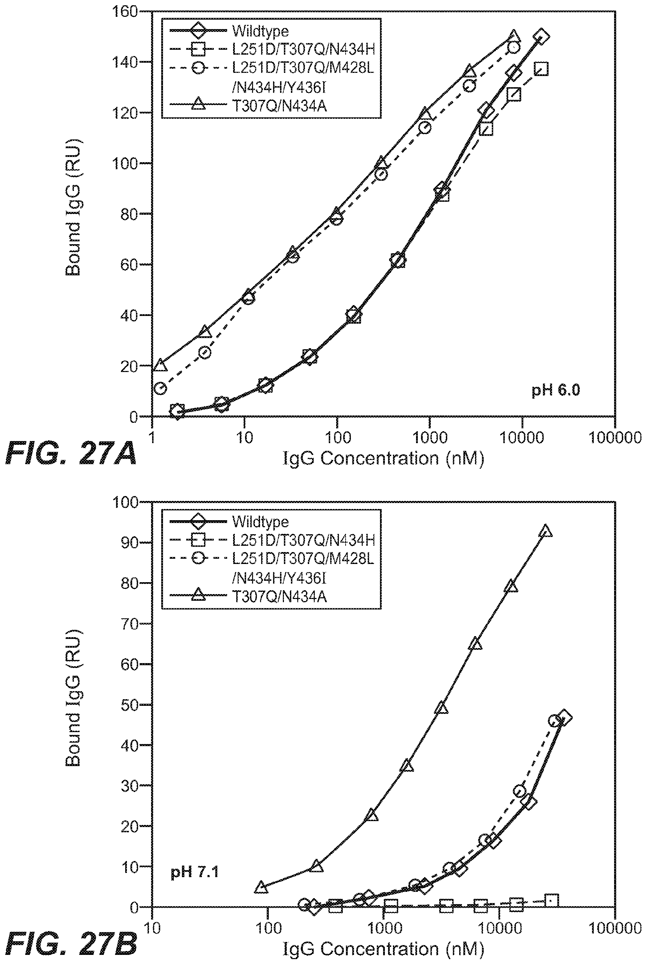

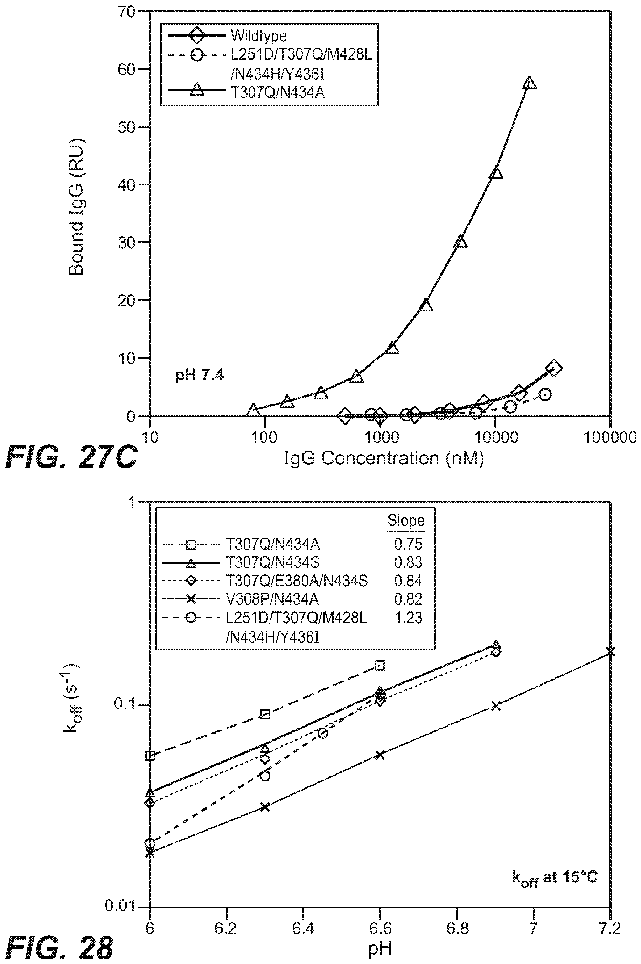

**Fig. 27** Panels A-C: Binding of anti-HER2 (traztuzumab) IgGi wild-type, variant T307Q/N434A, variant L251D/T307Q/N434H and variant L251D/T307Q/M428L/N434H/Y436I against human FcRn at (A) pH 6.0, (B) pH 7.1, and (C) pH 7.4. The binding was measured using BIAcore at 25°C. There was no detectable binding of variant L251D/T307Q/N434H to human FcRn at pH 7.4 in Fig. 27C.
**Fig. 28****:** The dissociation rate (k_{off}) of human FcRn against various anti-VEGF and anti-HER2 variants at different pHs. The anti-VEGF variants are T307Q/N434A, T307Q/N434S. T307Q/E380A/N434S and V308P/N434A. The anti-HER2 variant is L251D/T307Q/M428L/N434H/Y436I. The k_{off} values at different pHs were fitted against pH for each variant to yield the slope of the best-fit line (equation: log(k_{off}) = slope x pH +y-intercept).

### DETAILED DESCRIPTION OF THE INVENTION

The present invention relates to novel variants of Fc domains, including those found in antibodies, Fc fusions, and immuno-adhesins, that have an increased *in vivo* half-life. These variants comprise a human IgG Fc region, or fragment thereof that binds to an FcRn, that contains one or more amino acid modifications relative to a wild type human IgG Fc region which modifications increase the affinity of the IgG Fc region, or fragment thereof, for the FcRn.

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3rd. edition (2001) Cold Spring Harbor Laboratory Press, Cold Spring Harbor, N.Y. CURRENT PROTOCOLS IN MOLECULAR BIOLOGY (F. M. Ausubel, et al. eds., (2003)); the series METHODS IN ENZYMOLOGY (Academic Press, Inc.): PCR 2: A PRACTICAL APPROACH (M. J. MacPherson, B. D. Hames and G. R. Taylor eds. (1995)), Harlow and Lane, eds. (1988) ANTIBODIES, A LABORATORY MANUAL, and ANIMAL CELL CULTURE (R. I. Freshney, ed. (1987)); Oligonucleotide Synthesis (M. J. Gait, ed., 1984); Methods in Molecular Biology, Humana Press; Cell Biology: A Laboratory Notebook (J. E. Cellis, ed., 1998) Academic Press; Animal Cell Culture (R. I. Freshney), ed., 1987); Introduction to Cell and Tissue Culture (J. P. Mather and P. E. Roberts, 1998) Plenum Press; Cell and Tissue Culture: Laboratory Procedures (A. Doyle, J. B. Griffiths, and D. G. Newell, eds., 1993-8) J. Wiley and Sons; *Handbook of Experimental Immunology* (D. M. Weir and C. C. Blackwell, eds.); Gene Transfer Vectors for Mammalian Cells (J. M. Miller and M. P. Calos, eds., 1987); PCR: The Polymerase Chain Reaction, (Mullis et al., eds., 1994); Current Protocols in Immunology (J. E. Coligan et al., eds., 1991); Short Protocols in Molecular Biology (Wiley and Sons, 1999); Immunobiology (C. A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V. T. DeVita et al., eds., J.B. Lippincott Company, 1993).

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

### Definitions

For purposes of interpreting this specification, the following definitions will apply and whenever appropriate, terms used in the singular will also include the plural and vice versa. Throughout the present specification and claims, the numbering of the residues in an immunoglobulin heavy chain is that of the EU index as in Kabat et al., Sequences of Proteins of Immunological Interest, 5th Ed. Public Health Service, National Institutes of Health, Bethesda, Md. (1992). The "EU index as in Kabat" refers to the residue numbering of the human IgGi EU antibody.

The term "*in vivo* half-life" or "the half life of the antibody *in vivo*" as used herein refers to a biological half-life of a particular type of IgG molecule or its fragments containing FcRn-binding sites in the circulation of a given animal and is represented by the time required for the circulating concentration of a molecule to decrease by 50%. In certain embodiments, when the concentration of a given IgG is plotted as a function of time, the curve is usually biphasic with a rapid α-phase which represents an equilibration of the injected IgG molecules between the intra- and extra-vascular space, and a longer β-phase which represents the elimination of the IgG molecules from the intravascular space. In certain embodiments, the term "*in vivo* half-life" corresponds to the half life of the IgG molecules in the β-phase. In certain embodiments, the concentration versus time curve of a given IgG is triphasic, with corresponding distribution into an α-phase and β-phase, and terminal elimination represented by a γ-phase. Therefore, in certain embodiments, *in vivo* half-life corresponds to the half-life of the terminal elimination phase, or the γ-phase. In certain embodiments, the concentration versus time curve of a given IgG is monophasic, with a single elimination phase. Therefore, in certain embodiments, *in vivo* half-life corresponds to the half-life of the single elimination phase.

By "parent polypeptide" or "wild-type polypeptide" as used herein is meant an unmodified polypeptide, a naturally occurring polypeptide, or an engineered modified version of a naturally occurring polypeptide which lacks one or more of the Fc region amino acid modifications disclosed herein and which differs in effector function compared to variant IgG as herein disclosed. The parent polypeptide may comprise a native sequence Fc region or an Fc region with pre-existing amino acid sequence modifications (such as additions, deletions and/or substitutions). The parent polypeptide may also comprise non-natural amino acids as described below. Parent polypeptide may refer to the polypeptide itself, compositions that comprise the parent polypeptide, or the amino acid sequence that encodes it. Parent polypeptide, includes, without limitation, parent immunoglobulin, wild-type immunoglobulin, parent antibody and wild-type antibody.

Accordingly, by "parent immunoglobulin," "parent IgG," "wild-type immunoglobulin" or "wild-type IgG" as used herein is meant an unmodified immunoglobulin, a naturally occurring immunoglobulin, or an engineered modified version of a naturally occurring immunoglobulin which lacks one or more of the Fc region amino acid modifications disclosed herein and which differs in effector function compared to variant IgG as herein disclosed. The parent immunoglobulin may comprise a native sequence Fc region or an Fc region with pre-existing amino acid sequence modifications (such as additions, deletions and/or substitutions). The parent immunoglobulin may also comprise non-natural amino acids as described below. Parent immunoglobulin may refer to the immunoglobulin itself, compositions that comprise the parent immunoglobulin, or the amino acid sequence that encodes it.

By "parent antibody" or "wild-type antibody" as used herein is meant an unmodified antibody, a naturally occurring antibody, or an engineered modified version of a naturally occurring antibody which lacks one or more of the Fc region amino acid modifications disclosed herein and which differs in effector function compared to variant IgG as herein disclosed. The parent antibody may comprise a native sequence Fc region or an Fc region with pre-existing amino acid sequence modifications (such as additions, deletions and/or substitutions). The parent antibody may also comprise non-natural amino acids as described below. Parent antibody may refer to the antibody itself, compositions that comprise the parent antibody, or the amino acid sequence that encodes it.

In certain embodiments, "parent IgG," "parent antibody," "wild-type IgG," or "wild-type antibody" includes, but not limited to, known commercial, recombinantly produced antibodies as described herein. In certain embodiments, wild-type IgG is an IgG having the wild-type human IgG Fc region. In certain embodiments, the wild-type human IgG Fc region refers to Fc region which lacks one or more of the Fc region amino acid modifications disclosed herein. In certain embodiments, the wild-type human IgG Fc region refers to Fc region with one or more Fc region amino acid modifications not disclosed herein. In certain embodiments, the wild-type IgG is bevacizumab. According to the present disclosure, the variant IgG of such wild-type antibody is an Fc fusion protein comprising Fab domain fragments of the wild-type antibody, or the domain or domains of a non-antibody protein, and an Fc domain fragment comprising one or more of the Fc region modifications disclosed herein. According to the present disclosure, the wild-type human IgG Fc region refers to an IgG Fc region with Fc mutation L251D or L251D/434H, but has no other Fc region amino acid modifications disclosed herein.

By "variant," "variant protein" or "protein variant" as used herein is meant a protein that differs from that of a parent protein by virtue of at least one amino acid modification. Protein variant may refer to the protein itself, a composition comprising the protein, or the amino sequence that encodes it. The protein variant may have at least one amino acid modification compared to the parent polypeptide, *e.g.* from about one to about ten amino acid modifications. The protein variant may also have at least two amino acid modifications in the IgG Fc region, or at least three amino acid modifications in the IgG Fc region. The protein variant sequence herein may possess at least about 80% homology with a parent protein sequence; at least about 90% homology, more preferably at least about 95% homology. Protein variants may also comprise non-natural amino acids, as defined below. The term "protein variant" includes immunoglobulin variant and antibody variant as described herein.

The term "immunoglobulin variant," "variant immunoglobulin," "variant IgG" or "IgG variant" as used herein is meant an immunoglobulin sequence that differs from that of a parent or wild-type immunoglobulin sequence by virtue of at least one amino acid modification. In certain embodiments, the variant IgG is an anti-VEGF antibody. In one embodiment, the variant IgG is a variant of bevacizumab comprising one or more amino acid modification in the Fc region of the antibody.

By "antibody variant" or "variant antibody" as used herein is meant an antibody that differs from a parent antibody by virtue of at least one amino acid modification. In certain embodiments, the variant antibody has one or more amino acid modifications in the Fc region relative to wild-type antibody.

By "position" as used herein is meant a location in the sequence of a protein. Positions may be numbered sequentially, or according to an established format, for example the EU index as in Kabat.

The term "Fc region-containing polypeptide" or "Fc polypeptide" refers to a polypeptide that comprises all or part of an Fc region. For example, Fc polypeptides include antibodies, Fc fusions, isolated Fcs, and Fc fragments.

The term "Fc region-comprising antibody" refers to an antibody that comprises an Fc region. The C-terminal lysine (residue 447 according to the EU numbering system) of the Fc region may be removed, for example, during purification of the antibody or by recombinant engineering of the nucleic acid encoding the antibody. Accordingly, a composition comprising an antibody having an Fc region can comprise an antibody with K447, with all K447 removed, or a mixture of antibodies with and without the K447 residue.

An "amino acid modification" refers to a change in the amino acid sequence of a predetermined amino acid sequence. Exemplary modifications include an amino acid substitution, insertion and/or deletion. In certain embodiments, the amino acid modification is a substitution.

An "amino acid modification at" a specified position, *e.g.* of the Fc region, refers to the substitution or deletion of the specified residue, or the insertion of at least one amino acid residue adjacent the specified residue. By insertion "adjacent" a specified residue is meant insertion within one to two residues thereof. The insertion may be N-terminal or C-terminal to the specified residue.

An "amino acid substitution" refers to the replacement of at least one existing amino acid residue in a predetermined amino acid sequence with another different "replacement" amino acid residue. The replacement residue or residues may be "naturally occurring amino acid residues" (*i.e.* encoded by the genetic code) and selected from the group consisting of: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val). In certain embodiments, the replacement residue is not cysteine. Substitution with one or more non-naturally occurring amino acid residues is also encompassed by the definition of an amino acid substitution herein.

A "non-naturally occurring amino acid residue" refers to a residue, other than those naturally occurring amino acid residues listed above, which is able to covalently bind adjacent amino acid residues(s) in a polypeptide chain. Examples of non-naturally occurring amino acid residues include norleucine, ornithine, norvaline, homoserine and other amino acid residue analogues such as those described in Ellman et al. Meth. Enzym. 202:301-336 (1991); US Patent No. 6,586,207; WO 98/48032; WO 03/073238; U.S. Publication No. 2004-0214988A1; WO 05135727A2; WO 05/74524A2; J. W. Chin et al., (2002), Journal of the American Chemical Society 124:9026-9027; J. W. Chin, & P. G. Schultz, (2002), ChemBioChem 11:1135-1137; and J. W. Chin, et al., (2002), PICAS United States of America 99:11020-11024. An "amino acid insertion" refers to the incorporation of at least one amino acid into a predetermined amino acid sequence. While the insertion will usually consist of the insertion of one or two amino acid residues, the present application contemplates larger "peptide insertions", *e.g.* insertion of about three to about five or even up to about ten amino acid residues. The inserted residue(s) may be naturally occurring or non-naturally occurring as disclosed above.

An "amino acid deletion" refers to the removal of at least one amino acid residue from a predetermined amino acid sequence.

In certain embodiments, the term "increase," "increased half life" or "increased *in vivo* half life" refers to an overall increase of at least about 25%, 30%, 40%, 50%, 60%, 70%, 75%, 80%, 85%, 90%, 95%, 100%, 150%, 200%, 300% or greater, in the *in vivo* half life of a variant IgG of the invention detected by standard art known methods such as those described herein, as compared to a wild-type IgG or an IgG having the wild-type human IgG Fc region. In certain embodiments, the term increase refers to the increased *in vivo* half life of the variant IgG, wherein the increase is at least about 1.25X, 1.5X, 1.75X, 2X, 3X, 4X, 5X, or 10X or greater as compared to a wild-type IgG or an IgG having the wild-type human IgG Fc region. In certain embodiments, the wild-type IgG or the IgG having the wild-type human IgG Fc region is bevacizumab. In certain embodiments, the mean half-life of bevacizumab is about 10 to 12 days as measured in cynomolgus monkeys, or about 20 days as measured in humans.

"Hinge region" is generally defined as stretching from Glu216 to Pro230 of human IgG1 (Burton, Molec. Immunol. 22:161-206 (1985)). Hinge regions of other IgG isotypes may be aligned with the IgG1 sequence by placing the first and last cysteine residues forming inter-heavy chain S-S bonds in the same positions.

The "lower hinge region" of an Fc region is normally defined as the stretch of residues immediately C-terminal to the hinge region, i.e. residues 233 to 239 of the Fc region. Prior to the present invention, FcγR binding was generally attributed to amino acid residues in the lower hinge region of an IgG Fc region.

"C1q" is a polypeptide that includes a binding site for the Fc region of an immunoglobulin. C1q together with two serine proteases, C1r and C1s, forms the complex C1, the first component of the complement dependent cytotoxicity (CDC) pathway. Human C1q can be purchased commercially from, *e.g.* Quidel, San Diego, Calif.

The term "binding domain" refers to the region of a polypeptide that binds to another molecule. In the case of an FcR, the binding domain can comprise a portion of a polypeptide chain thereof (*e.g.*, the α chain thereof) which is responsible for binding an Fc region. One useful binding domain is the extracellular domain of an FcR α chain.

The term "antibody" herein is used in the broadest sense and specifically covers monoclonal antibodies (including full length monoclonal antibodies), polyclonal antibodies, multispecific antibodies (*e.g.*, bispecific antibodies), and antibody fragments so long as they exhibit the desired biological activity.

An "isolated" antibody is one which has been identified and separated and/or recovered from a component of its natural environment. Contaminant components of its natural environment are materials which would interfere with research, diagnostic or therapeutic uses for the antibody, and may include enzymes, hormones, and other proteinaceous or nonproteinaceous solutes. In some embodiments, an antibody is purified (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. Isolated antibody includes the antibody *in situ* within recombinant cells since at least one component of the antibody's natural environment will not be present. Ordinarily, however, isolated antibody will be prepared by at least one purification step.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

The term "constant domain" refers to the portion of an immunoglobulin molecule having a more conserved amino acid sequence relative to the other portion of the immunoglobulin, the variable domain, which contains the antigen binding site. The constant domain contains the C_{H}1, C_{H}2 and C_{H}3 domains of the heavy chain and the CHL domain of the light chain.

The "variable region" or "variable domain" of an antibody refers to the amino-terminal domains of the heavy or light chain of the antibody. The variable domain of the heavy chain may be referred to as "V_{H}." The variable domain of the light chain may be referred to as "V_{L}." These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions (HVRs) both in the light-chain and the heavy-chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen-binding site of antibodies (see Kabat et al., Sequences of Proteins of Immunological Interest, Fifth Edition, National Institute of Health, Bethesda, MD (1991)). The constant domains are not involved directly in the binding of an antibody to an antigen, but exhibit various effector functions, such as participation of the antibody in antibody-dependent cellular toxicity.

The "light chains" of antibodies (immunoglobulins) from any vertebrate species can be assigned to one of two clearly distinct types, called kappa (κ) and lambda (λ), based on the amino acid sequences of their constant domains.

The term IgG "isotype: or "subclass" as used herein is meant any of the subclasses of immunoglobulins defined by the chemical and antigenic characteristics of their constant regions.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively. The subunit structures and three-dimensional configurations of different classes of immunoglobulins are well known and described generally in, for example, Abbas et al. Cellular and Mol. Immunology, 4th ed. (W.B. Saunders, Co., 2000). An antibody may be part of a larger fusion molecule, formed by covalent or non-covalent association of the antibody with one or more other proteins or peptides.

The term "IgG subclass modification" as used herein is meant an amino acid modification that converts one amino acid of one IgG isotype to the corresponding amino acid in a different, aligned IgG isotype. For example, because IgG₁ comprises a tyrosine and IgG₂ a phenylalanine at EU position 296, a F296Y substitution in IgG₂ is considered an IgG subclass modification.

By "non-naturally occurring modification" as used herein is meant an amino acid modification that is not isotypic. For example, because none of the IgGs comprise a glutamic acid at position 332, substitution at position 332 with glutamic acid (332E) in IgG₁, IgG₂, IgG₃, or IgG₄ is considered a non-naturally occurring modification.

The terms "full length antibody," "intact antibody" and "whole antibody" are used herein interchangeably to refer to an antibody in its substantially intact form. The terms particularly refer to an antibody with heavy chains that contain an Fc region.

A "naked antibody" for the purposes herein is an antibody that is not conjugated to a cytotoxic moiety or radiolabel.

"Antibody fragments" comprise a portion of an intact antibody, preferably comprising the antigen binding region thereof. In certain embodiments, antibody fragments comprise an Fc region or a portion of Fc region comprising one or more Fc region modification disclosed herein. Examples of antibody fragments include Fab, Fab', F(ab')₂, and Fv fragments; diabodies; linear antibodies; single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three HVRs of each variable domain interact to define an antigen-binding site on the surface of the VH-VL dimer. Collectively, the six HVRs confer antigen-binding specificity to the antibody. However, even a single variable domain (or half of an Fv comprising only three HVRs specific for an antigen) has the ability to recognize and bind antigen, although at a lower affinity than the entire binding site.

The Fab fragment contains the heavy- and light-chain variable domains and also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv, see, *e.g.,* Pluckthün, in The Pharmacology of Monoclonal Antibodies, vol. 113, Rosenburg and Moore eds., (Springer-Verlag, New York, 1994), pp. 269-315.

The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

The term "monoclonal antibody" as used herein refers to an antibody obtained from a population of substantially homogeneous antibodies, *i.e.*, the individual antibodies comprising the population are identical except for possible mutations, *e.g.*, naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target binding sequence, to improve its production in cell culture, to reduce its immunogenicity *in vivo*, to create a multispecific antibody, etc., and that an antibody comprising the altered target binding sequence is also a monoclonal antibody of this invention. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method (e.g., Kohler and Milstein, Nature, 256:495-97 (1975); Hongo et al., Hybridoma, 14 (3): 253-260 (1995), Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681 (Elsevier, N.Y., 1981)), recombinant DNA methods (see, e.g., U.S. Patent No. 4,816,567), phage-display technologies (see, e.g., Clackson et al., Nature, 352: 624-628 (1991); Marks et al., J. Mol. Biol. 222: 581-597 (1992); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004), and technologies for producing human or human-like antibodies in animals that have parts or all of the human immunoglobulin loci or genes encoding human immunoglobulin sequences (see, e.g., WO 1998/24893; WO 1996/34096; WO 1996/33735; WO 1991/10741; Jakobovits et al., Proc. Natl. Acad. Sci. USA 90: 2551 (1993); Jakobovits et al., Nature 362: 255-258 (1993); Bruggemann et al., Year in Immunol. 7:33 (1993); U.S. Patent Nos. 5,545,807; 5,545,806; 5,569,825; 5,625,126; 5,633,425; and 5,661,016; Marks et al., Bio/Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg and Huszar, Intern. Rev. Immunol. 13: 65-93 (1995).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see, *e.g.,* U.S. Patent No. 4,816,567; and Morrison et al., Proc. Natl. Acad. Sci. USA 81:6851-6855 (1984)). Chimeric antibodies include PRIMATIZED® antibodies wherein the antigen-binding region of the antibody is derived from an antibody produced by, e.g., immunizing macaque monkeys with the antigen of interest.

"Humanized" forms of non-human (*e.g.*, murine) antibodies are chimeric antibodies that contain minimal sequence derived from non-human immunoglobulin. In one embodiment, a humanized antibody is a human immunoglobulin (recipient antibody) in which residues from a HVR of the recipient are replaced by residues from a HVR of a non-human species (donor antibody) such as mouse, rat, rabbit, or nonhuman primate having the desired specificity, affinity, and/or capacity. In some instances, FR residues of the human immunoglobulin are replaced by corresponding non-human residues. Furthermore, humanized antibodies may comprise residues that are not found in the recipient antibody or in the donor antibody. These modifications may be made to further refine antibody performance. In general, a humanized antibody will comprise substantially all of at least one, and typically two, variable domains, in which all or substantially all of the hypervariable loops correspond to those of a non-human immunoglobulin, and all or substantially all of the FRs are those of a human immunoglobulin sequence. The humanized antibody optionally will also comprise at least a portion of an immunoglobulin constant region (Fc), typically that of a human immunoglobulin. For further details, see, *e.g.,* Jones et al., Nature 321:522-525 (1986); Riechmann et al., Nature 332:323-329 (1988); and Presta, Curr. Op. Struct. Biol. 2:593-596 (1992). See also, *e.g.,* Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409.

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, *e.g.*, immunized xenomice (see, *e.g.*, U.S. Pat. Nos. 6,075,181 and 6,150,584 regarding XENOMOUSE™ technology). See also, for example, Li et al., Proc. Natl. Acad. Sci. USA, 103:3557-3562 (2006) regarding human antibodies generated via a human B-cell hybridoma technology.

A "species-dependent antibody" is one which has a stronger binding affinity for an antigen from a first mammalian species than it has for a homologue of that antigen from a second mammalian species. Normally, the species-dependent antibody "binds specifically" to a human antigen (*i.e.* has a binding affinity (Kd) value of no more than about 1 x 10-7 M, preferably no more than about 1 x 10-8 M and most preferably no more than about 1 x 10-9 M) but has a binding affinity for a homologue of the antigen from a second nonhuman mammalian species which is at least about 50 fold, or at least about 500 fold, or at least about 1000 fold, weaker than its binding affinity for the human antigen. The species-dependent antibody can be any of the various types of antibodies as defined above, but preferably is a humanized or human antibody.

The term "hypervariable region," "HVR," or "HV," when used herein refers to the regions of an antibody variable domain which are hypervariable in sequence and/or form structurally defined loops. Generally, antibodies comprise six HVRs; three in the VH (HI, H2, H3), and three in the VL (L1, L2, L3). In native antibodies, H3 and L3 display the most diversity of the six HVRs, and H3 in particular is believed to play a unique role in conferring fine specificity to antibodies. See, *e.g.,* Xu et al., Immunity 13:37-45 (2000); Johnson and Wu, in Methods in Molecular Biology 248:1-25 (Lo, ed., Human Press, Totowa, NJ, 2003). Indeed, naturally occurring camelid antibodies consisting of a heavy chain only are functional and stable in the absence of light chain. See, *e.g.,* Hamers-Casterman et al., Nature 363:446-448 (1993); Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk J. Mol. Biol. 196:901-917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop Kabat ---- ----- --- | | AbM ------- | Chothia ------- | Contact |
|---|---|---|---|---|
| L1 | L24-L34 | L24-L34 | L26-L32 | L30-L36 |

| L2 | L50-L56 | L50-L56 | L50-L52 | L46-L55 |
|---|---|---|---|---|
| L3 | L89-L97 | L89-L97 | L91-L96 | L89-L96 |

| H1 | H31-H35E | H26-H35B | H26-H32 | H30-H35B |
|---|---|---|---|---|
| (Kabat Numbering) | | | | |
| H1 | H31-H35 | H26-H35 | H26-H32 | H30-H35 |

| (Chothia Numbering) | | | | |
|---|---|---|---|---|
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2) and 89-97 or 89-96 (L3) in the VL and 26-35 (HI), 50-65 or 49-65 (H2) and 93-102, 94-102, or 95-102 (H3) in the VH. The variable domain residues are numbered according to Kabat et al., supra, for each of these definitions.

"Framework" or "FR" residues are those variable domain residues other than the HVR residues as herein defined.

The term "variable domain residue numbering as in Kabat" or "amino acid position numbering as in Kabat," and variations thereof, refers to the numbering system used for heavy chain variable domains or light chain variable domains of the compilation of antibodies in Kabat et al., *supra.* Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

The Kabat numbering system is generally used when referring to a residue in the variable domain (approximately residues 1-107 of the light chain and residues 1-113 of the heavy chain) (*e.g.*, Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (*e.g.*, the EU index reported in Kabat *et al., supra*)*.* The "EU index as in Kabat" refers to the residue numbering of the human IgGi EU antibody. Unless stated otherwise herein, references to residue numbers in the variable domain of antibodies means residue numbering by the Kabat numbering system. Unless stated otherwise herein, references to residue numbers in the constant domain of antibodies means residue numbering by the EU numbering system (*see e.g.*, PCT Publication No. WO2006073941).

An "affinity matured" antibody is one with one or more alterations in one or more HVRs thereof which result in an improvement in the affinity of the antibody for antigen, compared to a parent antibody which does not possess those alteration(s). In one embodiment, an affinity matured antibody has nanomolar or even picomolar affinities for the target antigen. Affinity matured antibodies may be produced using certain procedures known in the art. For example, Marks et al. Biol/Technology 10:779-783 (1992) describes affinity maturation by VH and VL domain shuffling. Random mutagenesis of HVR and/or framework residues is described by, for example, Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "blocking" antibody or an "antagonist" antibody is one which inhibits or reduces biological activity of the antigen it binds. Certain blocking antibodies or antagonist antibodies substantially or completely inhibit the biological activity of the antigen.

An "agonist antibody," as used herein, is an antibody which partially or fully mimics at least one of the functional activities of a polypeptide of interest.

"Growth inhibitory" antibodies are those that prevent or reduce proliferation of a cell expressing an antigen to which the antibody binds.

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (*e.g.* B cell receptor); and B cell activation.

The term "Fc region" herein is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. In certain embodiments, the Fc region of an immunoglobulin comprises two constant domains, C_{H}2 and C_{H}3.

The "C_{H}2 domain" of a human IgG Fc region (also referred to as "Cγ2" domain) usually extends from about amino acid 231 to about amino acid 340. The C_{H}2 domain is unique in that it is not closely paired with another domain. Rather, two N-linked branched carbohydrate chains are interposed between the two C_{H}2 domains of an intact native IgG molecule. It has been speculated that the carbohydrate may provide a substitute for the domain-domain pairing and help stabilize the C_{H}2 domain. Burton, Molec. Immunol. 22:161-206 (1985).

The "C_{H}3 domain" comprises the stretch of residues C-terminal to a C_{H}2 domain in an Fc region (i.e. from about amino acid residue 341 to about amino acid residue 447 of an IgG).

A "functional Fc region" possesses an "effector function" of a native sequence Fc region. Exemplary "effector functions" include Fc receptor binding; C1q binding; CDC; ADCC; phagocytosis; down regulation of cell surface receptors (*e.g.* B cell receptor; BCR), *etc.* Such effector functions generally require the Fc region to be combined with a binding domain (*e.g.*, an antibody variable domain) and can be assessed using various assays.

A "native sequence Fc region" comprises an amino acid sequence identical to the amino acid sequence of an Fc region found in nature. Native sequence human Fc regions include a native sequence human IgG₁ Fc region (non-A and A allotypes); native sequence human IgG₂ Fc region; native sequence human IgG₃ Fc region; and native sequence human IgG₄ Fc region as well as naturally occurring variants thereof (*see e.g.*, SEQ ID NO: 11 to SEQ ID NO: 17).

A "variant Fc region" comprises an amino acid sequence which differs from that of a native sequence Fc region by virtue of at least one amino acid modification, In certain embodiments, variant Fc region comprises an amino acid sequence which differs from that of a native sequence Fc region by one or more amino acid substitution(s). In certain embodiments, the variant Fc region has at least one amino acid substitution compared to the Fc region of a wild-type IgG or a wild-type antibody. In certain embodiments, the variant Fc region has two or more amino acid substitutions in the Fc region of the wild-type antibody. In certain embodiments, the variant Fc region has three or more amino acid substitutions in the Fc region of the wild-type antibody. In certain embodiments, the variant Fc region has at least one, two or three or more Fc region amino acid substitutions described herein. In certain embodiments, the variant Fc region herein will possess at least about 80% homology with a native sequence Fc region and/or with an Fc region of a parent polypeptide. In certain embodiments, the variant Fc region herein will possess at least about 90% homology with a native sequence Fc region and/or with an Fc region of a parent polypeptide. In certain embodiments, the variant Fc region herein will possess at least about 95% homology with a native sequence Fc region and/or with an Fc region of a parent polypeptide.

"Fc receptor" or "FcR" describes a receptor that binds to the Fc region of an antibody. In some embodiments, an FcR is a native human FcR. In some embodiments, an FcR is one which binds an IgG antibody (a gamma receptor) and includes receptors of the FcγRI, FcγRII, and FcγRIII subclasses, including allelic variants and alternatively spliced forms of those receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcyRIIB contains an immunoreceptor tyrosine-based inhibition motif (ITIM) in its cytoplasmic domain. (see, *e.g.,* Daëron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known (see, *e.g.*, Ghetie and Ward., Immunol. Today 18(12):592-598 (1997); Ghetie et al., Nature Biotechnology, 15(7):637-640 (1997); Hinton et al., J. Biol. Chem. 279(8):6213-6216 (2004); WO 2004/92219 (Hinton et al.)*.*

The *in vivo* or serum half life of human FcRn high affinity binding polypeptides can be assayed, *e.g.*, in transgenic mice, in humans, or in non-human primates to which the polypeptides with a variant Fc region are administered. *See also*, *e.g.*, Petkova et al. International Immunology 18(12): 1759-1769 (2006).

"Human effector cells" are leukocytes which express one or more FcRs and perform effector functions. In certain embodiments, the cells express at least FcγRIII and perform ADCC effector function(s). Examples of human leukocytes which mediate ADCC include peripheral blood mononuclear cells (PBMC), natural killer (NK) cells, monocytes, cytotoxic T cells, and neutrophils. The effector cells may be isolated from a native source, e.g., from blood.

"Antibody-dependent cell-mediated cytotoxicity" or "ADCC" refers to a form of cytotoxicity in which secreted Ig bound onto Fc receptors (FcRs) present on certain cytotoxic cells (*e.g.* NK cells, neutrophils, and macrophages) enable these cytotoxic effector cells to bind specifically to an antigen-bearing target cell and subsequently kill the target cell with cytotoxins. The primary cells for mediating ADCC, NK cells, express FcγRIII only, whereas monocytes express FcγRI, FcγRII, and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 or U.S. Patent No. 6,737,056 (Presta), may be performed. Useful effector cells for such assays include PBMC and NK cells. Alternatively, or additionally, ADCC activity of the molecule of interest may be assessed *in vivo*, *e.g.*, in an animal model such as that disclosed in Clynes et al. PNAS (USA) 95:652-656 (1998).

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, *e.g.*, as described in Gazzano-Santoro et al., J. Immunol. Methods 202:163 (1996), may be performed. Polypeptide variants with altered Fc region amino acid sequences (polypeptides with a variant Fc region) and increased or decreased C1q binding capability are described, *e.g.*, in US Patent No. 6,194,551 B1 and WO 1999/51642. See also, *e.g.,* Idusogie et al. J. Immunol. 164: 4178-4184 (2000).

A polypeptide variant with "altered" FcR binding affinity or ADCC activity is one which has either enhanced or diminished FcR binding activity and/or ADCC activity compared to a parent polypeptide or to a polypeptide comprising a native sequence Fc region. The polypeptide variant which "displays increased binding" to an FcR binds at least one FcR with better affinity than the parent polypeptide. The polypeptide variant which "displays decreased binding" to an FcR, binds at least one FcR with lower affinity than a parent polypeptide. Such variants which display decreased binding to an FcR may possess little or no appreciable binding to an FcR, *e.g.*, 0-20% binding to the FcR compared to a native sequence IgG Fc region.

The polypeptide variant which "mediates antibody-dependent cell-mediated cytotoxicity (ADCC) in the presence of human effector cells more effectively" than a parent antibody is one which *in vitro* or *in vivo* is substantially more effective at mediating ADCC, when the amounts of polypeptide variant and parent antibody used in the assay are essentially the same. Generally, such variants will be identified using the *in vitro* ADCC assay as herein disclosed, but other assays or methods for determining ADCC activity, *e.g.* in an animal model *etc.*, are contemplated.

"Binding affinity" generally refers to the strength of the sum total of noncovalent interactions between a single binding site of a molecule (*e.g.*, an antibody) and its binding partner (*e.g.*, an antigen). Unless indicated otherwise, as used herein, "binding affinity" refers to intrinsic binding affinity which reflects a 1:1 interaction between members of a binding pair (*e.g.*, antibody and antigen). The affinity of a molecule X for its partner Y can generally be represented by the dissociation constant (Kd), the reciprocal of the association constant (Ka). Affinity can be measured by common methods known in the art, including those described herein. Low-affinity antibodies generally bind antigen slowly and/or tend to dissociate readily, whereas high-affinity antibodies generally bind antigen faster and/or tend to remain bound longer. A variety of methods of measuring binding affinity are known in the art, any of which can be used for purposes of the present invention. Specific illustrative and exemplary embodiments for measuring binding affinity are described in the following.

In certain embodiments, the "K_{D}," "Kd," "Kd" or "Kd value" according to this invention is measured by using surface plasmon resonance assays using a BIACC)RE®-2000 or a BIACORE®-3000 (BIAcore, Inc., Piscataway, NJ) at 25°C with immobilized antigen CM5 chips at ∼10 response units (RU). Briefly, carboxymethylated dextran biosensor chips (CM5, BIACORE, Inc.) are activated with *N*-ethyl-*N*'-(3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of antigen, 1 M ethanolamine is injected to block unreacted groups. For kinetics measurements, serial dilutions of polypeptide, *e.g.*, full length antibody, are injected in PBS with 0.05% TWEEN-20™ surfactant (PBST) at 25°C at a flow rate of approximately 25 µl/min. Association rates (kₒₙ) and dissociation rates (k_{off}) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio k_{off}/kₒₙ. *See, e.g.*, Chen et al., J. Mol. Biol. 293:865-881 (1999). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody in PBS, pH 7.2, in the presence of increasing concentrations of antigen as measured in a spectrometer, such as a stop-flow equipped spectrophometer (Aviv Instruments) or a 8000-series SLM-AMINCO™ spectrophotometer (ThermoSpectronic) with a stirred cuvette.

An "on-rate," "rate of association," "association rate," or "kₒₙ" according to this invention can also be determined as described above using a BIACORE®-2000 or a BIACORE®-3000 system (BIAcore, Inc., Piscataway, NJ).

The term "substantially similar" or "substantially the same," as used herein, denotes a sufficiently high degree of similarity between two numeric values such that one of skill in the art would consider the difference between the two values to be of little or no biological and/or statistical significance within the context of the biological characteristic measured by said values (*e.g.*, Kd values). In certain embodiments, the difference between said two values is, for example, less than about 50%, less than about 40%, less than about 30%, less than about 20%, and/or less than about 10% as a function of the reference/comparator value.

The phrase "substantially reduced," or "substantially different," as used herein, denotes a sufficiently high degree of difference between two numeric values such that one of skill in the art would consider the difference between the two values to be of statistical significance within the context of the biological characteristic measured by said values (*e.g.*, Kd values). In certain embodiments, the difference between said two values is, for example, greater than about 10%, greater than about 20%, greater than about 30%, greater than about 40%, and/or greater than about 50% as a function of the value for the reference/comparator molecule.

An "acceptor human framework" for the purposes herein is a framework comprising the amino acid sequence of a VL or VH framework derived from a human immunoglobulin framework or a human consensus framework. An acceptor human framework "derived from" a human immunoglobulin framework or a human consensus framework may comprise the same amino acid sequence thereof, or it may contain pre-existing amino acid sequence changes. In some embodiments, the number of pre-existing amino acid changes are 10 or less, 9 or less, 8 or less, 7 or less, 6 or less, 5 or less, 4 or less, 3 or less, or 2 or less. Where pre-existing amino acid changes are present in a VH, preferably those changes occur at only three, two, or one of positions 71H, 73H and 78H; for instance, the amino acid residues at those positions may be 71A, 73T and/or 78A. In one embodiment, the VL acceptor human framework is identical in sequence to the VL human immunoglobulin framework sequence or human consensus framework sequence.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., *supra.* In one embodiment, for the VL, the subgroup is subgroup kappa I as in Kabat et al., *supra.* In one embodiment, for the VH, the subgroup is subgroup III as in Kabat et al., *supra.*

A "VH subgroup III consensus framework" comprises the consensus sequence obtained from the amino acid sequences in variable heavy subgroup III of Kabat *et al.*

A "VL subgroup I consensus framework" comprises the consensus sequence obtained from the amino acid sequences in variable light kappa subgroup I of Kabat *et al.*

"Purified" means that a molecule is present in a sample at a concentration of at least 95% by weight, or at least 98% by weight of the sample in which it is contained.

An "isolated" nucleic acid molecule is a nucleic acid molecule that is separated from at least one other nucleic acid molecule with which it is ordinarily associated, for example, in its natural environment. An isolated nucleic acid molecule further includes a nucleic acid molecule contained in cells that ordinarily express the nucleic acid molecule, but the nucleic acid molecule is present extrachromosomally or at a chromosomal location that is different from its natural chromosomal location.

The term "vector," as used herein, is intended to refer to a nucleic acid molecule capable of transporting another nucleic acid to which it has been linked. One type of vector is a "plasmid," which refers to a circular double stranded DNA into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors," or simply, "expression vectors." In general, expression vectors of utility in recombinant DNA techniques are often in the form of plasmids. In the present specification, "plasmid" and "vector" may be used interchangeably as the plasmid is the most commonly used form of vector.

"Polynucleotide," or "nucleic acid," as used herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (*e.g.*, methyl phosphonates, phosphotriesters, phosphoamidates, carbamates, *etc.*) and with charged linkages (*e.g.*, phosphorothioates, phosphorodithioates, *etc.*), those containing pendant moieties, such as, for example, proteins (*e.g*., nucleases, toxins, antibodies, signal peptides, ply-L-lysine, *etc.),* those with intercalators (*e.g.*, acridine, psoralen, *etc.*), those containing chelators (*e.g.*, metals, radioactive metals, boron, oxidative metals, *etc.*), those containing alkylators, those with modified linkages (*e.g.*, alpha anomeric nucleic acids, *etc.*), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR₂ ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single-stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

As used herein, "codon set" refers to a set of different nucleotide triplet sequences used to encode desired variant amino acids. A set of oligonucleotides can be synthesized, for example, by solid phase synthesis, including sequences that represent all possible combinations of nucleotide triplets provided by the codon set and that will encode the desired group of amino acids. A standard form of codon designation is that of the IUB code, which is known in the art and described herein. A codon set typically is represented by 3 capital letters in italics, e.g., NNK, NNS, XYZ, DVK and the like. A "non-random codon set", as used herein, thus refers to a codon set that encodes select amino acids that fulfill partially, preferably completely, the criteria for amino acid selection as described herein. Synthesis of oligonucleotides with selected nucleotide "degeneracy" at certain positions is well known in that art, for example the TRIM approach (Knappek et al. (1999) J. Mol. Biol. 296:57-86); Garrard & Henner (1993) Gene 128:103). Such sets of oligonucleotides having certain codon sets can be synthesized using commercial nucleic acid synthesizers (available from, for example, Applied Biosystems, Foster City, CA), or can be obtained commercially (for example, from Life Technologies, Rockville, MD). Therefore, a set of oligonucleotides synthesized having a particular codon set will typically include a plurality of oligonucleotides with different sequences, the differences established by the codon set within the overall sequence. Oligonucleotides, as used according to the invention, have sequences that allow for hybridization to a variable domain nucleic acid template and also can, but does not necessarily, include restriction enzyme sites useful for, for example, cloning purposes.

The expression "linear antibodies" refers to the antibodies described in Zapata et al. (1995 Protein Eng, 8(10):1057-1062). Briefly, these antibodies comprise a pair of tandem Fd segments (VH-CH1-VH-CH1) which, together with complementary light chain polypeptides, form a pair of antigen binding regions. Linear antibodies can be bispecific or monospecific.

As used herein, "library" refers to a plurality of antibody or antibody fragment sequences (for example, variant IgGs of the invention), or the nucleic acids that encode these sequences, the sequences being different in the combination of variant amino acids that are introduced into these sequences according to the methods of the invention.

"Phage display" is a technique by which polypeptides are displayed as fusion proteins to at least a portion of coat protein on the surface of phage, *e.g.*, filamentous phage, particles. A utility of phage display lies in the fact that large libraries of randomized protein variants can be rapidly and efficiently sorted for those sequences that bind to a target antigen with high affinity. Display of peptide and protein libraries on phage has been used for screening millions of polypeptides for ones with specific binding properties. Polyvalent phage display methods have been used for displaying small random peptides and small proteins through fusions to either gene III or gene VIII of filamentous phage. Wells and Lowman (1992) Curr. Opin. Struct. Biol. 3:355-362, and references cited therein. In a monovalent phage display, a protein or peptide library is fused to a gene III or a portion thereof, and expressed at low levels in the presence of wild type gene III protein so that phage particles display one copy or none of the fusion proteins. Avidity effects are reduced relative to polyvalent phage so that sorting is on the basis of intrinsic ligand affinity, and phagemid vectors are used, which simplify DNA manipulations. Lowman and Wells (1991) Methods: A companion to Methods in Enzymology 3:205-0216.

A "phagemid" is a plasmid vector having a bacterial origin of replication, e.g., Co1E1, and a copy of an intergenic region of a bacteriophage. The phagemid may be used on any known bacteriophage, including filamentous bacteriophage and lambdoid bacteriophage. The plasmid will also generally contain a selectable marker for antibiotic resistance. Segments of DNA cloned into these vectors can be propagated as plasmids. When cells harboring these vectors are provided with all genes necessary for the production of phage particles, the mode of replication of the plasmid changes to rolling circle replication to generate copies of one strand of the plasmid DNA and package phage particles. The phagemid may form infectious or non-infectious phage particles. This term includes phagemids which contain a phage coat protein gene or fragment thereof linked to a heterologous polypeptide gene as a gene fusion such that the heterologous polypeptide is displayed on the surface of the phage particle.

The term "phage vector" means a double stranded replicative form of a bacteriophage containing a heterologous gene and capable of replication. The phage vector has a phage origin of replication allowing phage replication and phage particle formation. The phage is preferably a filamentous bacteriophage, such as an M13, f1, fd, Pf3 phage or a derivative thereof, or a lambdoid phage, such as lambda, 21, phi80, phi81, 82, 424, 434, etc., or a derivative thereof.

As used herein, "solvent accessible position" refers to a position of an amino acid residue in the variable regions of the heavy and light chains of a source antibody or antigen binding fragment that is determined, based on structure, ensemble of structures and/or modeled structure of the antibody or antigen binding fragment, as potentially available for solvent access and/or contact with a molecule, such as an antibody-specific antigen. These positions are typically found in the CDRs and on the exterior of the protein. The solvent accessible positions of an antibody or antigen binding fragment, as defined herein, can be determined using any of a number of algorithms known in the art. In one embodiment, solvent accessible positions are determined using coordinates from a 3-dimensional model of an antibody, preferably using a computer program such as the InsightII program (Accelrys, San Diego, CA). Solvent accessible positions can also be determined using algorithms known in the art (*e.g.*, Lee and Richards (1971) J. Mol. Biol. 55, 379 and Connolly (1983) J. Appl. Cryst. 16, 548). Determination of solvent accessible positions can be performed using software suitable for protein modeling and 3-dimensional structural information obtained from an antibody. Software that can be utilized for these purposes includes SYBYL Biopolymer Module software (Tripos Associates). Generally, where an algorithm (program) requires a user input size parameter, the "size" of a probe which is used in the calculation is set at about 1.4 Angstrom or smaller in radius. In addition, determination of solvent accessible regions and area methods using software for personal computers has been described by Pacios (1994) Comput. Chem. 18(4): 377-386.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "VEGF" or "VEGF-A" as used herein refers to the 165-amino acid human vascular endothelial cell growth factor and related 121-, 189-, and 206- amino acid human vascular endothelial cell growth factors, as described by Leung et al. (1989) Science 246:1306, and Houck et al. (1991) Mol. Endocrin, 5:1806, together with the naturally occurring allelic and processed forms thereof. The term "VEGF" also refers to VEGFs from non-human species such as mouse, rat or primate. Sometimes the VEGF from a specific species are indicated by terms such as hVEGF for human VEGF, mVEGF for murine VEGF, and *etc.* The term "VEGF" is also used to refer to truncated forms of the polypeptide comprising amino acids 8 to 109 or 1 to 109 of the 165-amino acid human vascular endothelial cell growth factor. Reference to any such forms of VEGF may be identified in the present application, *e.g.*, by "VEGF (8-109)," "VEGF (1-109)," "VEGF-A₁₀₉" or "VEGF 165." The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF.

A "VEGF antagonist" refers to a molecule capable of neutralizing, blocking, inhibiting, abrogating, reducing or interfering with VEGF activities including, but not limited to, its binding to one or more VEGF receptors. VEGF antagonists include, without limitation, anti-VEGF antibodies and antigen-binding fragments thereof, receptor molecules and derivatives which bind specifically to VEGF thereby sequestering its binding to one or more receptors, anti-VEGF receptor antibodies, VEGF receptor antagonists such as small molecule inhibitors of the VEGFR tyrosine kinases and immunoadhesins that binds to VEGF such as VEGF Trap. The term "VEGF antagonist," as used herein, specifically includes molecules, including antibodies, antibody fragments, other binding polypeptides, peptides, and non-peptide small molecules, that bind to VEGF and are capable of neutralizing, blocking, inhibiting, abrogating, reducing or interfering with VEGF activities. Thus, the term "VEGF activities" specifically includes VEGF mediated biological activities of VEGF.

The terms "biological activity" and "biologically active" with regard to VEGF polypeptide or "VEGF activity" refer to physical/chemical properties and biological functions associated with full-length and/or truncated VEGF. In certain embodiments, VEGF activity is inducing and/or stimulating and/or promoting angiogenesis. In certain embodiments, VEGF activity is inducing and/or stimulating and/or promoting neovascularization. In certain embodiments, VEGF activity is inducing and/or modulating vascular permeability. In certain embodiments, VEGF activity is inducing and/or stimulating and/or promoting endothelial cell migration and/or endothelial cell proliferation.

Anti-VEGF neutralizing antibodies suppress the growth of a variety of human tumor cell lines in nude mice (Kim et al., Nature 362:841-844 (1993); Warren et al., J. Clin. Invest. 95:1789-1797 (1995); Borgström et al., Cancer Res. 56:4032-4039 (1996); Melnyk et al., Cancer Res. 56:921-924 (1996)) and also inhibit intraocular angiogenesis in models of ischemic retinal disorders. Adamis et al., Arch. Ophthalmol. 114:66-71 (1996).

The term "anti-VEGF antibody" or "an antibody that binds to VEGF" refers to an antibody that is capable of binding to VEGF with sufficient affinity and specificity that the antibody is useful as a diagnostic and/or therapeutic agent in targeting VEGF. For example, the anti-VEGF antibody of the invention can be used as a therapeutic agent in targeting and interfering with diseases or conditions wherein the VEGF activity is involved. *See, e.g.,* U.S. Patents 6,582,959, 6,703,020; WO98/45332; WO 96/30046; WO94/10202, WO2005/044853; ; EP 0666868B1; US Patent Applications 20030206899, 20030190317, 20030203409, 20050112126, 20050186208, and 20050112126; Popkov et al., Journal of Immunological Methods 288:149-164 (2004); and WO2005012359. The antibody selected will normally have a sufficiently strong binding affinity for VEGF. For example, the antibody may bind hVEGF with a K_{d} value of between 100 nM-1 pM. Antibody affinities may be determined by a surface plasmon resonance based assay (such as the BIAcore assay as described in PCT Application Publication No. WO2005/012359); enzyme-linked immunoabsorbent assay (ELISA); and competition assays (e.g. RIA's), for example. The antibody may be subjected to other biological activity assays, *e.g.*, in order to evaluate its effectiveness as a therapeutic. Such assays are known in the art and depend on the target antigen and intended use for the antibody. Examples include the HUVEC inhibition assay; tumor cell growth inhibition assays (as described in WO 89/06692, for example); antibody-dependent cellular cytotoxicity (ADCC) and complement-mediated cytotoxicity (CDC) assays (US Patent 5,500,362); and agonistic activity or hematopoiesis assays (see WO 95/27062). An anti-VEGF antibody will usually not bind to other VEGF homologues such as VEGF-B, VEGF-C, VEGF-D or VEGF-E, nor other growth factors such as PlGF, PDGF or bFGF. In one embodiment, anti-VEGF antibodies include a monoclonal antibody that binds to the same epitope as the monoclonal anti-VEGF antibody A4.6.1 produced by hybridoma ATCC HB 10709; a recombinant humanized anti-VEGF monoclonal antibody (*see* Presta et al. (1997) Cancer Res. 57:4593-4599), including but not limited to the antibody known as "bevacizumab (BV)," also known as "rhuMAb VEGF" or "AVASTIN®." AVASTIN® is presently commercially available. Bevacizumab comprises mutated human IgGi framework regions and antigen-binding complementarity-determining regions from the murine antibody A.4.6.1 that blocks binding of human VEGF to its receptors. Approximately 93% of the amino acid sequence of bevacizumab, including most of the framework regions, is derived from human IgG₁, and about 7% of the sequence is derived from A4.6.1. Bevacizumab has a molecular mass of about 149,000 daltons and is glycosylated. Bevacizumab and other humanized anti-VEGF antibodies are further described in U.S. Pat. No. 6,884,879, issued February 26, 2005. Additional anti-VEGF antibodies include the G6 or B20 series antibodies (*e.g.*, G6-23, G6-31, B20-4.1), as described in PCT Application Publication No. WO2005/012359. For additional preferred antibodies see U.S. Pat. Nos. 7,060,269, 6,582,959, 6,703,020; 6,054,297; WO98/45332; WO 96/30046; WO94/10202; EP 0666868B1; U.S. Patent Application Publication Nos. 2006009360, 20050186208, 20030206899, 20030190317, 20030203409, and 20050112126; and Popkov et al., Journal of Immunological Methods 288:149-164 (2004).

The term "B20 series polypeptide" as used herein refers to a polypeptide, including an antibody that binds to VEGF. B20 series polypeptides includes, but not limited to, antibodies derived from a sequence of the B20 antibody or a B20-derived antibody described in US Publication No. 20060280747, US Publication No. 20070141065 and/or US Publication No. 20070020267. In one embodiment, B20 series polypeptide is B20-4.1 as described in US Publication No. 20060280747, US Publication No. 20070141065 and/or US Publication No. 20070020267. In another embodiment, B20 series polypeptide is B20-4.1.1 described in PCT Publication No. WO 2009/073160.

The term "G6 series polypeptide" as used herein refers to a polypeptide, including an antibody that binds to VEGF. G6 series polypeptides includes, but not limited to, antibodies derived from a sequence of the G6 antibody or a G6-derived antibody described in US Publication No. 20060280747, US Publication No. 20070141065 and/or US Publication No. 20070020267. G6 series polypeptides, as described in US Publication No. 20060280747, US Publication No. 20070141065 and/or US Publication No. 20070020267 include, but not limited to, G6-8, G6-23 and G6-31.

An "angiogenic factor or agent" is a growth factor which stimulates the development of blood vessels, *e.g.*, promote angiogenesis, endothelial cell growth, stabiliy of blood vessels, and/or vasculogenesis, *etc.* For example, angiogenic factors, include, but are not limited to, *e.g.,* VEGF and members of the VEGF family (VEGF-B, VEGF-C and VEGF-D), PlGF, PDGF family, fibroblast growth factor family (FGFs), TIE ligands (Angiopoietins), ephrins, delta-like ligand 4 (DLL4), Del-1, fibroblast growth factors: acidic (aFGF) and basic (bFGF), follistatin, granulocyte colony-stimulating factor (G-CSF), hepatocyte growth factor (HGF) /scatter factor (SF), Interleukin-8 (IL-8), leptin, midkine, neuropilins, placental growth factor, platelet-derived endothelial cell growth factor (PD-ECGF), platelet-derived growth factor, especially PDGF-BB or PDGFR-beta, pleiotrophin (PTN), progranulin, proliferin, transforming growth factor-alpha (TGF-alpha), transforming growth factor-beta (TGF-beta), tumor necrosis factor-alpha (TNF-alpha), etc. It would also include factors that accelerate wound healing, such as growth hormone, insulin-like growth factor-I (IGF-I), VIGF, epidermal growth factor (EGF), CTGF and members of its family, and TGF-alpha and TGF-beta. *See, e.g.*, Klagsbrun and D'Amore (1991) Annu. Rev. Physiol. 53:217-39; Streit and Detmar (2003) Oncogene 22:3172-3179; Ferrara & Alitalo (1999) Nature Medicine 5(12):1359-1364; Tonini et al. (2003) Oncogene 22:6549-6556 *(e.g.,* Table 1 listing known angiogenic factors); and, Sato (2003) Int. J. Clin. Oncol. 8:200-206.

An "anti-angiogenesis agent" or "angiogenesis inhibitor" refers to a small molecular weight substance, a polynucleotide (including, *e.g.*, an inhibitory RNA (RNAi or siRNA)), a polypeptide, an isolated protein, a recombinant protein, an antibody, or conjugates or fusion proteins thereof, that inhibits angiogenesis, vasculogenesis, or undesirable vascular permeability, either directly or indirectly. It should be understood that the anti-angiogenesis agent includes those agents that bind and block the angiogenic activity of the angiogenic factor or its receptor. For example, an anti-angiogenesis agent is an antibody or other antagonist to an angiogenic agent as defined above, *e.g.,* antibodies to VEGF-A or to the VEGF-A receptor (*e.g.*, KDR receptor or Flt-1 receptor), anti-PDGFR inhibitors such as GLEEVEC® (Imatinib Mesylate), small molecules that block VEGF receptor signaling (*e.g.*, PTK787/ZK2284, SU6668, SUTENT®/SU11248 (sunitinib malate), AMG706, or those described in, *e.g.*, international patent application WO 2004/113304). Anti-angiogensis agents also include native angiogenesis inhibitors , *e.g.*, angiostatin, endostatin, *etc. See, e.g.*, Klagsbrun and D'Amore (1991) Annu. Rev. Physiol. 53:217-39; Streit and Detmar (2003) Oncogene 22:3172-3179 *(e.g.,* Table 3 listing anti-angiogenic therapy in malignant melanoma); Ferrara & Alitalo (1999) Nature Medicine 5(12):1359-1364; Tonini et al. (2003) Oncogene 22:6549-6556 *(e.g.,* Table 2 listing known anti-angiogenic factors); and, Sato (2003) Int. J. Clin. dOncol. 8:200-206 *(e.g.,* Table 1 listing anti-angiogenic agents used in clinical trials).

A "disorder" is any condition or disease that would benefit from treatment with a composition or method of the invention. This includes chronic and acute disorders or diseases including those pathological conditions which predispose the mammal to the disorder in question. Non-limiting examples of disorders that can be treated using the antibodies and antibody fragments of the invention include various diseases and disorders provided herein under "Definitions."

The terms "cell proliferative disorder" and "proliferative disorder" refer to disorders that are associated with some degree of abnormal cell proliferation. In one embodiment, the cell proliferative disorder is cancer.

"Tumor," as used herein, refers to all neoplastic cell growth and proliferation, whether malignant or benign, and all pre-cancerous and cancerous cells and tissues. The terms "cancer", "cancerous", "cell proliferative disorder", "proliferative disorder" and "tumor" are not mutually exclusive as referred to herein.

The tumor can be a solid tumor or a non-solid or soft tissue tumor. Examples of soft tissue tumors include leukemia (*e.g.*, chronic myelogenous leukemia, acute myelogenous leukemia, adult acute lymphoblastic leukemia, acute myelogenous leukemia, mature B-cell acute lymphoblastic leukemia, chronic lymphocytic leukemia, polymphocytic leukemia, or hairy cell leukemia), or lymphoma (*e.g.*, non-Hodgkin's lymphoma, cutaneous T-cell lymphoma, or Hodgkin's disease). A solid tumor includes any cancer of body tissues other than blood, bone marrow, or the lymphatic system. Solid tumors can be further separated into those of epithelial cell origin and those of non-epithelial cell origin. Examples of solid tumors include tumors of colon, breast, prostate, lung, kidney, liver, pancreas, ovary, head and neck, oral cavity, stomach, duodenum, small intestine, large intestine, gastrointestinal tract, anus, gall bladder, labium, nasopharynx, skin, uterus, male genital organ, urinary organs, bladder, and skin. Solid tumors of non-epithelial origin include sarcomas, brain tumors, and bone tumors.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia or lymphoid malignancies. More particular examples of such cancers include, but not limited to, squamous cell cancer (*e.g*., epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer and gastrointestinal stromal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, superficial spreading melanoma, lentigo maligna melanoma, acral lentiginous melanomas, nodular melanomas, multiple myeloma and B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); hairy cell leukemia; chronic myeloblastic leukemia; and post-transplant lymphoproliferative disorder (PTLD), as well as abnormal vascular proliferation associated with phakomatoses, edema (such as that associated with brain tumors), Meigs' syndrome, brain, as well as head and neck cancer, and associated metastases. In certain embodiments, cancers that are amenable to treatment by the variant IgGs of the invention include breast cancer, colorectal cancer, rectal cancer, non-small cell lung cancer, glioblastoma, non-Hodgkins lymphoma (NHL), renal cell cancer, prostate cancer, liver cancer, pancreatic cancer, soft-tissue sarcoma, kaposi's sarcoma, carcinoid carcinoma, head and neck cancer, ovarian cancer, mesothelioma, and multiple myeloma. In some embodiments, the cancer is selected from the group consisting of small cell lung cancer, gliblastoma, neuroblastomas, melanoma, breast carcinoma, gastric cancer, colorectal cancer (CRC), and hepatocellular carcinoma. Yet, in some embodiments, the cancer is selected from the group consisting of non-small cell lung cancer, colorectal cancer, glioblastoma and breast carcinoma, including metastatic forms of those cancers.

The term cancer embraces a collection of proliferative disorders, including but not limited to pre-cancerous growths, benign tumors, malignant tumors and dormant tumors. Benign tumors remain localized at the site of origin and do not have the capacity to infiltrate, invade, or metastasize to distant sites. Malignant tumors will invade and damage other tissues around them. They can also gain the ability to break off from where they started and spread to other parts of the body (metastasize), usually through the bloodstream or through the lymphatic system where the lymph nodes are located. Dormant tumors are quiescent tumors in which tumor cells are present but tumor progression is not clinically apparent. Primary tumors are classified by the type of tissue from which they arise; metastatic tumors are classified by the tissue type from which the cancer cells are derived. Over time, the cells of a malignant tumor become more abnormal and appear less like normal cells. This change in the appearance of cancer cells is called the tumor grade and cancer cells are described as being well-differentiated, moderately-differentiated, poorly-differentiated, or undifferentiated. Well-differentiated cells are quite normal appearing and resemble the normal cells from which they originated. Undifferentiated cells are cells that have become so abnormal that it is no longer possible to determine the origin of the cells.

Epithelial cancers generally evolve from a benign tumor to a preinvasive stage (*e.g.,* carcinoma *in situ*), to a malignant cancer, which has penetrated the basement membrane and invaded the subepithelial stroma.

By "dysplasia" is meant any abnormal growth or development of tissue, organ, or cells. In certain embodiments, the dysplasia is high grade or precancerous.

By "metastasis" is meant the spread of cancer from its primary site to other places in the body. Cancer cells can break away from a primary tumor, penetrate into lymphatic and blood vessels, circulate through the bloodstream, and grow in a distant focus (metastasize) in normal tissues elsewhere in the body. Metastasis can be local or distant. Metastasis is a sequential process, contingent on tumor cells breaking off from the primary tumor, traveling through the bloodstream, and stopping at a distant site. At the new site, the cells establish a blood supply and can grow to form a life-threatening mass. Both stimulatory and inhibitory molecular pathways within the tumor cell regulate this behavior, and interactions between the tumor cell and host cells in the distant site are also significant.

By "micrometastasis" is meant a small number of cells that have spread from the primary tumor to other parts of the body. Micrometastasis may or may not be detected in a screening or diagnostic test.

By "non-metastatic" is meant a cancer that is benign or that remains at the primary site and has not penetrated into the lymphatic or blood vessel system or to tissues other than the primary site. Generally, a non-metastatic cancer is any cancer that is a Stage 0, I, or II cancer, and occasionally a Stage III cancer.

Reference to a tumor or cancer as a "Stage 0," "Stage I," "Stage II," "Stage III," or "Stage IV" indicates classification of the tumor or cancer using the Overall Stage Grouping or Roman Numeral Staging methods known in the art. Although the actual stage of the cancer is dependent on the type of cancer, in general, a Stage 0 cancer is an in situ lesion, a Stage I cancer is small localized tumor, a Stage II and III cancer is a local advanced tumor which exhibits involvement of the local lymph nodes, and a Stage IV cancer represents metastatic cancer. The specific stages for each type of tumor is known to the skilled clinician.

By "primary tumor" or "primary cancer" is meant the original cancer and not a metastatic lesion located in another tissue, organ, or location in the subject's body.

By "benign tumor" or "benign cancer" is meant a tumor that remains localized at the site of origin and does not have the capacity to infiltrate, invade, or metastasize to a distant site.

"Cancer recurrence" herein refers to a return of cancer following treatment, and includes return of cancer in the primary organ, as well as distant recurrence, where the cancer returns outside of the primary organ.

By "tumor dormancy" is meant a prolonged quiescent state in which tumor cells are present but tumor progression is not clinically apparent. A dormant tumor may or may not be detected in a screening or diagnostic test.

By "tumor burden" is meant the number of cancer cells, the size of a tumor, or the amount of cancer in the body. Tumor burden is also referred to as tumor load.

By "tumor number" is meant the number of tumors.

Non-neoplastic conditions that are amenable to treatment with antibodies and antibody fragments of the invention include, but are not limited to, *e.g*., undesired or aberrant hypertrophy, benign prostatic hypertrophy, arthritis, rheumatoid arthritis (RA), psoriatic arthritis, neurodegenerative diseases (*e.g*. Alzheimer's disease, AIDS-related dementia, Parkinson's disease, amyotrophic lateral sclerosis, retinitis pigmentosa, spinal muscular atrophy and cerebellar degeneration), autoimmune disease, psoriasis, psoriatic plaques, sarcoidosis, atherosclerosis, atherosclerotic plaques, Hashimoto's thyroiditis, angiogenic disorders, ocular disease such as presumed ocular histoplasmosis syndrome, retinal vascularization, diabetic and other proliferative retinopathies including retinopathy of prematurity, diabetic nephropathy, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular disease, conditions involving abnormal proliferation of vascular epithelial cells, vascular restenosis, Guillain-Barre Syndrome, polyps such as colon polyps, familial adenomatosis polyposis, nasal polyps or gastrointestinal polyps, gastrointestinal ulcers, infantile hypertrophic pyloric stenosis, urinary obstructive syndrome, Menetrier's disease, secreting adenomas or protein loss syndrome, fibroadenoma, respiratory disease, cholecystitis, neurofibromatosis, arteriovenous malformations (AVM), meningioma, hemangioma, angiofibroma, thyroid hyperplasias (including Grave's disease), corneal and other tissue transplantation, inflammatory diseases, chronic inflammation, lung inflammation, acute lung injury/ARDS, sepsis, chronic occlusive pulmonary disease, primary pulmonary hypertension, malignant pulmonary effusions, atheroma, edema following burns, trauma, radiation, stroke, hypoxia or ischemia, edema from myocardial infarction, ischemic injury, damage following a cerebral ischemic event, cerebral edema (*e.g.,* associated with acute stroke/ closed head injury/ trauma), thrombus caused by platelet aggregation. fibrotic or edemia diseases such as hepatic cirrhosis, lung fibrosis, carcoidosis, throiditis, hyperviscosity syndrome systemic, synovial inflammation, pannus formation in RA, myositis ossificans, hypertropic bone formation, bone associated pathologies such as osteoarthritis, rickets and osteoporosis, refractory ascites, bone or joint inflammation, Myelodysplastic Syndrome, aplastic anemia, kidney or liver; T-cell mediated hypersensitivity disease, Paget's disease, polycystic kidney disease, 3rd spacing of fluid diseases (pancreatitis, compartment syndrome, burns, bowel disease), chronic inflammation such as IBD (Crohn's disease and ulcerative colitis), renal disorders, renal allograft rejection, graft versus host disease or transplant rejection, inflammatory bowel disease, acute and chronic nephropathies (including proliferative glomerulonephritis and diabetes-induced renal disease), nephrotic syndrome, undesired or aberrant tissue mass growth (non-cancer), obesity, adipose tissue mass growth, hemophilic joints, hypertrophic scars, inhibition of hair growth, Osler Weber-Rendu Syndrome, pyogenic granuloma retrolental fibroplasias, scleroderma, trachoma, vascular adhesions, synovitis, hypersensitivity reaction of the skin, skin disorders including psoriasis and dermatitis, eczema, photoaging (*e.g*. caused by UV radiation of human skin), hypertrophic scar formation, reproductive conditions such as endometriosis, ovarian hyperstimulation syndrome, polycystic ovarian disease, preeclampsia, dysfunctional uterine bleeding, or menometrorrhagia, uterine fibroids, premature labor, ascites, pericardial effusion (such as that associated with pericarditis), pleural effusion, endotoxic shock and fungal infection, certain microbial infections including microbial pathogens selected from adenovirus, hantaviruses, Borrelia burgdorferi, Yersinia spp., Bordetella pertussis and psychiatric disorders (*e.g*. schizophrenia, bipolar depression, autism, and attention deficit disorder).

A "respiratory disease" involves the respiratory system and includes chronic bronchitis, asthma including acute asthma and allergic asthma, cystic fibrosis, bronchiectasis, allergic or other rhinitis or sinusitis, .alpha. 1-antitrypsin deficiency, coughs, pulmonary emphysema, pulmonary fibrosis or hyper-reactive airways, chronic obstructive pulmonary disease, and chronic obstructive lung disorder.

An "autoimmune disease" herein is a non-malignant disease or disorder arising from and directed against an individual's own tissues. Examples of autoimmune diseases or disorders include, but are not limited to, inflammatory responses such as inflammatory skin diseases including psoriasis and dermatitis (e.g. atopic dermatitis and contact dermatitis); systemic scleroderma and sclerosis; responses associated with inflammatory bowel disease (such as Crohn's disease and ulcerative colitis); respiratory distress syndrome (including adult respiratory distress syndrome; ARDS); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus (e.g. Type I diabetes mellitus or insulin dependent diabetes mellitis); multiple sclerosis; Reynaud's syndrome; autoimmune thyroiditis; allergic encephalomyelitis; Sjorgen's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia (including, but not limited to cryoglobinemia or Coombs positive anemia); myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia etc.

The term "vascular disease or disorder" herein refers to the various diseases or disorders which impact the vascular system, including the cardiovascular system. Examples of such diseases include arteriosclerosis, vascular reobstruction, atherosclerosis, postsurgical vascular stenosis, restenosis, vascular occlusion or carotid obstructive disease, coronary artery disease, angina, small vessel disease, hypercholesterolemia, hypertension, and conditions involving abnormal proliferation or function of vascular epithelial cells.

As used herein, "treatment" (and variations such as "treat" or "treating") refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, variant IgGs of the invention are used to delay development of a disease or disorder or to slow the progression of a disease or disorder.

An "individual," "subject," or "patient" is a vertebrate. In certain embodiments, the vertebrate is a mammal. Mammals include, but are not limited to, farm animals (such as cows), sport animals, pets (such as cats, dogs, and horses), primates, mice and rats. In certain embodiments, a mammal is a human.

The term "pharmaceutical formulation" or "pharmaceutical composition" refers to a preparation which is in such form as to permit the biological activity of the active ingredient to be effective, and which contains no additional components which are unacceptably toxic to a subject to which the formulation would be administered. Such formulations may be sterile. *See also* section entitled Dosages, Formulations, and Duration.

A "sterile" formulation is aseptic or free from all living microorganisms and their spores.

The term "effective amount" or "therapeutically effective amount" refers to an amount of a drug effective to treat a disease or disorder in a subject. In certain embodiments, an effective amount refers to an amount effective, at dosages and for periods of time necessary, to achieve the desired therapeutic or prophylactic result. A therapeutically effective amount of a substance/molecule of the invention may vary according to factors such as the disease state, age, sex, and weight of the individual, and the ability of the substance/molecule, to elicit a desired response in the individual. A therapeutically effective amount encompasses an amount in which any toxic or detrimental effects of the substance/molecule are outweighed by the therapeutically beneficial effects. In the case of cancer, the effective amount of the drug may reduce the number of cancer cells; reduce the tumor size; inhibit (*i.e.,* slow to some extent and typically stop) cancer cell infiltration into peripheral organs; inhibit (*i.e.,* slow to some extent and typically stop) tumor metastasis; inhibit, to some extent, tumor growth; allow for treatment of the tumor, and/or relieve to some extent one or more of the symptoms associated with the disorder. To the extent the drug may prevent growth and/or kill existing cancer cells, it may be cytostatic and/or cytotoxic.

A "prophylactically effective amount" refers to an amount effective, at dosages and for periods of time necessary, to achieve the desired prophylactic result. Typically, but not necessarily, since a prophylactic dose is used in subjects prior to or at an earlier stage of disease, the prophylactically effective amount would be less than the therapeutically effective amount.

In the case of pre-cancerous, benign, early or late-stage tumors, the therapeutically effective amount of the angiogenic inhibitor may reduce the number of cancer cells; reduce the primary tumor size; inhibit (*i.e.,* slow to some extent and preferably stop) cancer cell infiltration into peripheral organs; inhibit (*i.e.,* slow to some extent and preferably stop) tumor metastasis; inhibit or delay, to some extent, tumor growth or tumor progression; and/or relieve to some extent one or more of the symptoms associated with the disorder. To the extent the drug may prevent growth and/or kill existing cancer cells, it may be cytostatic and/or cytotoxic.

The term "efficacy" is used herein in the broadest sense and refers to immunoglobuin's, antibody's or Fc fusion protein's ability to produce a desired effect. In certain embodiments, efficacy refers to the maximal observed effect of an immunoglobulin, antibody or Fc fusion protein at saturating levels. In certain embodiments, efficacy refers to the ECso of an immunoglobulin, antibody or Fc fusion protein. In certain embodiments, efficacy refers to the potency of an immunoglobulin, antibody or Fc fusion protein. In certain embodiments, efficacy refers to immunoglobulin's, antibody's or Fc fusion protein's ability to produce beneficial effects on the course or duration of a disease, including clinical benefit as defined herein.

The term "EC₅₀" refers to the concentration of an immunoglobulin, antibody or Fc fusion protein which induces a response halfway between the baseline and maximum. In certain embodiments, ECso represents the concentration of an immunoglobulin, antibody or Fc fusion protein where 50% of its maximal effect is observed. In certain embodiments, EC₅₀ represents the plasma or serum concentration required for obtaining 50% of the maximum effect *in vivo.*

Efficacy in treating cancer may be demonstrated by detecting the ability of an antibody, a fusion protein, a conjugated molecule, or a composition of the invention to inhibit or reduce the growth or metastasis of cancerous cells or to ameliorate or alleviate one or more symptoms associated with cancer. The treatment is considered therapeutic if there is, for example, a reduction in the growth or metastasis of cancerous cells, amelioration of one or more symptoms associated with cancer, or a decrease in mortality and/or morbidity following administration of an antibody, a fusion protein, a conjugated molecule, or a composition of the invention. Antibodies, fusion proteins or compositions of the invention can be tested for their ability to reduce tumor formation in *in vitro, ex vivo,* and *in vivo* assays. For cancer therapy, efficacy *in vivo* can, for example, be also measured by assessing the duration of survival, time to disease progression (TTP), the response rates (RR), duration of response, and/or quality of life. *See also* section entitled Efficacy of the Treatment.

Efficacy in treating inflammatory disorders may be demonstrated by detecting the ability of an antibody, a fusion protein, a conjugated molecule, or a composition of the invention to reduce or inhibit the inflammation in an animal or to ameliorate or alleviate one or more symptoms associated with an inflammatory disorder. The treatment is considered therapeutic if there is, for example, a reduction is in inflammation or amelioration of one or more symptoms following administration of an antibody, a fusion protein, a conjugated molecule, or a composition of the invention.

Efficacy in treating or preventing viral infection may be demonstrated by detecting the ability of an antibody, a fusion protein, a conjugated molecule, or a composition of the invention to inhibit the replication of the virus, to inhibit transmission or prevent the virus from establishing itself in its host, or to prevent, ameliorate or alleviate one or more symptoms associated with viral infection. The treatment is considered therapeutic if there is, for example, a reduction is viral load, amelioration of one or more symptoms or a decrease in mortality and/or morbidity following administration of an antibody, a fusion protein, a conjugated molecule, or a composition of the invention. Antibodies, fusion proteins, conjugated molecules, or compositions of the invention can also be tested for their ability to inhibit viral replication or reduce viral load in *in vitro* and *in vivo* assays.

Efficacy in treating or preventing bacterial infection may be demonstrated by detecting the ability of an antibody, a fusion protein or a composition of the invention to inhibit the bacterial replication, or to prevent, ameliorate or alleviate one or more symptoms associated with bacterial infection. The treatment is considered therapeutic if there is, for example, a reduction is bacterial numbers, amelioration of one or more symptoms or a decrease in mortality and/or morbidity following administration of an antibody, a fusion protein or a composition of the invention.

Clinical benefit can be measured by assessing various endpoints, *e.g*., inhibition, to some extent, of disease progression, including slowing down and complete arrest; reduction in the number of disease episodes and/or symptoms; reduction in lesion size; inhibition (*i.e.,* reduction, slowing down or complete stopping) of disease cell infiltration into adjacent peripheral organs and/or tissues; inhibition (*i.e.* reduction, slowing down or complete stopping) of disease spread; decrease of auto-immune response, which may, but does not have to, result in the regression or ablation of the disease lesion; relief, to some extent, of one or more symptoms associated with the disorder; increase in the length of disease-free presentation following treatment, *e.g*., progression-free survival; increased overall survival; higher response rate; and/or decreased mortality at a given point of time following treatment.

To "reduce or inhibit" is to decrease or reduce an activity, function, and/or amount as compared to a reference. In certain embodiments, by "reduce or inhibit" is meant the ability to cause an overall decrease of 20% or greater. In another embodiment, by "reduce or inhibit" is meant the ability to cause an overall decrease of 50% or greater. In yet another embodiment, by "reduce or inhibit" is meant the ability to cause an overall decrease of 75%, 85%, 90%, 95%, or greater. Reduce or inhibit can refer to the symptoms of the disorder being treated, the presence or size of metastases, the size of the primary tumor, or the size or number of the blood vessels in angiogenic disorders.

"Operable" cancer is cancer which is confined to the primary organ and suitable for surgery.

"Survival" refers to the patient remaining alive, and includes disease free survival (DFS), progression free survival (PFS) and overall survival (OS). Survival can be estimated by the Kaplan-Meier method, and any differences in survival are computed using the stratified log-rank test.

"Disease free survival (DFS)" refers to the patient remaining alive, without return of the cancer, for a defined period of time such as about 1 year, about 2 years, about 3 years, about 4 years, about 5 years, about 10 years, *etc.,* from initiation of treatment or from initial diagnosis. In one aspect of the invention, DFS is analyzed according to the intent-to-treat principle, i.e., patients are evaluated on the basis of their assigned therapy. The events used in the analysis of DFS can include local, regional and distant recurrence of cancer, occurrence of secondary cancer, and death from any cause in patients without a prior event (*e.g*., breast cancer recurrence or second primary cancer).

"Overall survival" refers to the patient remaining alive for a defined period of time, such as about 1 year, about 2 years, about 3 years, about 4 years, about 5 years, about 10 years, *etc.,* from initiation of treatment or from initial diagnosis.

By "extending survival" is meant increasing DFS and/or OS in a treated patient relative to an untreated patient, or relative to a control treatment protocol, such as treatment only with the chemotherapeutic agent. Survival is monitored for at least about six months, or at least about 1 year, or at least about 2 years, or at least about 3 years, or at least about 4 years, or at least about 5 years, or at least about 10 years, *etc.,* following the initiation of treatment or following the initial diagnosis.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

By "monotherapy" is meant a therapeutic regimen that includes only a single therapeutic agent for the treatment of the cancer or tumor during the course of the treatment period. In certain embodiments, monotherapy using a variant IgG means that the variant IgG is administered in the absence of an additional anti-cancer therapy during that treatment period.

By "maintenance therapy" is meant a therapeutic regimen that is given to reduce the likelihood of disease recurrence or progression. Maintenance therapy can be provided for any length of time, including extended time periods up to the life-span of the subject. Maintenance therapy can be provided after initial therapy or in conjunction with initial or additional therapies. Dosages used for maintenance therapy can vary and can include diminished dosages as compared to dosages used for other types of therapy.

"Neoadjuvant therapy" or "preoperative therapy" herein refers to therapy given prior to surgery. The goal of neoadjuvant therapy is to provide immediate systemic treatment, potentially eradicating micrometastases that would otherwise proliferate if the standard sequence of surgery followed by systemic therapy were followed. Neoadjuvant therapy may also help to reduce tumor size thereby allowing complete resection of initially unresectable tumors or preserving portions of the organ and its functions. Furthermore, neoadjuvant therapy permits an *in vivo* assessment of drug efficacy, which may guide the choice of subsequent treatments.

"Adjuvant therapy" herein refers to therapy given after surgery, where no evidence of residual disease can be detected, so as to reduce the risk of disease recurrence. The goal of adjuvant therapy is to prevent recurrence of the cancer, and therefore to reduce the chance of cancer-related death.

Herein, "standard of care" chemotherapy refers to the chemotherapeutic agents routinely used to treat a particular cancer.

"Definitive surgery" is used as that term is used within the medical community. Definitive surgery includes, for example, procedures, surgical or otherwise, that result in removal or resection of the tumor, including those that result in the removal or resection of all grossly visible tumor. Definitive surgery includes, for example, complete or curative resection or complete gross resection of the tumor. Definitive surgery includes procedures that occurs in one or more stages, and includes, for example, multi-stage surgical procedures where one or more surgical or other procedures are performed prior to resection of the tumor. Definitive surgery includes procedures to remove or resect the tumor including involved organs, parts of organs and tissues, as well as surrounding organs, such as lymph nodes, parts of organs, or tissues.

Administration "in combination with" one or more further therapeutic agents includes simultaneous (concurrent) and consecutive administration in any order.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

A "liposome" is a small vesicle composed of various types of lipids, phospholipids and/or surfactant which is useful for delivery of a drug (such as a variant IgG) to a mammal. The components of the liposome are commonly arranged in a bilayer formation, similar to the lipid arrangement of biological membranes.

The term "anti-neoplastic composition" refers to a composition useful in treating cancer comprising at least one active therapeutic agent, *e.g*., "anti-cancer agent." Examples of therapeutic agents (anti-cancer agents) include, but are limited to, *e.g*., chemotherapeutic agents, growth inhibitory agents, cytotoxic agents, agents used in radiation therapy, anti-angiogenesis agents, apoptotic agents, anti-tubulin agents, and other-agents to treat cancer, such as anti-HER-2 antibodies, anti-CD20 antibodies, an epidermal growth factor receptor (EGFR) antagonist (*e.g*., a tyrosine kinase inhibitor), HER1/EGFR inhibitor (*e.g*., erlotinib (TARCEVA®), platelet derived growth factor inhibitors (*e.g.,* GLEEVEC® (Imatinib Mesylate)), a COX-2 inhibitor (*e.g.,* celecoxib), interferons, cytokines, antagonists (*e.g*., neutralizing antibodies) that bind to one or more of the following targets ErbB2, ErbB3, ErbB4, PDGFR-beta, BlyS, APRIL, BCMA or VEGF receptor(s), TRAIL/Apo2, and other bioactive and organic chemical agents, etc.

The term "cytotoxic agent" as used herein refers to a substance that inhibits or prevents a cellular function and/or causes cell death or destruction. The term is intended to include radioactive isotopes (*e.g*., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu), chemotherapeutic agents *(e.g*., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells. In certain embodiments, the variant IgG may be conjugated with a cytotoxic agent.

A "toxin" is any substance capable of having a detrimental effect on the growth or proliferation of a cell.

A "chemotherapeutic agent" is a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (*e.g*., calicheamicin, especially calicheamicin gamma1I and calicheamicin omegaI1 (*see, e.g.,* Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®), liposomal doxorubicin TLC D-99 (MYOCET®), peglylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); combretastatin; folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoid, *e.g*., paclitaxel (TAXOL®), albumin-engineered nanoparticle formulation of paclitaxel (ABRAXANE™), and docetaxel (TAXOTERE®); chloranbucil; 6-thioguanine; mercaptopurine; methotrexate; platinum agents such as cisplatin, oxaliplatin (*e.g*., ELOXATIN®), and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid, including bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R) (*e.g.*, erlotinib (TARCEVA®)); and VEGF-A that reduce cell proliferation; vaccines such as THERATOPE® vaccine and gene therapy vaccines, for example, ALLOVECTIN® vaccine, LEUVECTIN® vaccine, and VAXID® vaccine; topoisomerase 1 inhibitor (*e.g.,* LURTOTECAN®); rmRH (*e.g.,* ABARELIX®); BAY439006 (sorafenib; Bayer); SU-11248 (sunitinib, SUTENT®, Pfizer); perifosine, COX-2 inhibitor (*e.g*. celecoxib or etoricoxib), proteosome inhibitor (*e.g.* PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors; tyrosine kinase inhibitors; serine-threonine kinase inhibitors such as rapamycin (sirolimus, RAPAMUNE®); farnesyltransferase inhibitors such as lonafarnib (SCH 6636, SARASAR™); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN™) combined with 5-FU and leucovorin, and pharmaceutically acceptable salts, acids or derivatives of any of the above; as well as combinations of two or more of the above.

Chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens and selective estrogen receptor modulators (SERMs), including, for example, tamoxifen (including NOLVADEX® tamoxifen), raloxifene, droloxifene, 4-hydroxytamoxifen, trioxifene, keoxifene, LY117018, onapristone, and FARESTON· toremifene; aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole; and anti-androgens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in abherant cell proliferation, such as, for example, PKC-alpha, Raf and H-Ras; ribozymes such as a VEGF expression inhibitor (e.g., ANGIOZYME® ribozyme) and a HER2 expression inhibitor; vaccines such as gene therapy vaccines, for example, ALLOVECTIN® vaccine, LEUVECTIN® vaccine, and VAXID® vaccine; PROLEUKIN® rIL-2; LURTOTECAN® topoisomerase 1 inhibitor; ABARELIX® rmRH; Vinorelbine and Esperamicins (see U.S. Pat. No. 4,675,187), and pharmaceutically acceptable salts, acids or derivatives of any of the above; as well as combinations of two or more of the above.

A "growth inhibitory agent" when used herein refers to a compound or composition which inhibits growth of a cell (such as a cell expressing VEGF) either *in vitro* or *in vivo.* Thus, the growth inhibitory agent may be one which significantly reduces the percentage of cells (such as a cell expressing VEGF) in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in Mendelsohn and Israel, eds., The Molecular Basis of Cancer, Chapter 1, entitled "Cell cycle regulation, oncogenes, and antineoplastic drugs" by Murakami et al. (W.B. Saunders, Philadelphia, 1995), e.g., p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one time administration and typical dosages range from 10 to 200 units (Grays) per day.

The "pathology" of a disease includes all phenomena that compromise the well-being of the patient. For cancer, this includes, without limitation, abnormal or uncontrollable cell growth, metastasis, interference with the normal functioning of neighboring cells, release of cytokines or other secretory products at abnormal levels, suppression or aggravation of inflammatory or immunological response, etc.

A "small molecule" is defined herein to have a molecular weight below about 500 Daltons.

The term "intravenous infusion" refers to introduction of a drug into the vein of an animal or human patient over a period of time greater than approximately 5 minutes, preferably between approximately 30 to 90 minutes, although, according to the invention, intravenous infusion is alternatively administered for 10 hours or less.

The term "intravenous bolus" or "intravenous push" refers to drug administration into a vein of an animal or human such that the body receives the drug in approximately 15 minutes or less, preferably 5 minutes or less.

The term "subcutaneous administration" refers to introduction of a drug under the skin of an animal or human patient, preferable within a pocket between the skin and underlying tissue, by relatively slow, sustained delivery from a drug receptacle. The pocket may be created by pinching or drawing the skin up and away from underlying tissue.

The term "subcutaneous infusion" refers to introduction of a drug under the skin of an animal or human patient, preferably within a pocket between the skin and underlying tissue, by relatively slow, sustained delivery from a drug receptacle for a period of time including, but not limited to, 30 minutes or less, or 90 minutes or less. Optionally, the infusion may be made by subcutaneous implantation of a drug delivery pump implanted under the skin of the animal or human patient, wherein the pump delivers a predetermined amount of drug for a predetermined period of time, such as 30 minutes, 90 minutes, or a time period spanning the length of the treatment regimen.

The term "subcutaneous bolus" refers to drug administration beneath the skin of an animal or human patient, where bolus drug delivery is preferably less than approximately 15 minutes, more preferably less than 5 minutes, and most preferably less than 60 seconds. Administration is preferably within a pocket between the skin and underlying tissue, where the pocket is created, for example, by pinching or drawing the skin up and away from underlying tissue.

The word "label" when used herein refers to a detectable compound or composition which is conjugated directly or indirectly to the polypeptide. The label may be itself be detectable (e.g., radioisotope labels or fluorescent labels) or, in the case of an enzymatic label, may catalyze chemical alteration of a substrate compound or composition which is detectable.

### Antibodies

Antibodies are proteins which exhibit binding specificity to a specific antigen. Native antibodies are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains.

Depending on the amino acid sequence of the constant region of their heavy chains, antibodies or immunoglobulins can be assigned to different classes. There are five major classes of immunoglobulins: IgA, IgD, IgE, IgG and IgM, and several of these may be further divided into subclasses (isotypes), *e.g*. IgG₁, IgG₂, IgG₃, and IgG₄; IgA₁ and IgA₂. A variety of human IgG₁, IgG₂, IgG₃, and IgG₄ allotypes have been described (reviewed by M.-P. LeFranc and G. LeFranc in: "The Human IgG Subclasses," F. Shakib (ed.), pp. 43-78, Pergamon Press, Oxford (1990)). The different isotypes of the IgG class, including lgG₁, lgG₂ₛ, lgG₃, and lgG₄, have unique physical, biological, and clinical properties. Human IgGi is the most commonly used antibody for therapeutic purposes, and the majority of engineering studies have been constructed in this context.

The present application is directed to variant IgG immunoglobulins that include amino acid modifications that alter the biological properties of the IgG. The variant immunoglobulins of the present application include antibodies that comprise a modified Fc region that display longer half lives *in vivo* compared to the wild-type antibodies.

The half life of a variant IgG disclosed as herein can be increased by at least 50%, by at least 75% or by at least 100% compared to the half life of the IgG having the wild-type human IgG Fc region.

Alternatively, the half life of a variant IgG of the present invention is at least about 15 days, at least about 20, at least about 25 days, at least about 30 days, at least about 35 days or at least about 40 days. The variant IgG is variant IgG₁.

The half life of the variant IgG of the present invention can be the half life as measured inhumans or in cynomolgus monkeys.

### Antibody Fragments

The present disclosure covers antibody fragments. Of particular interest are antibodies that comprise Fc regions, Fc fusions and the constant region of the heavy chain. In certain embodiments, the antibody fragments are the fragments of variant immunoglobulins (IgGs) comprising Fc regions. Antibody fragments may be generated by traditional means, such as enzymatic digestion, or by recombinant techniques.

Various techniques have been developed for the production of antibody fragments. Traditionally, these fragments were derived via proteolytic digestion of intact antibodies (*see, e.g.,* Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from *E. coli,* thus allowing the facile production of large amounts of these fragments. Antibody fragments can be isolated from the antibody phage libraries. Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host cell culture. Other techniques for the production of antibody fragments will be apparent to the skilled practitioner. The antibody fragment may also be a "linear antibody", *e.g*., as described in U.S. Pat. No. 5,641,870, for example. Such linear antibodies may be monospecific or bispecific.

### Humanized Antibodies

The present disclosure covers humanized antibodies which may be a humanized variant IgGs with one or more amino acid modifications in the Fc region relative to wild-type IgG. Various methods for humanizing non-human antibodies are known in the art. For example, a humanized antibody can have one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies can be important to reduce antigenicity. According to the so-called "best-fit" method, the sequence of the variable domain of a rodent antibody is screened against the entire library of known human variable-domain sequences. The human sequence which is closest to that of the rodent is then accepted as the human framework for the humanized antibody. See, e.g., Sims et al. (1993) J. Immunol. 151:2296; Chothia et al. (1987) J. Mol. Biol. 196:901. Another method uses a particular framework derived from the consensus sequence of all human antibodies of a particular subgroup of light or heavy chains. The same framework may be used for several different humanized antibodies. See, e.g., Carter et al. (1992) Proc. Natl. Acad. Sci. USA, 89:4285; Presta et al. (1993) J. Immunol., 151:2623.

It is further generally desirable that antibodies be humanized with retention of high affinity for the antigen and other favorable biological properties. To achieve this goal, according to one method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

### Human Antibodies

The human antibodies disclosed herein may be human variant IgGs with one or more amino acid modifications in the Fc region relative to wild-type IgG. Human antibodies can be constructed by combining Fv clone variable domain sequence(s) selected from human-derived phage display libraries with known human constant domain sequences(s) as described above. Alternatively, human monoclonal antibodies can be made by the hybridoma method. Human myeloma and mouse-human heteromyeloma cell lines for the production of human monoclonal antibodies have been described, for example, by Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).

It is now possible to produce transgenic animals (*e.g*. mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. *See, e.g.,* Jakobovits et al., Proc. Natl. Acad. Sci USA, 90: 2551 (1993); Jakobovits et al., Nature, 362: 255 (1993); Bruggermann et al., Year in Immunol., 7: 33 (1993).

Gene shuffling can also be used to derive human antibodies from non-human, e.g. rodent, antibodies, where the human antibody has similar affinities and specificities to the starting non-human antibody. According to this method, which is also called "epitope imprinting", either the heavy or light chain variable region of a non-human antibody fragment obtained by phage display techniques as described herein is replaced with a repertoire of human V domain genes, creating a population of non-human chain/human chain scFv or Fab chimeras. Selection with antigen results in isolation of a non-human chain/human chain chimeric scFv or Fab wherein the human chain restores the antigen binding site destroyed upon removal of the corresponding non-human chain in the primary phage display clone, i.e. the epitope governs (imprints) the choice of the human chain partner. When the process is repeated in order to replace the remaining non-human chain, a human antibody is obtained (see PCT WO 93/06213 published April 1, 1993). Unlike traditional humanization of non-human antibodies by CDR grafting, this technique provides completely human antibodies, which have no FR or CDR residues of non-human origin.

### Bispecific Antibodies

Bispecific antibodies are monoclonal antibodies that have binding specificities for at least two different antigens. These bispecific antibodies can be bispecific antibodies with one or more amino acid modifications in the Fc region relative to wild-type antibody or can be human or humanized antibodies. The binding specificities can be for VEGF and the other is for any other antigen. The bispecific antibodies may bind to two different epitopes of VEGF. Bispecific antibodies may also be used to localize cytotoxic agents to cells which express VEGF. These antibodies possess a VEGF-binding arm and an arm which binds a cytotoxic agent, such as, *e.g.,* saporin, anti-interferon-α, vinca alkaloid, ricin A chain, methotrexate or radioactive isotope hapten. In certain antibodies, the binding specificities are for IL-4 and IL-13. Bispecific antibodies can be prepared as full length antibodies or antibody fragments comprising Fc region.

Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature, 305: 537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed in WO 93/08829 published May 13, 1993, and in Traunecker et al., EMBO J., 10: 3655 (1991).

According to a different approach, antibody variable domains with the desired binding specificities (antibody-antigen combining sites) are fused to immunoglobulin constant domain sequences. The fusion, for example, is with an immunoglobulin heavy chain constant domain, comprising at least part of the hinge, CH2, and CH3 regions. In certain embodiments, the first heavy-chain constant region (CH1), containing the site necessary for light chain binding, is present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

The bispecific antibodies can be composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach, the interface between a pair of antibody molecules can be engineered to maximize the percentage of heterodimers which are recovered from recombinant cell culture. The interface comprises at least a part of the C_{H}3 domain of an antibody constant domain. In this method, one or more small amino acid side chains from the interface of the first antibody molecule are replaced with larger side chains (*e.g*. tyrosine or tryptophan). Compensatory "cavities" of identical or similar size to the large side chain(s) are created on the interface of the second antibody molecule by replacing large amino acid side chains with smaller ones (*e.g*. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/00373, and EP 03089). Heteroconjugate antibodies may be made using any convenient cross-linking method. Suitable cross-linking agents are well known in the art, and are disclosed in US Patent No. 4,676,980, along with a number of cross-linking techniques.

The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (VH) connected to a light-chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### Multivalent Antibodies

A multivalent antibody may be internalized (and/or catabolized) faster than a bivalent antibody by a cell expressing an antigen to which the antibodies bind. The antibodies as disclosed herein can be multivalent antibodies (which are other than of the IgM class) with three or more antigen binding sites (*e.g*. tetravalent antibodies), which can be readily produced by recombinant expression of nucleic acid encoding the polypeptide chains of the antibody. The multivalent antibody can comprise a dimerization domain and three or more antigen binding sites. In certain embodiments, the dimerization domain comprises (or consists of) an Fc region or a hinge region. In this scenario, the antibody will comprise an Fc region and three or more antigen binding sites amino-terminal to the Fc region. In certain embodiments, a multivalent antibody comprises (or consists of) three to about eight antigen binding sites. In one such embodiment, a multivalent antibody comprises (or consists of) four antigen binding sites. The multivalent antibody comprises at least one polypeptide chain (for example, two polypeptide chains), wherein the polypeptide chain(s) comprise two or more variable domains. For instance, the polypeptide chain(s) may comprise VD1-(X1)n -VD2-(X2)n -Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein may further comprise at least two (for example, four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### Single-Domain Antibodies

This disclosure covers also a single-domain antibody comprising Fc region. Such a single-domain antibody may have one or more amino acid modifications in the Fc region relative to wild-type IgG. A single-domain antibody is a single polypeptide chain comprising all or a portion of the heavy chain variable domain or all or a portion of the light chain variable domain of an antibody.

### Antibody Modifications

Amino acid sequence modification(s) of the immunoglobulins described herein are disclosed. Such modifications may comprise one or more amino acid modifications to the variant IgGs of the present invention. It may also be desirable to further alter the binding affinity, *in vivo* half-life and/or other biological properties of the variant IgGs of the present invention. These amino acid modifications may comprise one or more amino acid modifications in the Fc region not described herein. Modified amino acid sequences of the variant IgGs may be prepared by introducing appropriate changes into the nucleotide sequence encoding the antibody, or by peptide synthesis. Such modifications include, for example, deletions from, and/or insertions into and/or substitutions of, residues within the amino acid sequences of the antibody. Any combination of deletion, insertion, and substitution can be made to arrive at the final construct, provided that the final construct possesses the desired characteristics. The amino acid alterations may be introduced in the subject antibody amino acid sequence at the time that sequence is made.

A useful method for identification of certain residues or regions of the antibody that are preferred locations for mutagenesis is called "alanine scanning mutagenesis" as described by Cunningham and Wells (1989) Science, 244:1081-1085. Here, a residue or group of target residues are identified (*e.g*., charged residues such as arg, asp, his, lys, and glu) and replaced by a neutral or negatively charged amino acid (*e.g*., alanine or polyalanine) to affect the interaction of the amino acids with antigen. Those amino acid locations demonstrating functional sensitivity to the substitutions then are refined by introducing further or other modifications at, or for, the sites of substitution. Thus, while the site for introducing an amino acid sequence modification is predetermined, the nature of the mutation *per se* need not be predetermined. For example, to analyze the performance of a mutation at a given site, ala scanning or random mutagenesis is conducted at the target codon or region and the expressed immunoglobulins are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional modifications of the antibody molecule include the fusion to the N- or C-terminus of the antibody to an enzyme (*e.g.* for ADEPT) or a polypeptide which increases the serum half-life of the antibody.

A variant IgG disclosed herein may be altered to increase or decrease the extent to which the antibody is glycosylated. Glycosylation of polypeptides is typically either N-linked or O-linked. N-linked refers to the attachment of a carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition or deletion of glycosylation sites to the antibody is conveniently accomplished by altering the amino acid sequence such that one or more of the above-described tripeptide sequences (for N-linked glycosylation sites) is created or removed. The alteration may also be made by the addition, deletion, or substitution of one or more serine or threonine residues to the sequence of the original antibody (for O-linked glycosylation sites).

The carbohydrate attached to the Fc region of the variant IgGs may be altered. Native antibodies produced by mammalian cells typically comprise a branched, biantennary oligosaccharide that is generally attached by an N-linkage to Asn297 of the CH2 domain of the Fc region. *See, e.g.,* Wright et al. (1997) TIBTECH 15:26-32. The oligosaccharide may include various carbohydrates, *e.g*., mannose, N-acetyl glucosamine (GlcNAc), galactose, and sialic acid, as well as a fucose attached to a GlcNAc in the "stem" of the biantennary oligosaccharide structure. Modifications of the oligosaccharide in a variant IgG disclosed herein may be made in order to create variant IgGs with certain additionally improved properties. A variant IgG as disclosed herein may further comprise an amino substitution at position 297 to alanine.

For example, antibody modifications are contemplated having a carbohydrate structure that lacks fucose attached (directly or indirectly) to an Fc region. Such modifications may have improved ADCC function. *See, e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody modifications include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al.*,* especially at Example 11), and knockout cell lines, such as alpha-1,6-fucosyltransferase gene, *FUT8,* knockout CHO cells *(see, e.g.,* Yamane-Ohnuki et al. Biotech. Bioeng. 87: 614 (2004); Kanda, Y. et al., Biotechnol. Bioeng., 94(4):680-688 (2006); and WO2003/085107).

Antibody modifications are further provided with bisected oligosaccharides, *e.g*., in which a biantennary oligosaccharide attached to the Fc region of the antibody is bisected by GlcNAc. Such antibody variants may have reduced fucosylation and/or improved ADCC function. Examples of such antibody modifications are described, *e.g.,* in WO 2003/011878 (Jean-Mairet et al.); US Patent No. 6,602,684 (Umana et al.); and US 2005/0123546 (Umana et al.). Antibody modifications with at least one galactose residue in the oligosaccharide attached to the Fc region are also provided. Such antibody modifications may have improved CDC function. Such antibody modifications are described, *e.g.,* in WO 1997/30087 (Patel et al.); WO 1998/58964 (Raju, S.); and WO 1999/22764 (Raju, S.).

Antibody modifications that possess some but not all effector functions, which make it a desirable candidate for many applications in which the half life of the antibody *in vivo* is important yet certain effector functions (such as complement and ADCC) are unnecessary or deleterious may be introduced. The Fc activities of the antibody can be measured to ensure that only the desired properties are maintained. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-92 (1991). Non-limiting examples of *in vitro* assays to assess ADCC activity of a molecule of interest is described in U.S. Patent No. 5,500,362 (s*ee*, *e.g.* Hellstrom, I., et al. Proc. Nat'l Acad. Sci. USA 83:7059-7063 (1986)) and Hellstrom, I et al., Proc. Nat'l Acad. Sci. USA 82:1499-1502 (1985); 5,821,337 *(see* Bruggemann, M. et al., J. Exp. Med. 166:1351-1361 (1987)). Alternatively, non-radioactive assays methods may be employed (*see,* for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (*see,* for example, Petkova, S.B. et al., Int'l. Immunol. 18(12): 1759-1769 (2006)).

Other antibody modifications having one or more amino acid substitutions are possible. Sites of interest for substitutional mutagenesis include the hypervariable regions, but FR alterations are also contemplated. Conservative substitutions are shown in Table 1 under the heading of "preferred substitutions." More substantial changes, denominated "exemplary substitutions" are provided in Table 1, or as further described below in reference to amino acid classes. Amino acid substitutions may be introduced into an antibody of interest and the products screened, *e.g*., for a desired activity, such as improved antigen binding, decreased immunogenicity, improved ADCC or CDC, *etc.*

**TABLE 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Modifications in the biological properties of an antibody may be accomplished by selecting substitutions that affect (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, in Biochemistry, second ed., pp. 73-75, Worth Publishers, New York (1975)):
(1) non-polar: Ala (A), Val (V), Leu (L), Ile (I), Pro (P), Phe (F), Trp (W), Met (M)
(2) uncharged polar: Gly (G), Ser (S), Thr (T), Cys (C), Tyr (Y), Asn (N), Gln (Q)
(3) acidic: Asp (D), Glu (E)
(4) basic: Lys (K), Arg (R), His(H)

Alternatively, naturally occurring residues may be divided into groups based on common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class. Such substituted residues also may be introduced into the conservative substitution sites or, into the remaining (non-conserved) sites.

One type of substitutional modification involves substituting one or more hypervariable region residues of a parent antibody (*e.g*. a humanized or human antibody). In certain embodiments, the parent antibody is the wild-type counterpart variant IgG (*e.g.,* a variant IgG of the invention without any additional alteration in its amino acid sequence). Generally, the resulting antibodies selected for further development will have modified (*e.g*., improved) biological properties relative to the parent antibody from which they are generated. An exemplary substitutional modification is an affinity matured antibody, which may be conveniently generated using phage display-based affinity maturation techniques. Briefly, several hypervariable region sites (*e.g.* 6-7 sites) are mutated to generate all possible amino acid substitutions at each site. The antibodies thus generated are displayed from filamentous phage particles as fusions to at least part of a phage coat protein (*e.g.,* the gene III product of M13) packaged within each particle. The phage-displayed antibodies are then screened for their biological activity (*e.g.* binding affinity). In order to identify candidate hypervariable region sites for modification, scanning mutagenesis (*e.g.*, alanine scanning) can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to techniques known in the art, including those elaborated herein. Once such modified antibodies are generated, the panel of antibodies is subjected to screening using techniques known in the art, including those described herein, and antibodies with superior properties in one or more relevant assays may be selected for further development.

Nucleic acid molecules encoding amino acid sequence of the modified antibody (*e.g*., modified variant IgG) are prepared by a variety of methods known in the art. These methods include, but are not limited to, isolation from a natural source (in the case of naturally occurring amino acid sequence modifications) or preparation by oligonucleotide-mediated (or site-directed) mutagenesis, PCR mutagenesis, and cassette mutagenesis of an earlier prepared modified antibody or a non-modified version of the antibody.

In accordance with this description and the teachings of the art, it is contemplated that an antibody modification may comprise one or more alterations as compared to the wild-type counterpart variant IgG (*e.g.,* a variant IgG of the invention without any additional alteration in its amino acid sequence). These antibody modifications comprising additional alterations would nonetheless retain substantially the same characteristics required for therapeutic utility as compared to the wild-type counterpart variant IgG. In certain embodiments, the wild-type counterpart variant IgG is a variant of bevacizumab.

Antibody modifications may also comprise modifications in the interface of Fc polypeptides comprising the Fc region, wherein the modifications facilitate and/or promote heterodimerization. These modifications comprise introduction of a protuberance into a first Fc polypeptide and a cavity into a second Fc polypeptide, wherein the protuberance is positionable in the cavity so as to promote complexing of the first and second Fc polypeptides. Methods of generating antibodies with these modifications are known in the art, *e.g.,* as described in U.S. Pat. No. 5,731,168.

It may also be desirable to create cysteine engineered antibodies, *e.g*., "thioMAbs," in which one or more residues of an antibody are substituted with cysteine residues. These substitutions may occur at accessible sites of the antibody. By substituting those residues with cysteine, reactive thiol groups are thereby positioned at accessible sites of the antibody and may be used to conjugate the antibody to other moieties, such as drug moieties or linker-drug moieties, as described further herein. For example, any one or more of the following residues may be substituted with cysteine: V205 (Kabat numbering) of the light chain; A118 (EU numbering) of the heavy chain; and S400 (EU numbering) of the heavy chain Fc region.

### Antibody Derivatives

The variant IgGs of the present invention may be further modified to contain additional nonproteinaceous moieties that are known in the art and readily available. The IgG may be conjugated with a cytotoxic agent. In certain embodiments, the variant IgG to which the cytotoxic agent is bound is internalized by the cell, resulting in increased therapeutic efficacy of the conjugate in killing the cancer cell to which it binds. The cytotoxic agent targets or interferes with nucleic acid in the cancer cell.

Moieties suitable for derivatization of the antibody are water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (*e.g.,* glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

Conjugates of an antibody and nonproteinaceous moiety that may be selectively heated by exposure to radiation can be contructed. The nonproteinaceous moiety can be a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### Making Variant IgGs

The variant IgGs can be made by any method known in the art. For example, the variant IgG sequences can be used to create nucleic acids that encode the member sequences, and that may then be cloned into host cells, expressed and assayed, if desired. These practices are carried out using well-known procedures, and a variety of methods that may find use in are described in Molecular Cloning--A Laboratory Manual, 3rd Ed. (Maniatis, Cold Spring Harbor Laboratory Press, New York, 2001), and Current Protocols in Molecular Biology (John Wiley & Sons). The nucleic acids that encode the variant IgGs may be incorporated into an expression vector in order to express the protein. Expression vectors typically include a protein operably linked, that is, placed in a functional relationship, with control or regulatory sequences, selectable markers, any fusion partners, and/or additional elements. The variant IgGs may be produced by culturing a host cell transformed with nucleic acid, preferably an expression vector, containing nucleic acid encoding the variant IgGs, under the appropriate conditions to induce or cause expression of the protein. A wide variety of appropriate host cells may be used, including but not limited to mammalian cells, bacteria, insect cells, and yeast. For example, a variety of cell lines that may find use are described in the ATCC cell line catalog, available from the American Type Culture Collection. The methods of introducing exogenous nucleic acid into host cells are well known in the art, and will vary with the host cell used.

The variants IgGs may be purified or isolated after expression. Antibodies may be isolated or purified in a variety of ways known to those skilled in the art. Standard purification methods include chromatographic techniques, electrophoretic, immunological, precipitation, dialysis, filtration, concentration, and chromatofocusing techniques. As is well known in the art, a variety of natural proteins bind antibodies, for example bacterial proteins A, G, and L, and these proteins may find use in purification. Often, purification may be enabled by a particular fusion partner. For example, proteins may be purified using glutathione resin if a GST fusion is employed, Ni⁺² affinity chromatography if a His-tag is employed, or immobilized anti-flag antibody if a flag-tag is used. For general guidance in suitable purification techniques, *see* Antibody Purification: Principles and Practice, 3rd Ed., Scopes, Springer-Verlag, NY, 1994.

### Screening Variant IgGs

Variant IgGs of the present invention may be screened using a variety of methods, including but not limited to those that use *in vitro* assays, *in vivo* and cell-based assays, and selection technologies. Automation and high-throughput screening technologies may be utilized in the screening procedures. Screening may employ the use of a fusion partner or label, for example an immune label, isotopic label, or small molecule label such as a fluorescent or calorimetric dye.

The functional and/or biophysical properties of variant IgGs can be screened in an *in vitro* assay. The protein can be screened for functionality, for example its ability to catalyze a reaction or its binding affinity to its target.

A subset of screening methods are those that select for favorable members of a library. The methods are herein referred to as "selection methods," and these methods can be used for screening variant IgGs. When protein libraries are screened using a selection method, only those members of a library that are favorable, that is which meet some selection criteria, are propagated, isolated, and/or observed. A variety of selection methods are known in the art that may used for screening protein libraries. Other selection methods that may be used include methods that do not rely on display, such as in vivo methods. A subset of selection methods referred to as "directed evolution" methods are those that include the mating or breading of favorable sequences during selection, sometimes with the incorporation of new mutations.

Variants IgGs can also be screened using one or more cell-based or *in vivo* assays. For such assays, purified or unpurified proteins are typically added exogenously such that cells are exposed to individual variants or pools of variants belonging to a library. These assays are typically, but not always, based on the function of the variant IgG; that is, the ability of the variant IgG to bind to its target and mediate some biochemical event, for example effector function, ligand/receptor binding inhibition, apoptosis, and the like. Such assays often involve monitoring the response of cells to the IgG, for example cell proliferation, cell migration, angiogenesis, cell survival, cell death, change in cellular morphology, or transcriptional activation such as cellular expression of a natural gene or reporter gene. For example, such assays may measure the ability of IgG variants to elicit ADCC, ADCP, or CDC. For some assays additional cells or components, that is in addition to the target cells, may need to be added, for example serum complement, or effector cells such as peripheral blood monocytes (PBMCs), NK cells, macrophages, and the like. Such additional cells may be from any organism, preferably humans, mice, rat, rabbit, and monkey. In certain embodiments, antibodies may inhibit angiogenesis and methods for monitoring such activity are well known in the art. In yet another embodiment, antibodies may cause apoptosis of certain cell lines expressing the target, or they may mediate attack on target cells by immune cells which have been added to the assay. Methods for monitoring cell death or viability are known in the art, and include the use of dyes, immunochemical, cytochemical, and radioactive reagents. Transcriptional activation may also serve as a method for assaying function in cell-based assays. Alternatively, cell-based screens are performed using cells that have been transformed or transfected with nucleic acids encoding the variants. That is, variant IgGs are not added exogenously to the cells.

The biological properties of the variant IgGs may be characterized in cell, tissue, and whole organism experiments. Drugs are often tested in animals, including but not limited to mice, rats, rabbits, dogs, cats, pigs, and monkeys, in order to measure a drug's efficacy for treatment against a disease or disease model, or to measure a drug's pharmacokinetics, toxicity, and other properties. The animals may be referred to as disease models. Therapeutics are often tested in mice, including but not limited to nude mice, SCID mice, xenograft mice, and transgenic mice (including knockins and knockouts). Such experimentation may provide meaningful data for determination of the potential of the protein to be used as a therapeutic. Any organism, preferably mammals, may be used for testing. For example because of their genetic similarity to humans, monkeys can be suitable therapeutic models, and thus may be used to test the efficacy, toxicity, pharmacokinetics, or other property of the variant IgGs. Tests of the in humans are ultimately required for approval as drugs, and thus of course these experiments are contemplated. Thus the variant IgGs may be tested in humans to determine their therapeutic efficacy, toxicity, immunogenicity, pharmacokinetics, and/or other clinical properties.

### Therapeutic Uses of Variant IgGs

The variant IgGs may find use in a wide range of products. For example, the IgG variant may be a therapeutic, a diagnostic, or a research reagent. The variant IgG may find use in an antibody composition that is monoclonal or polyclonal. The variant IgG may also be used to block, antagonize or agonize the target antigen, such as VEGF. In certain embodiments, the variant IgGs are used to block or neutralize VEGF activity. In one embodiment, the VEGF activity is angiogenesis.

The variant IgGs may be used for various therapeutic purposes, including but not limited to treating patient with neoplastic and/or non-neoplastic diseases as defined herein under "Definitions." In certain embodiments, neoplastic disease is cancer. In certain embodiments, patients are first treated with wild-type IgG and later treated with variant IgG. For example, the patients are first treated with bevacizumab and then later treated with the variant IgG, *e.g.,* a variant of bevacizumab. In certain embodiments, the patients are treated with bevacizumab for about 6 months and then later treated with the variant IgG, *e.g.,* a variant of bevacizumab.

A number of antibodies and Fc fusions that are approved for use, in clinical trials, or in development are herein referred to as "clinical products and candidates." The variant IgGs of the present invention may find use in a range of clinical products and candidates. Examples of antibodies which may be modified include, but are not limited to, an antibody that can bind to a target antigen such as VEGF, EGFR (ErbB-1), Her2/neu (ErbB-2), Her3 (ErbB-3), Her4 (ErbB-4), CD20, IgE, CD11, low density lipoprotein (LDL), interleukin 4 (IL-4), interleukin 13 (IL-13), an eptitope of hepatitis C, A-beta, IL-17A, IL-17F, DR6, DR5, an epitope of human cytomegalovirus, an epitope of staph aureus, tissue factor, alpha4beta7 integrin, alpha5beta1 integrin, CTLA4, CD3, an epitope of the RSV, NFalpha, CD147, IL8, MUC18, MUC1, alpha4beta1 (VLA-4) integrin, lymphotoxin alpha receptor, lymphotoxin beta receptor (LTBR), TGF-β2, IL-12, TGFβ1, Eotaxin1, BAFF, TRAIL-R1, IL15, Heparanase I; CD40, CD154, CD80, CD23, macrophage migration factor (MIF), KDR, flk-1, VE cadherin, carcinoembryonic antigen (CEA), CD22, CTLA4, CD30, intercellular adhesion molecule-1, anti-fibroblast growth factor receptor 3 (FGFR-3), gamma interferon, IL-12, Ep-CAM antibody and beta2 integrin.

Examples of clinical products and candidates which may be modified include, but are not limited to, anti-VEGF antibody AVASTIN® (bevacizumab, Genentech) (see for example, U.S. Patent No. 7,169,901;humanized anti-HER2 monoclonal antibody HERCEPTIN® (trastuzumab, Genentech) (see for example, U.S. Patent No. 6,489,447;chimeric anti-CD20 antibody RITUXAN® (rituximab, IDEC/Genentech/Roche); anti-IgE antibody XOLAIR® (omalizumab, Genentech); other anti-CD20 antibodies; anti-CD11a antibody RAPTIVA® (efalizumab, Genentech/Xoma); anti-Her2 antibody OMNITARG® (pertuzumab, Genentech); an anti-oxLDL antibody (see for example, U.S. Publication No. 20040202653 and WO 2004030607; anti-CD4 antibody MTRX1011A (see for example, WO 02/102853; bispecific antibodies wherein target antigens are IL-4 and IL-13; an anti-HCV antibody; an anti- IL-17A/F antibody; an anti-A-beta antibody; an anti-DR6 antibody; anti-human cytomegalovirus (HCMV) antibody; anti-HER receptor family antibodies; an anti-tissue factor antibody; MLN-02 antibody, a humanized IgG₁ monoclonal antibody to α4β7 integrin (formerly LDP-02, Genentech/Millennium Pharmaceuticals); humanized anti-CD 18 F(ab')₂ antibody; and a humanized anti-IgE IgG₁ antibody rhuMab-E25 (Genentech/Norvartis/Tanox Biosystems).

The additional clinical products and candidates which may be so modified include, but are not limited to, a chimeric anti-CD20 antibody approved to treat Non-Hodgkin's lymphoma; an anti-CD20 antibody HuMax-CD20 (Genmab); anti-CD20 antibody AME-133 (see for example, U.S. Pat. No. 5,500,362, Applied Molecular Evolution); hA20 (Immunomedics, Inc.), HumaLYM (Intracel), and PRO70769 (PCT/US2003/040426). A number of antibodies that target members of the family of epidermal growth factor receptors, including EGFR (ErbB-1), Her2/neu (ErbB-2), Her3 (ErbB-3), Her4 (ErbB-4), may also benefit from the modifications to Fc region described in the present invention. For example the IgG variants may find use in an antibody that is substantially similar to ERBITUX® (cetuximab, Imclone) (U.S. Pat. No. 4,943,533; WO 96/40210; a chimeric anti-EGFR antibody in clinical trials for a variety of cancers; ABX-EGF (U.S. Pat. No. 6,235,883, Abgenix/Immunex/Amgen); HuMax-EGFr (U.S. Ser. No. 10/172,317, incorporated by reference in its entirety, Genmab); 425, EMD55900, EMD62000, and EMD72000 (Merck KGaA) (U.S. Pat. No. 5,558,864; Murthy et al. 1987, Arch Biochem Biophys. 252(2):549-60; Rodeck et al., 1987, J Cell Biochem. 35(4):315-20; Kettleborough et al., 1991, Protein Eng. 4(7):773-83; ICR62 (Institute of Cancer Research) (WO 95/20045; Modjtahedi et al., 1993, J. Cell Biophys. 1993, 22(1-3):129-46; Modjtahedi et al., 1993, Br J Cancer. 1993, 67(2):247-53; Modjtahedi et al, 1996, Br J Cancer, 73(2):228-35; Modjtahedi et al, 2003, Int J Cancer, 105(2):273-80); TheraCIM hR3 (YM Biosciences, Canada and Centro de Immunologia Molecular, Cuba (U.S. Pat. Nos. 5,891,996; 6,506,883; Mateo et al, 1997, Immunotechnology, 3(1):71-81);mAb-806 (Ludwig Institute for Cancer Research, Memorial Sloan-Kettering) (Jungbluth et al. 2003, Proc Natl Acad Sci U S A. 100(2):639-44); KSB-102 (KS Biomedix); MR1-1 (IVAX, National Cancer Institute) (WO 0162931A2); and SC100 (Scancell) (WO 01/88138). In certain embodiments, the IgG variants of the present invention may find use in CAMPATH® (alemtuzumab, Genzyme Corporation), a humanized monoclonal antibody currently approved for treatment of B-cell chronic lymphocytic leukemia. The IgG variants of the present invention may find use in a variety of antibodies or Fc fusions that are substantially similar to other clinical products and candidates, including but not limited to VEGF-Trap (Regeneron); muromonab-CD3 (Orthoclone OKT3®), an anti-CD3 antibody (Ortho Biotech/Johnson & Johnson); anti-CD20 antibody ZEVALIN® (ibritumomab tiuxetan, IDEC/Schering AG); anti-CD33 antibody MYLOTARG®, an (p67 protein) antibody gemtuzumab ozogamicin (Celltech/Wyeth); an anti-LFA-3 Fc fusion antibody AMEVIVE® (alefacept, Biogen); REOPRO® (abciximab, Centocor/Lilly); SIMULECT® (basiliximab, Novartis); SYNAGIS® (palivizumab, MedImmune); anti-TNFalpha antibody REMICADE® (infliximab, Centocor); anti-TNFalpha antibody HUMIRA®, (adalimumab, Abbott); humanized IgG₄ anti-TNF antibody HUMICADE® (Celltech); anti-TNFalpha Fc fusion ENBREL® (etanercept, Immunex/Amgen); anti-CD147 antibody ABX-CBL (Abgenix); anti-IL8 antibody ABX-IL8 (Abgenix); anti-MUC18 antibody ABX-MA1 (Abgenix); an anti-MUCl antibody pemtumomab (R1549, 90Y-muHMFG1) (Antisoma); anti-MUCl antibody therex (R1550) (Antisoma); AngioMab (AS1405) (Antisoma); HuBC-1 (Antisoma); Thioplatin (AS1407) (Antisoma); TYSABRI® (formally ANTEGREN®, natalizumab), an anti-alpha-4-beta-1 (VLA-4) and alpha-4-beta-7 antibody (Biogen); anti-VLA-1 integrin antibody VLA-1 mAb (Biogen); anti-lymphotoxin beta receptor (LTBR) antibody LTBR mAb (Biogen); anti-TGF-β2 antibody CAT-152 (Cambridge Antibody Technology); J695, an anti-IL-12 antibody (Cambridge Antibody Technology/Abbott); CAT-192, an anti-TGFβ1 antibody (Cambridge Antibody Technology/Genzyme); CAT-213, an anti-Eotaxinl antibody (Cambridge Antibody Technology); LYMPHOSTAT-B®, an anti-Blys antibody (Cambridge Antibody Technology/ Human Genome Sciences Inc.; TRAIL-RlmAb, an anti-TRAIL-Rl antibody (Cambridge Antibody Technology/Human Genome Sciences, Inc.); HUMAX-CD4, an anti-CD4 antibody (Genmab); HuMax-IL15, an anti-IL15 antibody (Genmab/Amgen); HuMax-Inflam (Genmab/ Medarex); HuMax-Cancer, an anti-Heparanase I antibody (Genmab/Medarex/Oxford GlycoSciences); HuMax-Lymphoma (Genmab/Amgen); HuMax-TAC (Genmab); IDEC-131, an anti-CD40L antibody (IDEC Pharmaceuticals); IDEC-151 (clenoliximab), an anti-CD4 antibody (IDEC Pharmaceuticals); IDEC-114, an anti-CD80 antibody (IDEC Pharmaceuticals); IDEC-152, an anti-CD23 antibody (IDEC Pharmaceuticals); anti-macrophage migration factor (MIF) antibodies (IDEC Pharmaceuticals); BEC2, an anti-idiotypic antibody (Imclone); IMC-1C11, an anti-KDR antibody (Imclone); DC101, an anti-flk-1 antibody (Imclone); anti-VE cadherin antibodies (Imclone); CEA-CIDE® (labetuzumab), an anti-carcinoembryonic antigen (CEA) antibody (Immunomedics); LYMPHOCIDE® (Epratuzumab), an anti-CD22 antibody (Immunomedics); AFP-Cide (Immunomedics); MyelomaCide (Immunomedics); LkoCide (Immunomedics); ProstaCide (Immunomedics); MDX-010, an anti-CTLA4 antibody (Medarex); MDX-060, an anti-CD30 antibody (Medarex); MDX-070 (Medarex); MDX-018 (Medarex); OSIDEM® (IDM-1), an anti-Her2 antibody (Medarex /Immuno-Designed Molecules); CNTO 148, an anti-TNFα antibody (Medarex/Centocor/J&J); CNTO 1275, an anti-cytokine antibody (Centocor/J&J); MOR101 and MOR102, anti-intercellular adhesion molecule-1 (ICAM-1, also known as CD54) antibodies (MorphoSys); MOR201, an anti-fibroblast growth factor receptor 3 (FGFR-3) antibody (MorphoSys); NUVION® (visilizumab), an anti-CD3 antibody (Protein Design Labs); HuZAF®, an anti-gamma interferon antibody (Protein Design Labs); antibodies to α5β1 integrin (Protein Design Labs); anti-IL-12 (Protein Design Labs); ING-1, an anti-Ep-CAM antibody (Xoma); and MLN01, an anti-beta2 integrin antibody (Xoma).

The variant IgGs with modifications in the IgG Fc region of the present invention may be incorporated into the aforementioned clinical candidates and products, or into antibodies and Fc fusions that are substantially similar to them. The variant IgGs of the present invention may also be incorporated into versions of the aforementioned clinical candidates and products that are humanized, affinity matured, engineered, or modified in some other way.

In certain embodiments, the variant IgGs of the present invention may find use in the treatment of benign, pre-cancerous, or non-metastatic cancers; for the treatment of dormant tumors or micrometases; for the prevention of tumor recurrence or re-growth; or for treatment or prevention of cancer in a subject at risk for developing cancer. For example, variant IgGs comprising Fc modifications as described herein may be used for adjuvant therapy for the treatment of a subject with nonmetastatic cancer, following definitive surgery or for neoadjuvant therapy for the treatment of a subject with an operable cancer where the therapy is provided prior to the surgical removal of operable cancer in the subject. While the therapeutic applications are separated below prevention, neoadjuvant therapy, and adjuvant therapy, it will be appreciated by the skilled artisan that these categories are not necessarily mutually exclusive.

### Classification of Tumors

Cancer staging systems describe how far the cancer has spread anatomically and attempt to put patients with similar prognosis and treatment in the same staging group. Several tests may be performed to help stage cancer including biopsy and certain imaging tests such as a chest x-ray, mammogram, bone scan, CT scan, and MRI scan. Blood tests and a clinical evaluation are also used to evaluate a patient's overall health and detect whether the cancer has spread to certain organs.

To stage cancer, the American Joint Committee on Cancer first places the cancer, particularly solid tumors, in a letter category using the TNM classification system. Cancers are designated the letter T (tumor size), N (palpable nodes), and/or M (metastases). T1, T2, T3, and T4 describe the increasing size of the primary lesion; N0, N1, N2, N3 indicates progressively advancing node involvement; and M0 and M1 reflect the absence or presence of distant metastases.

In the second staging method, also known as the Overall Stage Grouping or Roman Numeral Staging, cancers are divided into stages 0 to IV, incorporating the size of primary lesions as well as the presence of nodal spread and of distant metastases. In this system, cases are grouped into four stages denoted by Roman numerals I through IV, or are classified as "recurrent." For some cancers, stage 0 is referred to as "in situ" or "Tis," such as ductal carcinoma in situ or lobular carcinoma in situ for breast cancers. High grade adenomas can also be classified as stage 0. In general, stage I cancers are small localized cancers that are usually curable, while stage IV usually represents inoperable or metastatic cancer. Stage II and III cancers are usually locally advanced and/or exhibit involvement of local lymph nodes. In general, the higher stage numbers indicate more extensive disease, including greater tumor size and/or spread of the cancer to nearby lymph nodes and/or organs adjacent to the primary tumor. These stages are defined precisely, but the definition is different for each kind of cancer and is known to the skilled artisan.

Many cancer registries, such as the NCI's Surveillance, Epidemiology, and End Results Program (SEER), use summary staging. This system is used for all types of cancer. It groups cancer cases into five main categories:
*In situ* is early cancer that is present only in the layer of cells in which it began.
*Localized* is cancer that is limited to the organ in which it began, without evidence of spread.
*Regional* is cancer that has spread beyond the original (primary) site to nearby lymph nodes or organs and tissues.
*Distant* is cancer that has spread from the primary site to distant organs or distant lymph nodes.
*Unknown* is used to describe cases for which there is not enough information to indicate a stage.

In addition, it is common for cancer to return months or years after the primary tumor has been removed. Cancer that recurs after all visible tumor has been eradicated, is called recurrent disease. Disease that recurs in the area of the primary tumor is locally recurrent, and disease that recurs as metastases is referred to as a distant recurrence. A dormant tumor is a tumor that exists in a quiescent state in which tumor cells are present but tumor progression is not clinically apparent. Micrometastases are a small metastases or a number of cells that have spread from the primary tumor to other parts of the body. Micrometastasis may or may not be detected in a screening or diagnostic test. The methods of the invention are useful for preventing the occurrence of dormant tumors or micrometastases or the recurrence of the tumor, for example, in a setting where a dormant tumor or micrometastases is present but may or may not be clinically detected.

The methods of the invention are also useful for the treatment of early cancers including but not limited to benign, pre-cancerous, or non-metastatic tumors. This includes any stage 0, I, or II tumor; any non-metastatic stage II tumor; any condition that typically precedes or develops into a cancer, including but not limited to, dysplasia; and any tumor that remains localized at the site of origin and has not infiltrated, invaded, or metastasized to distant sites. Examples of benign, pre-cancerous, or non-metastatic tumors include a polyp, adenoma, fibroma, lipoma, gastrinoma, insulinoma, chondroma, osteoma, hemangioma, lymphangioma, meningioma, leiomyoma, rhabdomyoma, squamous cell papilloma, acoustic neuromas, neurofibroma, bile duct cystanoma, leiomyomas, mesotheliomas, teratomas, myxomas, trachomas, granulomas, hamartoma, transitional cell papilloma, pleiomorphic adenoma of the salivary gland, desmoid tumor, dermoid cystpapilloma, cystadenoma, focal nodular hyperplasia, and nodular regenerative hyperplasia.

Because angiogenesis is involved in both primary tumor growth and metastasis, the anti-angiogenic treatment provided by the invention is capable of inhibiting the neoplastic growth of tumor at the primary site as well as preventing metastasis of tumors at the secondary sites, therefore allowing attack of the tumors by other therapeutics.

Additional information regarding adjuvant and neoadjuvant therapies and the treatment of early stage tumors are disclosed in U.S. Application No. 12/002,605 and PCT Application PCT/US2007/088000).

### Prevention

In certain embodiments, variant IgGs can be used for the treatment of benign, pre-cancerous, or early stage cancers, or for the treatment or prevention of tumor recurrence. In certain embodiments, the variant IgG is an anti-VEGF antibody. In one embodiment, the variant IgG is a variant of bevacizumab. In one embodiment, the variant IgG comprises the complementarity determining regions of bevacizumab. In another embodiment, the variant IgG comprises the heavy chain variable domain (SEQ ID NO:1) and light chain variable domain (SEQ ID NO:2). The variant IgG may comprise the heavy chain variable domain (SEQ ID NO:7) and light chain variable domain (SEQ ID NO:8).

The methods can be used to treat the cancer itself or to prevent progression of the cancer to a metastatic or invasive stage or to a higher grade or stage. For example, the methods of the invention can be used to treat a subject with Stage 0 cancer or polyps in order to prevent progression to a Stage I or higher stage tumor. Similarly, in a patient having Stage II cancer, the methods can be used to prevent progression of the cancer to a Stage III or Stage IV cancer.

Variant IgGs can also be used to prevent the recurrence of a tumor. For example, if a tumor has been identified and treated (e.g., with chemotherapy or surgically removed), a variant IgG can be used to prevent the recurrence of the colorectal tumor either locally or a metastasis of the colorectal tumor. For the prevention of the recurrence of the tumor, the variant IgGs can be used, for example, to treat a dormant tumor or micrometastases, or to prevent the growth or re-growth of a dormant tumor or micrometastases, which may or may not be clinically detectable.

For example, variant IgGs can be used for the prevention of cancer in a subject who has never had cancer or who is at risk for developing a cancer. There are a variety of risk factors known to be associated with cancer and many of them are described herein. In addition, a subject known to have an inherited cancer syndrome is considered to be at risk for developing a cancer.

### Neoadjuvant Therapy

Methods of neoadjuvant therapy prior to the surgical removal of operable cancer in a subject, *e.g.,* a human patient, may comprise administering to the patient (*e.g.,* where the patient has been diagnosed with a tumor and/or cancer) an effective amount of a variant IgG, such as the variant IgG anti-VEGF antibody or a variant of bevacizumab. The variant IgG may comprise the complementarity determining regions of bevacizumab, the heavy chain variable domain (SEQ ID NO:1) and light chain variable domain (SEQ ID NO:2) or the heavy chain variable domain (SEQ ID NO:7) and light chain variable domain (SEQ ID NO:8).

The variant IgG may be administered in combination with at least one chemotherapeutic agent. The additional step of administering to the subject an effective amount of a variant IgG after surgery to prevent recurrence of the cancer can also be employed with the neoadjuvant therapies described herein. For the methods that include the additional step of administering to the subject an effective amount of a variant IgG after surgery, any of the adjuvant methods described herein can be used.

For example, one method includes treating cancer in a subject comprising the following steps: a) a first stage comprising a plurality of treatment cycles wherein each cycle comprises administering to the subject an effective amount of a variant IgG and, optionally, at least one chemotherapeutic agent at a predetermined interval; b) a definitive surgery whereby the cancer is removed; and, optionally, c) a second stage comprising a plurality of maintenance cycles wherein each cycle comprises administering to the subject an effective amount of a variant IgG with or without any chemotherapeutic agent at a predetermined interval.

For example, an administration schedule of the neoadjuvant therapy may comprise a first step wherein a variant IgG and one or more chemotherapeutic agents are administered to the patients in a plurality of neoadjuvant cycles, followed by a surgery to definitively remove the tumor. The neoadjuvant therapy may last for less than one year, in one embodiment, less than six months prior to surgery.

### Adjuvant Therapy

Methods of adjuvant therapy may comprise administering a variant IgG to a subject with nonmetastatic cancer, following definitive surgery. The variant IgG can be an anti-VEGF antibody or an IgG variant of bevacizumab which may comprise the complementarity determining regions of bevacizumab, the heavy chain variable domain (SEQ ID NO:1) and light chain variable domain (SEQ ID NO:2) or the heavy chain variable domain (SEQ ID NO:7) and light chain variable domain (SEQ ID NO:8).

For example, a method can include following steps: a) a first stage comprising a plurality of treatment cycles wherein each cycle comprises administering to the subject an effective amount of a variant IgG and optionally, at least one chemotherapeutic agent at a predetermined interval; and b) a second stage comprising a plurality of maintenance cycles wherein each cycle comprises administering to the subject an effective amount of a variant IgG without any chemotherapeutic agent at a predetermined interval; wherein the combined first and second stages last for at least one year after the initial postoperative treatment. In one embodiment, the first stage comprises a first plurality of treatment cycles wherein a variant IgG and a first chemotherapy regimen are administered, followed by a second plurality of treatment cycles wherein a variant IgG and a second chemotherapy regimen are administered.

The variant IgG is generally administered after a period of time in which the subject has recovered from the surgery. This period of time can include the period required for wound healing or healing of the surgical incision, the time period required to reduce the risk of wound dehiscence, or the time period required for the subject to return to a level of health essentially similar to or better than the level of health prior to the surgery. The period between the completion of the definitive surgery and the first administration of the variant IgG can also include the period needed for a drug holiday, wherein the subject requires or requests a period of time between therapeutic regimes. Generally, the time period between completion of definitive surgery and the commencement of the variant IgG therapy can include less than one week, 1 week, 2 weeks, 3 weeks, 4 weeks (28 days), 5 weeks, 6 weeks, 7 weeks, 8 weeks, 3 months, 4 months, 5 months, 6 months, 7 months, 8 months, 9 months, 10 months, 11 months, 1 year, 2 years, 3 years, or more. In one embodiment, the period of time between definitive surgery and administering the variant IgG is greater than 4 weeks (28 days) and less than 1 year.

In one administration schedule, the adjuvant therapy comprises a first stage wherein a variant IgG and one or more chemotherapeutic agents are administered to the patients in a plurality of treatment cycles; and a second stage wherein a variant IgG is used as a single agent in a plurality of maintenance cycles. For example, the variant IgG can be variant of bevacizumab and a treatment cycle can be eight weeks, which means patients receive one dose of chemotherapy and one dose of variant bevacizumab every eight weeks. The treatment cycle can also be twelve weeks, which means patients receive one dose of chemotherapy and one dose of variant bevacizumab, every twelve week. The adjuvant therapy can last for at least one year from the initiation of the treatment, and the subject's progress will be followed after that time. The progress of the therapy is easily monitored by conventional techniques and assays.

### Dosages, Formulations, and Duration

The variant IgG composition will be formulated, dosed, and administered in a fashion consistent with good medical practice. Factors for consideration in this context include, but not limited to, the particular disorder being treated, the particular mammal being treated, the clinical condition of the individual patient, the cause of the disorder, the site of delivery of the agent, the method of administration, the scheduling of administration, and other factors known to medical practitioners. For the prevention or treatment of disease, the appropriate dosage of a variant IgG, *e.g.,* an antibody, of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The variant IgG is suitably administered to the patient at one time or over a series of treatments.

Pharmaceutical formulations described herein may also contain more than one active compound as necessary for the particular indication being treated, preferably those with complementary activities that do not adversely affect each other. Such molecules are suitably present in combination in amounts that are effective for the purpose intended.

The "therapeutically effective amount" of the variant IgG, *e.g.,* an antibody, to be administered will be governed by considerations discussed herein, and is the minimum amount necessary to prevent, ameliorate, or treat a disease or disorder. For example, the "therapeutically effective amount" of the variant IgG to be administered is the minimum amount necessary to prevent, ameliorate, or treat, or stabilize, a benign, precancerous, or early stage cancer; or to treat or prevent the occurrence or recurrence of a tumor, a dormant tumor, or a micrometastases, for example, in the neoadjuvant or adjuvant setting. The variant IgG need not be, but is optionally formulated with one or more agents currently used to prevent or treat the disorder in question. The effective amount of such other agents depends on the amount of variant IgG present in the formulation, the type of disorder or treatment, and other factors discussed above. These are generally used in the same dosages and with administration routes as used hereinbefore or about from 1 to 99% of the heretofore employed dosages. Generally, alleviation or treatment of a disease or disorder involves the lessening of one or more symptoms or medical problems associated with the disease or disorder. In the case of cancer, the therapeutically effective amount of the drug can accomplish one or a combination of the following: reduce the number of cancer cells; reduce the tumor size; inhibit (*i.e.,* to decrease to some extent and/or stop) cancer cell infiltration into peripheral organs; inhibit tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. A composition of this invention can be used to prevent the onset or reoccurrence of the disease or disorder in a subject or mammal.

The duration of therapy will continue for as long as medically indicated or until a desired therapeutic effect (*e.g.,* those described herein) is achieved. In certain embodiments, the variant IgG therapy is continued for 2 months, 4 months, 6 months, 8 months, 10 months, 1 year, 2 years, 3 years, 4 years, 5 years, 10 years or for a period of years up to the lifetime of the subject.

Generally, alleviation or treatment of a benign, precancerous, or early stage cancer or the adjuvant or neoadjuvant therapy of a cancer (benign or malignant) involves the lessening of one or more symptoms or medical problems associated with the cancer. The therapeutically effective amount of the drug can accomplish one or a combination of the following to reduce (*e.g.,* by 20%, 30%, 40%, 50%, 60%, 70%, 80%, 90%, 100% or more) the number of cancer cells in the tumor; to reduce the size of the tumor; to reduce the tumor burden; to inhibit (*i.e.,* to decrease to some extent and/or stop) cancer cell infiltration into peripheral organs; to reduce vessel density in the tumor; to inhibit tumor metastasis; to reduce or inhibit tumor growth or tumor cell proliferation; to reduce or prevent the growth of a dormant tumor; to reduce or prevent the growth or proliferation of a micrometastases; to reduce or prevent the re-growth of a tumor after treatment or removal (*e.g*., in adjuvant therapy); to increase or extend the DFS or OS of a subject susceptible to or diagnosed with a benign, precancerous, or non-metastatic tumor or a malignant tumor; to reduce the size of a tumor to allow for surgery (*e.g*., in neoadjuvant therapy); and/or to relieve to some extent one or more of the symptoms associated with the cancer. The variant IgG can also be used to prevent the occurrence or recurrence of cancer in the subject.

For the prevention or treatment of a disease, the appropriate dosage of a variant IgG of the invention (when used alone or in combination with one or more other additional therapeutic agents) will depend on the type of disease to be treated, the type of antibody, the severity and course of the disease, whether the variant IgG is administered for preventive or therapeutic purposes, previous therapy, the patient's clinical history and response to the variant IgG, and the discretion of the attending physician. In certain embodiments, the variant IgG is suitably administered to the patient at one time or over a series of treatments. Depending on the type and severity of the disease, about 1 µg/kg to 20 mg/kg (*e.g.,* 0.1mg/kg-15mg/kg) of variant IgG can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. In one embodiment, depending on the condition, the treatment is sustained until the cancer is treated, as measured by the methods described herein or known in the art. One exemplary dosage of the variant IgG would be in the range from about 0.05 mg/kg to about 20 mg/kg. Thus, one or more doses of about 0.5 mg/kg, 2.0 mg/kg, 4.0 mg/kg, 7.5 mg/kg, 10 mg/kg or 15 mg/kg (or any combination thereof) may be administered to the patient. Such doses may be administered intermittently, *e.g.,* every three, every eight or every twelve weeks (*e.g.,* such that the patient receives from about two to about twenty, or *e.g.,* about six doses of the antibody). For example, an initial higher loading dose, followed by one or more lower doses may be administered. Such a dosing regimen may comprise administering an initial loading dose of about 4 mg/kg, followed by a weekly maintenance dose of about 2 mg/kg of the antibody. However, other dosage regimens may be useful. The progress of this therapy is easily monitored by conventional techniques and assays. Also here, an IgG variant anti-VEGF antibody, such as an IgG variant of bevacizumab may be used.

For example, in some dosing regimens the frequency of administration of the variant IgG is reduced compared to the frequency of administration of the wild-type IgG due to the increased half life of the variant IgG or, the variant IgG is administered less frequently than the recommended or prescribed dosage frequency of the wild-type IgG.

For example when the variant IgG is a variant of bevacizumab, the variant IgG may be administered every 4 weeks or at longer intervals. Alternatively, the variant IgG may be administered every 6 weeks or longer, every 8 weeks or longer, every 10 weeks or longer or, every 12 weeks or longer.

The variant IgG may be administered initially every 2 weeks, and subsequently every 4 weeks or at longer intervals or may be administered initially every 2-3 weeks, and subsequently every 6 weeks or longer. Alternatively, the variant IgG may be administered initially every 2-4 weeks, and subsequently every 8 weeks or longer or, the variant IgG may be administered initially every 2-5 weeks, and subsequently every 10 weeks or longer. Within other alternative regimens, the variant IgG may be administered initially every 2-6 weeks, and subsequently every 12 weeks or longer or the variant IgG, *e.g.,* a variant of bevacizumab, can be initially administered with the prescribed dosage frequency of bevacizumab, and later administered less frequently than the prescribed dosage frequency of bevacizumab. Alternatively, bevacizumab is initially administered at the prescribed dosage frequency and a variant of bevacizumab is later administered less frequently than the prescribed dosage frequency of bevacizumab.

Alternatively, the variant IgG is administered every 14 days or at longer intervals, every 21 days or longer. In certain embodiments, the variant IgG is administered every 28 days or longer, every 60 days or longer, every month or at longer intervals, every two month or longer or every three months or longer.

The patient may also be treated with a combination of the variant IgG and one or more other therapeutic agent(s). Such a combined administration includes co-administration or concurrent administration, using separate formulations or a single pharmaceutical formulation, and consecutive administration in either order, wherein optionally there is a time period while both (or all) active agents simultaneously exert their biological activities. The effective amounts of therapeutic agents administered in combination with a variant IgG will be at the physician's or veterinarian's discretion. Dosage administration and adjustment is done to achieve maximal management of the conditions to be treated. The dose will additionally depend on such factors as the type of therapeutic agent to be used and the specific patient being treated. Exemplary suitable dosages for the variant IgG are those presently used for its wild-type IgG and can be lowered due to the increased half life and/or the combined action (synergy) of the variant IgG and the additional therapeutic agent used. The combination of the inhibitors may also potentiate the efficacy of a single inhibitor. The term "potentiate" refers to an improvement in the efficacy of a therapeutic agent at its common or approved dose.

The dosing regimens discussed herein can be used in combination with a chemotherapy which may involve the traditional high-dose intermittent administration or using smaller and more frequent doses of the chemotherapeutic agents without scheduled breaks ("metronomic chemotherapy").

Treatments may also include a combination of the variant IgG and one or more chemotherapeutic agent(s). For example, the chemotherapeutic agent may be administered prior to, or following, administration of the variant IgG or alternatively, the timing between at least one administration of the chemotherapeutic agent and at least one administration of the variant IgG is approximately 1 month or less. Alternatively, the timing between at least one administration of the chemotherapeutic agent and at least one administration of the variant IgG is approximately 2 weeks or less. Alternatively, the chemotherapeutic agent and the variant IgG are administered concurrently to the patient, in a single formulation or separate formulations. Treatment with the combination of the chemotherapeutic agent and the variant IgG may result in a synergistic, or greater than additive, therapeutic benefit to the patient.

The chemotherapeutic agent, if administered, is usually administered at dosages known therefore, or optionally lowered due to combined action of the drugs or negative side effects attributable to administration of the antimetabolite chemotherapeutic agent. Preparation and dosing schedules for such chemotherapeutic agents may be used according to manufacturers' instructions or as determined empirically by the skilled practitioner.

Various chemotherapeutic agents that can be combined are disclosed herein, *e.g*., under Definitions. Examples of chemotherapeutic agents to be combined with the variant IgG include, but are not limited to, *e.g.,* a taxoid (including docetaxel and paclitaxel), vinca (such as vinorelbine or vinblastine), platinum compound (such as carboplatin or cisplatin), aromatase inhibitor (such as letrozole, anastrazole, or exemestane), anti-estrogen (e.g. fulvestrant or tamoxifen), etoposide, thiotepa, cyclophosphamide, methotrexate, liposomal doxorubicin, pegylated liposomal doxorubicin, capecitabine, gemcitabine, COX-2 inhibitor (for instance, celecoxib), or proteosome inhibitor (*e.g*. PS342). "Cocktails" of different chemotherapeutic agents may be administered.

The progress of the therapies disclosed herein can be easily monitored by conventional techniques and assays.

For example, treatment or prevention of the occurrence or recurrence of a tumor, a dormant tumor, or a micrometastases can involve the prevention of tumor or metastases formation, generally after initial treatment or removal of a tumor (*e.g*., using an anti-cancer therapy such as surgery, chemotherapy, or radiation therapy). Surgery can leave behind residual tumor cells, or dormant micro-metastatic nodules, which have the potential to re-activate the "angiogenic program" and facilitate more exponential tumor growth. Although the presence of a dormant tumor or micrometastases is not necessarily detectable using clinical measurements or screens, a therapeutically effective amount is one that is sufficient to prevent or reduce detection of the dormant tumor, micrometastases, metastases, or tumor recurrence using techniques known to the clinician. In one example, a subject who is treated for a tumor by surgically removing the tumor is then treated with a variant IgG and monitored over time for the detection of a dormant tumor, micrometastases, or tumor recurrence. The variant IgG, *e.g.,* an anti-VEGF antibody, can be administered in combination with another anti-cancer therapy (*e.g*., prior to, with, or after the variant IgG) and one or both therapies can be continued as a maintenance therapy.

Additional measurements of therapeutic efficacy in the treatment of cancers are described in U.S. Patent Application Publication No. 20050186208.

Variant IgG of the invention (and any additional therapeutic agent) can be administered by any suitable means, including parenteral, subcutaneous, intraperitoneal, intracerobrospinal, intrapulmonary, and intranasal, and, if desired for local treatment, intralesional administration. Parenteral infusions include intramuscular, intravenous, intraarterial, intraperitoneal, or subcutaneous administration. In certain embodiments, the variant IgG, *e.g.,* an antibody, is suitably administered by pulse infusion, particularly with declining doses of the variant IgG. Dosing can be by any suitable route, *e.g.* by injections, such as intravenous or subcutaneous injections, depending in part on whether the administration is brief or chronic. In certain embodiments, the variant IgG is administered to a subject intravenously, *e.g*., as a bolus or by continuous infusion over a period of time.

The location of the binding target of a variant IgG, *e.g.,* an antibody, of the invention may be taken into consideration in preparation and administration of the variant IgG. When the binding target of a variant IgG is located in the brain, certain embodiments of the invention provide for the variant IgG to traverse the blood-brain barrier. Several art-known approaches exist for transporting molecules across the blood-brain barrier, including, but not limited to, physical methods, lipid-based methods, stem cell-based methods, and receptor and channel-based methods.

Physical methods of transporting a variant IgG, *e.g.,* an antibody, across the blood-brain barrier include, but are not limited to, circumventing the blood-brain barrier entirely, or by creating openings in the blood-brain barrier. Circumvention methods include, but are not limited to, direct injection into the brain (*see, e.g.,* Papanastassiou et al., Gene Therapy 9: 398-406 (2002)), interstitial infusion/convection-enhanced delivery (see, e.g., Bobo et al., Proc. Natl. Acad. Sci. USA 91: 2076-2080 (1994)), and implanting a delivery device in the brain (*see, e.g.,* Gill et al., Nature Med. 9: 589-595 (2003); and Gliadel Wafers™, Guildford Pharmaceutical). Methods of creating openings in the barrier include, but are not limited to, ultrasound (*see, e.g.,* U.S. Patent Publication No. 2002/0038086), osmotic pressure (*e.g*., by administration of hypertonic mannitol (Neuwelt, E. A., Implication of the Blood-Brain Barrier and its Manipulation, Vols 1 & 2, Plenum Press, N.Y. (1989)), permeabilization by, *e.g.,* bradykinin or permeabilizer A-7 (*see, e.g.,* U.S. Patent Nos. 5,112,596, 5,268,164, 5,506,206, and 5,686,416), and transfection of neurons that straddle the blood-brain barrier with vectors containing genes encoding the variant IgG (*see, e.g.,* U.S. Patent Publication No. 2003/0083299).

Lipid-based methods of transporting a variant IgG, *e.g.,* an antibody, across the blood-brain barrier include, but are not limited to, encapsulating the variant IgG in liposomes that are coupled to antibody binding fragments that bind to receptors on the vascular endothelium of the blood-brain barrier (*see, e.g.,* U.S. Patent Application Publication No. 20020025313), and coating the variant IgG in low-density lipoprotein particles (*see, e.g.,* U.S. Patent Application Publication No. 20040204354) or apolipoprotein E (*see, e.g.,* U.S. Patent Application Publication No. 20040131692).

Stem-cell based methods of transporting a variant IgG, *e.g.,* an antibody, across the blood-brain barrier entail genetically engineering neural progenitor cells (NPCs) to express the antibody of interest and then implanting the stem cells into the brain of the individual to be treated. *See* Behrstock et al. (2005) Gene Ther. 15 Dec. 2005 advanced online publication (reporting that NPCs genetically engineered to express the neurotrophic factor GDNF reduced symptoms of Parkinson disease when implanted into the brains of rodent and primate models).

Receptor and channel-based methods of transporting a variant IgG, *e.g.,* an antibody, across the blood-brain barrier include, but are not limited to, using glucocorticoid blockers to increase permeability of the blood-brain barrier (*see, e.g.,* U.S. Patent Application Publication Nos. 2002/0065259, 2003/0162695, and 2005/0124533); activating potassium channels (see, e.g., U.S. Patent Application Publication No. 2005/0089473), inhibiting ABC drug transporters (*see, e.g.,* U.S. Patent Application Publication No. 2003/0073713); coating antibodies with a transferrin and modulating activity of the one or more transferrin receptors (*see, e.g.,* U.S. Patent Application Publication No. 2003/0129186), and cationizing the antibodies (*see, e.g.,* U.S. Patent No. 5,004,697).

Pharmaceutical formulations comprising a variant IgG, *e.g.,* an antibody, of the invention are prepared for storage by mixing the variant IgG having the desired degree of purity with optional physiologically acceptable carriers, excipients or stabilizers (Remington: The Science and Practice of Pharmacy 20th edition (2000)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*., Zn-protein complexes); and/or non-ionic surfactants such as TWEEN™, PLURONICS™ or polyethylene glycol (PEG).

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington: The Science and Practice of Pharmacy 20th edition (2000).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the immunoglobulin of the invention, which matrices are in the form of shaped articles, *e.g*., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### Efficacy of the Treatment

Efficacy of variant IgGs can be measured in various ways, including but not limited to the methods described herein under "Definitions." For example, efficacy in treating tumor can be measured by detecting the ability of a variant IgG to inhibit or reduce the growth or metastasis of tumor. For example, a variant IgG has higher efficacy compared to a wild-type IgG if the variant IgG is able to reduce the rate of tumor growth compared to the tumor growth achieved with treatment using wild-type IgG or a variant IgG has higher efficacy compared to the wild-type IgG if the variant IgG can achieve maximum inhibition of tumor growth at a lower IgG dose than the dose that is needed for the wild-type IgG to achieve the same maximum inhibition of tumor growth. Alternatively, a variant IgG has higher efficacy compared to the wild-type IgG if the variant IgG has the ability to inhibit or reduce the growth or metastasis of cancerous cells at a lower IgG dose than the dose required for the wild-type IgG. Variant IgGs as disclosed herein may have equal or higher efficacy compared to wild-type IgGs or they do not have lower efficacy compared to wild-type IgGs.

The efficacy of the treatment of the invention can also be measured by various endpoints commonly used in evaluating neoplastic or non-neoplastic disorders. For example, cancer treatments can be evaluated by, *e.g.,* but not limited to, tumor regression, tumor weight or size shrinkage, time to progression, duration of survival, progression free survival, overall response rate, duration of response, quality of life, protein expression and/or activity. Because certain agents described herein, such as the anti-angiogenic agents, target the tumor vasculature and not necessarily the neoplastic cells themselves, they represent a unique class of anti-cancer drugs, and therefore can require unique measures and definitions of clinical responses to drugs. For example, tumor shrinkage of greater than 50% in a 2-dimensional analysis is the standard cut-off for declaring a response. However, the inhibitors of the invention may cause inhibition of metastatic spread without shrinkage of the primary tumor, or may simply exert a tumouristatic effect. Accordingly, approaches to determining efficacy of the therapy can be employed, including for example, measurement of plasma or urinary markers of angiogenesis and measurement of response through radiological imaging.

### Combination Therapies

Therapeutics described herein may be administered with other therapeutics concomitantly, *i.e.,* the therapeutics described herein may be co-administered with other therapies or therapeutics, including for example, small molecules, other biologicals, radiation therapy, surgery, *etc.*

Alternatively, an IgG variant can be the only therapeutically active agent administered to a patient. The IgG variant can also be administered in combination with one or more other therapeutic agents, including but not limited to anti-angiogenic agents, chemotherapeutic agents, cytokines, growth inhibitory agents, anti-hormonal agents, kinase inhibitors, cytotoxic agents, cardioprotectants, or other therapeutic agents. The IgG variants may be administered concomitantly with one or more other therapeutic regimens. For example, the IgG variant may be administered in conjunction with one or more antibodies, which may or may not be an IgG variant, or alternatively the IgG variants can be employed in combination with still other therapeutic techniques such as surgery.

In certain embodiments, additional agents, *e.g*., anti-cancer agents or therapeutics, or anti-angiogenesis agents, can also be administered in combination with variant IgG to treat various neoplastic or non-neoplastic conditions. For example, the neoplastic or non-neoplastic condition can be characterized by pathological disorder associated with aberrant or undesired angiogenesis. The variant IgGs of the invention can be administered serially or in combination with another agent that is effective for those purposes, either in the same composition or as separate compositions using the same or different administration routes.

Anti-angiogenic therapy in relationship to cancer is a cancer treatment strategy aimed at inhibiting the development of tumor blood vessels required for providing nutrients to support tumor growth. Since angiogenesis is involved in both primary tumor growth and metastasis, the anti-angiogenic treatment provided by the invention is capable of inhibiting the neoplastic growth of tumor at the primary site as well as preventing metastasis of tumors at the secondary sites, therefore allowing attack of the tumors by other therapeutics. For example, the anti-cancer agent or therapeutic can be an anti-angiogenic agent or the anti-cancer agent is a chemotherapeutic agent.

Many anti-angiogenic agents have been identified and are known in the arts, including those listed herein, *e.g.,* listed under Definitions, and by, *e.g.,* Carmeliet and Jain, Nature 407:249-257 (2000); Ferrara et al., Nature Reviews:Drug Discovery, 3:391-400 (2004); and Sato Int. J. Clin. Oncol., 8:200-206 (2003). *See also,* US Patent Publication No. 20030055006. For example, two or more angiogenesis inhibitors may optionally be co-administered to the patient in addition to variant IgG of the invention.

Other therapeutic agents that may be combined with the variant IgG include VEGF antagonist or VEGF receptor antagonists. Further therapeutic agents useful for combination tumor therapy with the variant IgG include antagonist of other factors that are involved in tumor growth, such as EGFR, ErbB2 (also known as Her2) ErbB3, ErbB4, or TNF. Alternatively, the variant IgG can be used in combination with small molecule receptor tyrosine kinase inhibitors (RTKIs) that target one or more tyrosine kinase receptors such as VEGF receptors, FGF receptors, EGF receptors and PDGF receptors. Many therapeutic small molecule RTKIs are known in the art, including, but are not limited to, vatalanib (PTK787), erlotinib (TARCEVA®), OSI-7904, ZD6474 (ZACTIMA®), ZD6126 (ANG453), ZD1839, sunitinib (SUTENT®), semaxanib (SU5416), AMG706, AG013736, Imatinib (GLEEVEC®), MLN-518, CEP-701, PKC- 412, Lapatinib (GSK572016), VELCADE®, AZD2171, sorafenib (NEXAVAR®), XL880, and CHIR-265.

Another option can be the use of a combination of two or more variant IgGs of the invention or the combination of at least one variant IgG with one or more additional anti-cancer therapies. Examples of anti-cancer therapies include, without limitation, surgery, radiation therapy (radiotherapy), biotherapy, immunotherapy, chemotherapy, or a combination of these therapies. For example, the anti-cancer therapy for the prostate cancer, ovarian cancer and breast cancer can be hormone therapy. In addition, cytotoxic agents, anti-angiogenic and antiproliferative agents can be used in combination with the variant IgG. An IgG variant can also be administered to the patient along with chemotherapy, radiation therapy, or both chemotherapy and radiation therapy.

The variant IgG can also be used as adjuvant therapy for the treatment of a nonmetastatic cancer following definitive surgery. In this example, the variant IgG can be provided with or without at least one additional chemotherapeutic agent.

Alternatively, the variant IgG can be used as neoadjuvant therapy for the treatment of an operable cancer prior to surgery. In this example, the variant IgG can be provided prior to surgery with or without at least one additional chemotherapeutic agent.

The the variant IgG and the one or more other therapeutic agents can be administered simultaneously or sequentially in an amount and for a time sufficient to reduce or eliminate the occurrence or recurrence of a tumor, a dormant tumor, or a micrometastases. The variant IgG and the one or more other therapeutic agents can be administered as maintenance therapy to prevent or reduce the likelihood of recurrence of the tumor.

Alternatively, a variant IgG with one or more chemotherapeutic agents (*e.g.,* a cocktail). Non-limiting examples of chemotherapeutic agents are described herein under Definitions. Preparation and dosing schedules for such chemotherapeutic agents may be used according to manufacturers' instructions or as determined empirically by the skilled practitioner, *see also,* section entitled Dosages, Formulations, and Duration.

### Articles of Manufacture

An article of manufacture containing materials useful for the treatment, prevention and/or diagnosis of the disorders described above can be used. The article of manufacture can comprise a container and a label or package insert on or associated with the container. Suitable containers include, for example, bottles, vials, syringes, *etc.* The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). The label or package insert indicates that the composition is used for treating the condition of choice. For example, the article of manufacture may comprise (a) a first container with a composition contained therein, wherein the composition comprises a variant IgG of the invention; and (b) a second container with a composition contained therein, wherein the composition comprises a further cytotoxic or otherwise therapeutic agent. The article of manufacture may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Alternatively, the variant IgG can be packaged alone or in combination with other therapeutic compounds as a kit. For example, the therapeutic compound can be an anti-cancer agent. The kit can include optional components that aid in the administration of the unit dose to patients, such as vials for reconstituting powder forms, syringes for injection, customized IV delivery systems, inhalers, *etc.* Additionally, the unit dose kit can contain instructions for preparation and administration of the compositions. The kit may be manufactured as a single use unit dose for one patient, multiple uses for a particular patient (at a constant dose or in which the individual compounds may vary in potency as therapy progresses); or the kit may contain multiple doses suitable for administration to multiple patients ("bulk packaging"). The kit components may be assembled in cartons, blister packs, bottles, tubes, and the like.

### EXAMPLES

### Example 1: Production of anti-VEGF (Bevacizumab) variants

The Fv regions of wild-type anti-VEGF (Bevacizumab) IgG₁ heavy and light were cloned separately into two pRK-based transient transfection plasmids containing human IgG₁ constant domains. Kunkel based site-directed mutagenesis was then used to generate all the anti-VEGF IgG₁ variants in which residues in the CH₂ and CH3 domains were mutated. The anti-VEGF variants generated in this study are summarized in Table 2 below. Each variant contains either single, double and triple mutations in the CH₂ and CH3 domains. Variants are numbered according to the EU index as in Kabat.

**Table 2**

| IgG₁ Variant |
|---|
| T307Q |
| A378V |
| N434A |
| N434H |
| N434S |
| Y436I |
| T307Q/A378V |
| T307Q/N434A |
| T307Q/N434S |
| T307Q/Y436I |
| T307Q/A378V/Y436I |
| T307Q/E380A/N434S |
| V308P/N434A |
| N434A/Y436I |

Plasmids containing the variants' heavy chain and wildtype light chain were co-transfected into the adenovirus-transformed human embryonic kidney cell line 293 by FUGENE® (Roche, Basel, Switzerland) according to the manufacturing protocol. After 24 hour of incubation with the transfection complexes, transfected cell were then cultured with either serum free media PSO₄ supplemented with 10mg/L of insulin and trace elements for 5 days or 1.3X GEM N Medium with 5mM Glutamine. Supernatant were collected, and conditioned with 1M TRIS pH 8.0 and 5M sodium chloride (NaCl) to give a final concentration of 30mM TRIS and 50mM NaCl. Conditioned supernatant were then purified using Protein A chromatography. Bound IgG₁ was eluted from the Protein A column with 0.1M glycine buffer pH 3.0. Next, purified IgG₁ were concentrated and injected over a Superdex-200 size exclusion chromatography column to remove any aggregates. Monomeric IgG₁ fractions were pooled together and later used for the binding studies. Anti-VEGF wild-type and anti-VEGF variants IgG₁ concentrations were calculated using absorbance reading at 280nM, and an absorbance of 1.5 was estimated to be 1mg/ml of IgG₁.

### Example 2: Production of human and cynomolgus monkey FcRn

Human FcRn is a heterodimer of an alpha chain and a β₂-microglobulin subunit. These two subunits were cloned separately into two pRK based transient transfection plasmids. Plasmids containing both alpha chain and a β₂-microglobulin were co-transfected into 293 cells using FUGENE® (Roche, Basel, Switzerland) according to manufacturing protocol. After 24 hour of incubation with the transfection complexes, transfected cell were then switched to serum free media PSO₄ supplemented with 10mg/L of insulin and trace elements for 5 days. Collected supernatant were filtered, and conditioned with 1M hydrochloric acid and 5M NaCl to give a final pH of 6.0 and concentration of 50mM NaCl. Conditioned supernatant were purified using IgG-sepharose chromatography. Bound FcRn was eluted from the column using a pH 8.0 buffer containing 30mM TRIS and 150mM NaCl. Eluted FcRn were further purified using a Superdex-75 size exclusion chromatography column to remove any aggregates. FcRn concentration was calculated using absorbance reading at 280nM, and an absorbance of 1.9 corresponded to be 1mg/ml of FcRn. Cynomolgus monkey FcRn is produced and purified similarly as human FcRn, except plasmids containing the cyno alpha chain and cyno β₂-microglobulin were used for the transfection.

### Example 3: FcRn binding studies: Injection of IgG₁ variants over FcRn

The binding of anti-VEGF variants against human FcRn were studied by surface plasmon resonance using a BIAcore 3000 instrument (GE healthcare, Piscataway, NJ). Human FcRn was coupled to the sensor chip using an amine coupling kit. Specifically, CM5 sensor chip was activated with EDC/NHS for 7min at 5µl/min. 100µg/ml of human FcRn were injected for 30 sec to 2 min at a flow rate of 10µl/min over the activated chip to give a maximum binding response unit (RU) of 50 to 200. After conjugation, FcRn coupled chip was blocked by an injection of 35µl of 1M ethanolamine hydrochloride at 5µl/min.

The anti-VEGF wildtype (WT) and anti-VEGF variants' binding to human FcRn at pH 6.0 or pH 7.4 were determined. The running buffer for the binding experiment is either PBS pH 6.0 or pH 7.4 containing 0.01% P20 and 0.02% sodium azide. Anti-VEGF (Bevacizumab) WT and anti-VEGF variants were buffer-exchanged into either pH 6.0 or pH 7.4 running buffer. All the experiments were performed at 25°C. For the pH 6.0 run, variants, with concentrations ranging from 15µM to 0.7nM, were flowed over a FcRn coated chip at 30µl/min for various times to achieve steady state and then were allowed to dissociate from the chip for 5min. For the pH 7.4 run, variants, with concentrations ranging from 30µM to 30nM, were injected over the FcRn coated chip at 20µl/min for various times to achieve steady state and then released for 2min. Variants were also flowed over an unconjugated spot on the sensor chip to allow subtraction of background non-specific binding from the binding to FcRn-coupled chip. Chip was regenerated with 30sec pulse of 0.1M TRIS pH 8.3 in between injections. Steady state RU for each injection was recorded at the end of each injection phase, and apparent dissociation constants (apparent K_{D}) were later estimated as the IgG concentrations that achieved 50% of maximum RU.

Results in **Figure 1A** and **1B****,** resulting from two different runs, show that all the variants have improved FcRn affinity over wildtype at pH 6. Estimates of the apparent dissociation constants (K_{D}) are shown in **Figure 2****.** As the FcRn coupling density differed for the two runs, the avidity level was different, resulting in slightly different apparent K_{D} values for the same variant. However, the affinity ranking of these variants remained the same for different runs. **Figure 3** shows that all of the anti-VEGF variants tested exhibit higher neutral pH binding to human FcRn compared to the wildtype. The affinity ranking of the variants based on pH 7.4 binding corresponded with the affinity ranking determined using pH 6 binding.

**Table 3**

| IgG₁ Variant |
|---|
| N434H |
| T307Q/N434A |
| T307Q/N434S |
| T307Q/E380A/N434S |
| V308P/N434A |

The human and cyno FcRn binding of the anti-VEGF wild-type (WT) and anti-VEGF variants shown in Table 3 were further evaluated using this assay format. Human or cyno FcRn was coated on sensor chips. Anti-VEGF wild-type and anti-VEGF variants were injected over the FcRn coated chips at 25°C in either pH 6.0 or pH 7.4 buffer. Steady state response units were recorded and plotted as a function of injection concentrations. All the anti-VEGF variants showed improved binding to human and cyno FcRn over anti-VEGF wild-type at both pH 6.0 and pH 7.4 *(see* **Fig. 4A-4D****).** The affinity ranking for the anti-VEGF variants determined in this assay was the same as the ranking determined using the monovalent K_{D}.

The human FcRn binding of the anti-HER2 wild-type (WT) and anti-HER2 variants shown in **Figure 26** were also evaluated using this assay format. The variants studied in **Figure 26** are L251A, L314A, L314D, L314K, E430A, E430K, L251D/N434H and L314D/N434H. Human FcRn was coated on sensor chips. Anti-HER2 wild-type and anti-HER2 variants were injected over the FcRn coated chips at 25°C in buffers with pH ranging from 6.0 to 7.2. Steady state response units were recorded and plotted as a function of injection concentrations for each pH. The affinities of wildtype and each variant were then estimated for each injection pH, and the affinity ratio of the variant to wildtype was plotted as a function of pH in **Figure 26****.** The affinity ratios for variants E430A, E430K and L251D/N434H decrease with increasing pH; whereas the affinity ratios for all of the other variants increase with increasing pH.

The bindings of anti-HER2 (traztuzumab) IgGi wild-type, variant T307Q/N434A, variant L251D/T307Q/N434H and variant L251D/T307Q/M428L/N434H/Y436I against human FcRn at pH 6.0 **(****Fig. 27A****),** pH 7.1 **(****Fig. 27B****),** and pH 7.4 **(****Fig. 27C****)** were also evaluated using similar assay format. Anti-HER2 wild-type and anti-HER2 variants were injected over the FcRn coated chips at 25°C in pH 6.0, pH 7.1 or pH 7.4 buffer. Steady state response units were recorded and plotted as a function of injection concentrations for each variant. Results show that at pH 6.0 **(****Fig. 27A****),** variant L251D/T307Q/N434H has similar FcRn affinity as wildtype, and variant L251D/T307Q/M428L/N434H/Y436I has similar FcRn affinity as variant T307Q/N434A. At pH 7.1 **(****Fig. 27B****),** variant L251D/T307Q/N434H has much lower FcRn affinity than wildtype; and variant L251D/T307Q/M428L/N434H/Y436I has similar FcRn affinity as wildtype and much lower FcRn affinity than variant T307Q/N434A. At pH 7.4 **(****Fig. 27C****),** variant L251D/T307Q/M428L/N434H/Y436I has lower FcRn affinity than wildtype and variant T307Q/N434A.

### Example 4: FcRn binding studies: Injection of human or cyno FcRn over IgG₁ variants

In the binding format using a BIAcore 3000 instrument (GE healthcare, Piscataway, NJ), anti-VEGF wild-type (Bevacizumab) and anti-VEGF variants were conjugated to different flowcells of the sensor chip using an amine coupling kit. Specifically, CM5 sensor chip was activated with EDC/NHS for 7 min at 5µl/min. 10 to 50µg/ml of antibodies were injected for 30 sec to 2 min at a flow rate of 10µl/min over the activated chip to give a maximum binding response unit (RU) of 50 to 200. After conjugation, FcRn coupled chip was blocked by an injection of 35µl of 1M ethanolamine hydrochloride at 5µl/min.

The running buffer for the binding experiments was PBS pH 6.0/0.01% P20/0.02% sodium azide (NaN₃). Soluble human or cyno FcRn dilutions from 20 µM to 0.15 nM were injected at a flow rate of 30µl/min for 10 min over the antibody-coated sensor chip at 25°C. Steady-state RUs were recorded at the end of the injection. The chip was regenerated with a 30 sec pulse of 0.1 M TRIS pH 8.5/0.15 M NaCl. FcRn was also injected over an unconjugated surface for background subtraction. The kinetic parameters and the monovalent equilibrium binding constants (K_{D}) were calculated using the BIAevaluation software (GE healthcare, Piscataway, NJ).

Results in **Figure 5** and **Figure 6** show that all of the anti-VEGF variants have improved FcRn affinity over wildtype to both human and cyno FcRn at pH 6.0. The affinity improvements were due to both increases in association rate constants and decreases in dissociation rate constants. Overall, FcRn affinity improvements of the anti-VEGF variants over wildtype using different binding assays are summarized in **Figure 8. Figure 8** shows that V308P/N434A variant has the highest FcRn affinity among the variants listed in Table 3, followed by T307Q/E380A/N434S, T307Q/N434S, and T307Q/N434A. N434H variant has the least amount of FcRn affinity improvement over wildtype.

### Example 5: Dissociation rates of the anti-VEGF and anti-HER2 variants at different pHs

To measure the dissociation rates at various pH's, 200nM to 2µM of human or cyno FcRn were first injected over antibody-conjugated flowcell at 30µl/min in PBS pH 6.0/0.01% P20/0.02% NaN₃ for 5 min to achieve steady state. Then PBS buffer at pHs ranging from 6 to 7.4 were injected at 30µl/min over the flowcell for 8min to allow the complex to dissociate. FcRn was also injected over an unconjugated surface for background subtraction. Dissociation rate constants were determined by fitting the dissociation phase of the sensorgram using the BIAevaluation software (GE healthcare, Piscataway, NJ). Results in **Figure 7** show that the k_{off}'s of the variants against both human FcRn **(****Fig. 7A****)** and cyno FcRn **(****Fig. 7B**) increase with increasing pH, and the rate of k_{off} increase for each variant was similar.

The dissociation rate (k_{off}) of anti-HER2 variant L251D/T307Q/M428L/N434H/Y436I against human FcRn at different pHs was similarly measured. The k_{off} of anti-HER2 variant L251D/T307Q/M428L/N434H/Y436I against human FcRn was plotted as a function of pH in **Figure 28****.** The k_{off} of anti-VEGF variants T307Q/N434A, T307Q/N434S. T307Q/E380A/N434S and V308P/N434A against human FcRn from **Figure 7** were also plotted in **Figure 28** for comparison. The k_{off} values at different pHs was fitted against pH for each variant to yield the slope of the best-fit line (equation: log(k_{off}) =slope x pH +y-intercept). The slopes of the anti-VEGF variants range from 0.75 to 0.84; whereas the slope of anti-HER2 variant L251D/T307Q/M428L/N434H/Y436I is about 1.2.

### Example 6: Binding against human VEGF

Recombinant form of VEGF-A₁₀₉ was conjugated onto a CM5 chip using an amine coupling kit. Specifically, CM5 sensor chip was activated with EDC/NHS for 7 min at 5µl/min. 1 to 2µg/ml of VEGF-A₁₀₉ were injected for 30 sec at a flow rate of 10µl/min over the activated chip to give a maximum binding response unit (RU) of 100 to 400. After conjugation, FcRn coupled chip was blocked by an injection of 35µl of 1M ethanolamine hydrochloride at 5µl/min. Two-fold dilutions of antibodies from 100nM to 6nM were injected over the VEGF-conjugated chip for 4 min in PBS/0.05% Tween/0.02% NaN₃ at 37°C. The complexes were allowed to dissociate for 18 min. The chip was regenerated with a 30 sec pulse of 20mM hydrochloric acid. Antibodies were also injected over an unconjugated surface for background subtraction. Results in **Figure 9** show that the Fc mutations do not alter the VEGF binding, and that all of the variants have the same binding response as wildtype.

### Example 7: In-vitro inhibition of cell proliferation

Various concentrations of anti-VEGF wildtype (Bevacizumab) and anti-VEGF variants were pre-incubated with recombinant human VEGF at room temperature for 1 hr. The concentrations of anti-VEGF wildtype (Bevacizumab) and anti-VEGF variants ranged from 33nM to 0.05nM. The concentration of recombinant human VEGF was 0.26nM. The complexes were then presented to the human umbilical vascular endothelial cells (HUVEC) in culture at 37°C and 5% CO₂. Viabilities of HUVEC after 4 days of culture were assessed by incubating the cells with 20% of ALAMAR BLUE® dye (Trek Diagnostic Systems, Cleveland, OH) for 6 hr at 37°C and 5% CO₂. Fluorescence of ALAMAR BLUE® was then detected with a Molecular Devices (Sunnyvale, CA) microplate reader. As shown in **Figure 10****,** all of the variants have the same level of proliferation inhibition as the wildtype and AVASTIN®, again confirming that the Fc mutations do not affect the variant's ability to neutralize VEGF.

### Example 8: Pharmacokinetic studies in cynomolgus monkeys

Thirty-six male and thirty-six female naive cynomolgus monkeys, weighed 2 - 5 kg and were 2 to 7 years old at pre-study physical examination, were assigned to six treatment groups each consisting of six males and six females. Animals were assigned to treatment groups using a computerized blocking procedure designed to achieve body weight balance with respect to treatment groups. Only animals that appeared to be healthy and that were free of obvious abnormalities were used in the study. All animals received a single intravenous bolus dose via saphenous vein followed by a 0.9% saline flush on day 1. The dose level for all groups was 5 mg/kg. Blood samples (approximately 1.0 mL) from the femoral vein were collected pre-dose and post-dose at 0.5, 2, 4, 8 hours, 1, 2, 4, 7, 10, 14, 21, 28, 35, 42, 49, 56 and 70 days. The serum concentration-time curves for all groups were constructed using the mean serum concentration of n=11 to 12 animals per group. Serum samples collected in this experiment were analyzed using an ELISA protocol described in Example 9.

### Example 9: Detection of antibody concentration in cynomolgus monkey serum by ELISA

Maxisorp ELISA plates (Thermo Fisher Scientific, Rochester, NY) were coated with 0.5 µg/ml of recombinant human VEGF in 50mM carbonate buffer, pH 9.6, at 4°C overnight. Plates were blocked with PBS, 0.5% BSA, 10ppm Proclin, pH 7.2 for 1 hr at room temperature and then washed with wash buffer (PBS/0.05% Tween 20/pH 7.2). Two folds serially diluted standards (anti-VEGF IgGi wildtype (Bevacizumab)) as well as 3-folds serially diluted cyno serum samples (starting at 1:10) in PBS buffer containing 0.5% bovine serum albumin, 0.05% Tween 20, 5mM EDTA pH 8.0, 0.25% CHAPS, 0.2% bovine gamma globulin, 10ppm Proclin and 0.35M NaCl were added to the blocked plates and incubated for 2 hrs at room temperature with shaking. Plates were washed 6 times, and bound drug was detected with sheep anti-human IgG (Fc specific)-HRP (Jackson ImmunoResearch, West Grove, PA) diluted 1:10K in assay buffer (PBS, pH 7.4, 0.5% BSA, 0.05% Tween 20, 10ppm Proclin) for 1 hr at room temperature with shaking. Plates were then washed 6 times again, followed by the addition of tetramethyl benzidine substrate (Moss, Pasadena, MD) for color development. The reaction was stopped after 20 minutes by the addition of 1M phosphoric acid (H₃PO₄). Plates were read on a Molecular Devices microplate reader at a wavelength of 450-620 nm. The serum profiles of the wildtype and five anti-VEGF variants in cynomolgus monkeys following a single IV dose of 5 mg/kg are shown in **Figure 11****.** All five variants exhibited reduced clearance and prolonged half-life compared to wildtype.

### Example 10: Pharmacokinetic data analysis

PK parameters were estimated using WinNonLin-Enterprise, version 5.1.1 (Pharsight Corporation; Mountain View, CA). A two-compartment model with IV-bolus input, first-order elimination, and micro-rate constants (Model 7) was used to describe the observed data. Concentrations were weighted using iterative reweighting (predicted to power n=-1) and the Gauss-Newton minimization algorithm with Levenberg and Hartley modification. The following PK parameters were reported using WinNonLin Model 7: AUC_{∞} = total drug exposure defined as area under the concentration-time curve extrapolated to infinity; *t*_{1/2,α} = half-life of the alpha phase (alpha half-life); *t*_{1/2, β} = half-life of the beta phase (beta half-life); Cₘₐₓ= maximum observed concentration; CL = clearance; V₁ = volume of the central compartment; Vₛₛ = volume of distribution at steady state.

For all dose groups, model selection was based on goodness of fit by visual inspection of the observed versus predicted serum concentration-time profile for each animal, examination of the weighted residuals sum of squares, and examination of the standard error and coefficient of variation for each parameter. PK parameters were presented as the mean ± standard deviation (SD) of each group.

**Figure 12** shows the tabulated pharmacokinetics parameters for the anti-VEGF wildtype and five variants, N434H, T307Q/N434A, T307Q/N434S, T307Q/E380A/N434S and V308P/N434A, following a single IV dose of 5 mg/kg to cynomolgus monkeys. The β (terminal) half-lives of variants are about 1.6- to 2.2- fold longer than the half-life of wildtype, with T307Q/N434A variant having the longest half-life of 24.9 days. To our knowledge, the half-life of the variant T307Q/434A, about 25 days, represents the longest half-life of a human IgG in cynomolgus monkey yet reported.

The relationship between half-life and FcRn affinity is shown in **Figure 13****.** Modest increase in pH 6.0 FcRn affinity results in prolonged terminal half-life, as evidenced by N434H and T307Q/N434A variants. However, additional increase in pH 6.0 FcRn affinity (T307Q/N434S, T307Q/E380A/N434A and V308P/N434A) does not further improved half-life, as slower dissociation rate and increased affinity at neutral pH may compensate for the benefit brought forth from the acidic-pH affinity increase. Instead, there is a trend towards reduced half-life at higher FcRn affinities at pH 6.0.

### Example 11: Pharmacokinetic studies in transgenic mice

The strain of mice used in the study is Mu.VEGFhuX.KI.R1.B6.129. MuVEGFhuMUTX (+/+) knock-in, RAG2 (-/-) knock-out mice contain two alleles of humanized forms of VEGF, which can be neutralized by wild-type anti-VEGF antibody (Bevacizumab). RAG2 (-/-) mice are immuno-deficient and do not generate functional T and B cells. Human tumors can be grown in these mice, which express humanized forms of VEGF in the absence of an overt immune response toward the tumor cells. Thus, VEGF derived from the human tumor and mouse stromal cell will be neutralized by wild-type anti-VEGF antibody (Bevacizumab), which does not neutralize murine VEGF. The PK of anti-VEGF wild-type and anti-VEGF variant T307Q/N434A were evaluated in these non-tumor bearing transgenic mice.

Two different PK studies were performed. The first study is a single-dose PK study. There are 4 groups with 8-9 animals per group, each receiving a single intravenous dose of 0.3 or 5 mg/kg of wildtype and variant T307Q/N434A in PBS. Dose volume to be administered varied from 5 to 15ml/kg depending on the concentration of the dosing solution and the weight of each animal. IV dosing was done via the tail vein. Samples from 3 mice were bled at each time point and about 125uL of blood samples for PK analysis were collected at 15 minutes, 8 hours, 24 hours, 2, 4, 7, 10, 14, 21 and 28 days post-dose. Blood samples were collected under anesthesia via periorbital sinus. At sac time points, mice were bled by cardiac puncture under isoflurane anesthesia.

The second study is a multi-dose PK study. There are 9 animals per group. Each animal received 0.3 or 5 mg/kg of variant T307Q/N434A in PBS at day 0, 3, 6, and 9. The methods of injection and samples collection were similar to the single-dose PK study. However, blood samples were collected at 15 min post first dose, day 3 (pre-dose), day 6 (pre-dose), day 9 (pre-dose), 15min post day-9 dose, day 11, day 14, day 21, day 28 and day 35.

Serum samples collected from the PK studies were analyzed using ELISA described in Examples 13. **Figures 14A** and **14B** show the pharmacokinetic profiles of the wildtype and variant T307Q/N434A determined using either VEGF capture **(****Fig. 14A****)** or human Fc capture **(****Fig. 14B****)** ELISA, respectively. Variant T307Q/N434A has similar PK profiles are wildtype following a single IV dose of 0.3 or 5 mg/kg. **Figure 15** confirms that the half-lives of wildtype and T307Q/N434A variant in transgenic mice are comparable. However, non-linear PK responses were observed, as antibodies dosed at 0.3mg/kg have shorter half-lives than that dosed at 5mg/kg, possibly due to the antigen dependent clearance. **Figure 16** shows the pharmacokinetic profiles of variant T307Q/N434A in humanized VEGF transgenic mice following a multi-dose of 0.3 or 5 mg/kg and the PK parameters are summarized in **Figure 17****.** Results show that the serum concentrations measured experimentally corresponded well with the concentrations predicted by a simulation using the single-dose PK parameters.

### Example 12: In vivo efficacy studies

Human HT-55, Colo-205 (colorectal carcinoma) and Calu-6 (lung carcinoma) cells were obtained from the American Type Culture Collection (Manassas, VA). The human colorectal carcinoma HM-7 cell line is a derivative of LS 174T. The Calu-6 and HM-7 were grown in Ham's F12, low glucose DMEM 1:1. Colo-205 and HT-55 were grown in RPMI 1640 medium. Both media were supplemented with 10% v/v FBS, 1% v/v penicillin/streptomycin (Invitrogen, Carlsbad, CA), 2 mM L-glutamine (Invitrogen, Carlsbad, CA) and 1 µg/ml FUNGIZONE™ (Invitrogen, Carlsbad, CA). Cells were grown at 37°C in 5% CO₂ until confluent, harvested, and resuspended in sterile Matrigel at 50 x 10⁶ cells per ml. Xenografts were established in 6- to 8-week-old RAG2 KO; hum-X VEGF KI double-homozygous mice (Genentech, South San Francisco, CA) by dorsal flank s.c. injection of 5 x10⁶ cells per mouse and allowed to grow. The treatment with antibody i.p. at the dose of 5, 0.5 and 0.05 mg/kg twice weekly were initiated 24 h after tumor cell inoculation. The transplanted tumors were measured twice weekly along the longest axis and the perpendicular axis as described. For each day on which tumors were measured, the tumor volume for each mouse was calculated, and the mean tumor volumes from the control antibody group (anti-Ragweed) and each anti-VEGF group were compared by Student's ttest, at a level of *P* < 0.05. Mice were killed when tumor volume reached 2,000 mm³.

Results from the first HM-7 xenografts study are shown in **Figure 18****.** **Figures 18A** and **18B** show that the variant T307Q/N434A was able to achieve maximum inhibition of tumor growth at both 0.5 mg/kg as well as 5 mg/kg treatment groups. Although the wild-type did inhibit tumor growth at both doses, it did not achieve maximum inhibition of tumor growth at 0.5 mg/kg. These results suggest that the T307Q/N434A variant is more efficacious than wildtype at 0.5mg/kg in treating HM-7 xenografts, despite similar levels of serum IgG concentration **(****Fig. 18C****).** A repeat efficacy study of HM-7 xenografts shown in **Figure 19** confirms that the T307Q/N434A variant is more efficacious than wildtype at 0.5 and 0.05mg/kg treatment groups. Indeed, the T307Q/N434A variant showed greater inhibition of tumor growth in all treatment groups compared to the wild-type **(****Fig. 19A** and **19B****).** The increased efficacy may be due to the higher blood-normalized antibody concentration in tumors for T307Q/N434A treated group compared to wildtype **(****Fig. 19E****).** The third efficacy study of HM-7 xenografts shown in **Figure 20** further validates the superior efficacy of T307Q/N434A over wildtype at 0.5 and 0.05mg/kg treatment groups.

For the HT-55 xenografts study, T307Q/N434A variant showed greater inhibition of tumor growth compared to wild-type in 0.05mg/kg treatment groups **(****Fig. 21****)** suggesting that T307Q/N434A is more efficacious than wildtype at 0.05mg/kg treatment groups. For the Colo-205 study, **Figure 22B** shows a significant difference in growth curves between 0.5mg/kg wild-type and 0.5mg/kg T307Q/N434A variant. **Figure 22A** and **22B** also shows an increase in inhibition of tumor growth by T307Q/N434A in 0.5 and 0.05mg/kg treatment groups, suggesting slightly higher efficacy for T307Q/N434A in 0.5 and 0.05mg/kg groups. A repeat Colo-205 study shown in **Figure 23** indicates that T307Q/N434A is slightly more efficacious than wildtype in treating Colo-205 xenografts. Finally, the efficacies of T307Q/N434A variant and wildtype in Calu-6 xenografts study were similar.

There may be several possible reasons for increased efficacy of the Fc variants. For example, the increased potency of a variant could be due to increased retention and/or recycling of the variant antibody mediated by the human FcRn expressed in certain tumors (*e.g.,* HM-7). This may lead to an increased mass-action effect of blocking locally produced VEGF, or may provide a mechanism for enhanced degradation of VEGF in the tumor. However, we found that the increased concentration of variant detected in the HM-7 tumors relative to the HT-55 and Calu-6 tumors is not directly correlated with cellular FcRn expression level, as HM-7 cells express lower amounts of FcRn than either HT-55 or Calu-6 cells **(****Figure 25****).** Other factors in the tumor microenvironment such as tumor pH, growth rate, and other tumor constituents may also play collaborative roles with FcRn in determining the distribution of IgGs. For example, the tumor microenvironment is mostly acidic, with pH ranging from 6.0 to 7.6 (median=7.1), while that of normal tissues ranges from 7.3 to 7.8 (median=7.55), *see* Song, C.W. et al., "Influence of Tumor pH on Therapeutic Response " in Cancer Drug Resistance, 21-42 (2007). Furthermore, different types of tumors can have a wide range of pH due to heterogeneous vascular supply and blood perfusion, *see* Song, C.W. et al., Cancer Drug Resistance, 21-42 (2007), *supra;* Gillies, R.J. et al., J Magn Reson Imaging 16, 430-450 (2002). Multiple *in-vitro* studies indicate that the amount of cell-associated Fc/IgG increases when cells were incubated at acidic pH, *see* Praetor, A. et al., Journal of cell science 112 (Pt 14), 2291-2299 (1999); McCarthy, K.M., Yoong, Y. & Simister, N.E. Bidirectional transcytosis of IgG by the rat neonatal Fc receptor expressed in a rat kidney cell line: a system to study protein transport across epithelia. Journal of cell science 113 (Pt 7), 1277-1285 (2000); Tesar, D.B. et al., Traffic 7, 1127-1142 (2006). Therefore, it is conceivable that pH differences among the tumor lines tested may affect the accumulation level of antibody within each tumor. Additionally, the acidic tumor microenvironment also activates VEGF expression (*see* Song, C.W. et al., Cancer Drug Resistance, 21-42 (2007), *supra*), which could mediate the retention of these anti-VEGF antibodies specifically. Furthermore, HM-7 tumors in mice were previously shown to have sparse stroma (*see* Liang, W. C. et al., J Biol Chem 281, 951-961 (2006)), while Calu-6 tumors induced strong host stromal response and was relatively stroma-rich (*see* Tejada, M. L. et al., Clin Cancer Res 12, 2676-2688 (2006)). The presence of mouse stromal cells, which express murine FcRn, may mask the improved recycling of an IgG variant by human tumor cells. This may lead to the lower concentrative effect in human xenografts having a greater component of murine stroma, for example, Calu-6.

### Example 13: Detection of antibody concentrations in serums and tumors of transgenic mice by ELISA

Two different ELISA assay formats with either two different antibody capture reagents (VEGF or anti-human IgG₁ Fc) coated on plates were used to detect antibody concentrations in transgenic mice. Maxisorp ELISA plates (Thermo Fisher Scientific, Rochester, NY) were coated with either 0.5 µg/ml of recombinant human VEGF or 0.25µg/ml (Fab'2) rabbit anti-human IgG₁ Fc (Jackson ImmunoResearch, West Grove, PA) in 50mM carbonate buffer, pH 9.6, at 4°C overnight. Plates were blocked with PBS, 0.5% BSA, 10ppm Proclin, pH 7.2 for 1 hr at room temperature and then washed with wash buffer (PBS/0.05% Tween 20/pH 7.2). Two folds serially diluted standards (Bevacizumab for VEGF format or human IgGi for Fc format) down to 12.5ng/ml as well as 3-folds serially diluted cyno serum samples (starting at 1:10) in PBS buffer containing 0.5% bovine serum albumin, 0.05% Tween 20, 5mM EDTA pH 8.0, 0.25% CHAPS, 0.2% bovine gamma globulin, 10ppm Proclin and 0.35M NaCl were added to the blocked plates and incubated for 2 hrs at room temperature with shaking. Plates were washed 6 times, and bound drug was detected with goat (Fab'2) anti-human IgG (Fc specific)-HRP conjugate (Jackson) diluted 1:20K to 1:60K in assay buffer (PBS, pH 7.4, 0.5% BSA, 0.05% Tween 20, 10ppm Proclin) for 1hr at room temperature with shaking. Plates were then washed 6 times again, followed by the addition of tetramethyl benzidine substrate (Moss, Pasadena, MD) for color development. The reaction was stopped after 20 minutes by the addition of 1M phosphoric acid (H₃PO₄). Plates were read on a Molecular Devices microplate reader at a wavelength of 450-620 nm.

### Example 14: IgG₁ Fc variants with various affinity improvements over wildtype and their in-vivo pharmacokinetic behaviors

Additional combinations of Fc mutations, shown in **Figure 24****,** were incorporated into human anti-HER2 (tratuzumab) to construct IgG variants. The IgGi variants were expressed using methods described in Example 1. The dissociation constants of the wild-type anti-HER2 IgGi and anti-HER2 IgGi variants are measured as described in Example 4 and the results are shown in **Fig. 24****.** Results show that by combining different mutations, we can construct an IgG variant such as M252Y/V308P/N434Y that is able to bind human FcRn with single-digit nanomolar affinity, representing an about 450-fold improvement over the wild-type IgGi.

However, high-affinity variants do not necessarily have improved pharmacokinetic behaviors *in vivo.* For example, two Fc mutations, N434A and N434W, were incorporated into a different human antibody to construct two IgGi variants. The two IgGi variants, N434A variant and N434W variant, resulted in approximately 3-fold and 40-fold higher FcRn affinity at pH 6.0 compared to the wildtype antibody, respectively, as shown in **Fig. 24****.** Their pharmacokinetic behavior was evaluated in cynomolgus monkeys, as described in Example 8 and 9, and compared to that of wildtype (approximately 6 to 9 days). The half-life of N434W (about 9.7 +/- 2.4 days) was less improved than that of the modestly affinity-improved variant, N434A (about 14.5 +/-2.2 days). These results suggest that too much increase in FcRn affinity may have a detrimental effect on the half-life of an Fc variant, and it is difficult to predict *a priori* whether an Fc variant with improved FcRn affinity will have improved half-life or not.

### Example 15: FcRn immunoprecipitation using a high-affinity IgG₁ variant.

Five million cells/each of either HM-7, HT-55, Calu-6 or Raji (B-cell lymphoma) lines were lysed by incubating in 25mM sodium phosphate buffer pH 6.0 containing 1% Nonidet P-40, 0.5% sodium deoxycholate, 0.1% SDS, 2mM EDTA, 150mM NaCl and IX protease inhibitor (Pierce, Rockland, IL) for 1hr at 4°C. Lysed cells were centrifuged at 12,000g for 30min at 4°C, and then 50 nM of trastuzumab Fc variant M252Y/V308P/N434Y (Yeung *et al.,* submitted) was added to the supernatant to capture the FcRn. After overnight incubation at 4°C, Protein-L (Pierce) resin was added and allowed to bind the complex for 4hr at 4°C. Resin was then washed five times with the lysis buffer and bound proteins were eluted with a 2X loading buffer (Invitrogen, Carlsbad, CA). Proteins were separated on a 4-12% BIS-TRIS gel (Invitrogen) and blotted onto a nitrocellulose membrane (Invitrogen). The membrane was blocked with 3% nonfat milk in PBS and probed with 1ng/ml of rabbit anti-human FcRn antibody (Santa Cruz, Santa Cruz, CA) at room temperature for 1 hr, then with goat anti-rabbit IgG-peroxidase conjugate at 1:10⁴ dilution (Pierce) for 1 hr at room temperature. Membrane was washed with PBS/0.05% Tween in between blocking and antibody incubation steps. The FcRn protein was visualized by the ECL detection kit (GE Healthcare, Piscataway, NJ). *See* **Figure 25****.**

### RESULTS and DISCUSSION

Two separate binding experiments with different anti-VEGF variants were performed at each pH, pH 6.0 and pH 7.4. The steady state binding response unit (RU) as a function of concentration for pH 6.0 and pH 7.4 was plotted in **Figure 1** and **Figure 2****,** respectively. The dissociation constants (K_{D}) at pH 6.0 were estimated from **Figure 1** and summarized in **Figure 2****.** The dissociation constants of the same variant calculated from two different runs were slightly different. For instance, variant N434A had a K_{D} of 550nM in the first run, but its K_{D} from the second run was 250nM. The difference was due to the avidity effect in the assay format which involved flowing a bivalent antibody over an FcRn-coupled chip. The level of avidity contribution to the dissociation constant depended on the level of FcRn coupled onto the chips, with higher level of FcRn coupling resulting in more avidity. This might explain the higher affinity observed in the second run, which had about two fold higher RU than the first run. Although there was avidity effect in the assay setup, this format most resembled the natural binding process inside the cells, where pinocytosed bivalent antibodies are allowed to bind to the membrane bound FcRn. While the absolute K_{D} might differ from run to run, the affinity ranking of these variants were consistent even with different level of FcRn coupled. Both **Figure 1** and **Figure 2** showed that V308P/N434A consistently had the highest affinity among the variants tested and all of variants tested have improved binding to FcRn at pH 6.0.

The affinities of the anti-VEGF variants to FcRn at pH 7.4 are much lower than their affinities at pH 6.0. Since the binding affinity at pH 7.4 was very low, the dissociation constants of the variants at pH 7.4 were not determined. However, **Figure 3** showed that the affinity ranking of these variants at pH 7.4 was the same as the ranking at pH 6.0, indicating the variants' binding to FcRn at pH 6.0 and pH 7.4 was coupled. As the variants' pH 6.0 binding is improved, so is the binding at pH 7.4. There is a delicate balance between how the pH 6.0 and 7.4 binding to FcRn affects the variant's half-life. Improved binding at pH 6.0 is suggested to have a beneficial role in variants' *in vivo* half-life, as higher affinity variants can bind FcRn better and hence be recycled more by the FcRn. On the other hand, substantially high binding at pH 7.4 is proposed to be unfavorable for variants' half-life, as the FcRn-bound antibodies may not be readily released back into circulation if they are bound too tightly.

Increased affinity at high pH can negate the favorable effects of increased affinity at low pH. For example, Dall'Acqua et al., J Immunol 169(9), 5171-5180, 2002, showed that human IgGi variants such as M252Y/S254T/T256E and G385D/Q386P/N389S did not have improved half-lives in mice, apparently because of their high pH binding affinity to murine FcRn. Therefore, it has been unclear how much improvement in high-pH binding can be tolerated in IgGi variants while still improving their pharmacokinetic half-lives.

## Claims

1. A variant IgG comprising a human IgG1 Fc region wherein the variant has the following substitutions relative to a wild-type human IgG1 Fc region, numbered according to the EU index as in Kabat, and has an increased half-life compared to the half-life of said IgG having the wild-type human IgG1 Fc region:
threonine at amino acid 307 of the Fc region is substituted with glutamine and asparagine at amino acid 434 of the Fc region is substituted with alanine; or
threonine at amino acid 307 of the Fc region is substituted with glutamine, glutamic acid at amino acid amino acid 380 of the Fc region is substituted with alanine, and asparagine at amino acid 434 of the Fc region is substituted with serine.

2. The variant IgG of claim 1, wherein the variant IgG is an anti-VEGF antibody.

3. The variant IgG of claim 1 or 2 comprising the heavy chain variable domain comprising SEQ ID NO: 1 and the light chain variable domain comprising SEQ ID NO: 2.

4. A pharmaceutical composition comprising the variant IgG of any one of claims 1 - 3 and a pharmaceutically acceptable carrier.

5. A kit comprising the variant IgG of any one of claims 1-3, in a container comprising a sterile access port, a pharmaceutically acceptable carrier, and instructions for use.

6. A variant IgG according to any one of claims 2 - 4, for use in a method of treating tumor in a subject, said method comprising administering to the subject an effective amount of the variant IgG.

7. A variant IgG according to any one of claims 2-4 for use in a method of inhibiting VEGF activity, wherein the VEGF activity is angiogenesis, in a subject with a disease or condition wherein the angiogenesis is involved, said method comprising administering to said subject an effective amount of the variant IgG.

8. A variant IgG according to any one of claims 2-4, for use in a method of inhibiting or preventing growth of cancer cells in a subject, said method comprising administering to said subject an effective amount of the variant IgG.

9. A variant IgG for the use of any of claims 6-8, wherein the method comprises administering the variant IgG to the subject every 4 weeks or longer.

## Patentansprüche

1. IgG-Variante, die eine humane IgG1-Fc-Region umfasst, wobei die Variante relativ zu einer humanen IgG1-Fc-Region vom Wildtyp die folgenden, gemäß dem EU-Index nach Kabat nummerierten Substitutionen aufweist und im Vergleich zu der Halbwertszeit des IgG mit der humanen IgG1-Fc-Region vom Wildtyp eine erhöhte Halbwertszeit aufweist:
Threonin an Aminosäure 307 der Fc-Region ist durch Glutamin ersetzt und Asparagin an Aminosäure 434 der Fc-Region ist durch Alanin ersetzt; oder
Threonin an Aminosäure 307 der Fc-Region ist durch Glutamin ersetzt, Glutaminsäure an Aminosäure 380 der Fc-Region ist durch Alanin ersetzt, und Asparagin an Aminosäure 434 der Fc-Region ist durch Serin ersetzt.

2. IgG-Variante nach Anspruch 1, wobei die IgG-Variante ein Anti-VEGF-Antikörper ist.

3. IgG-Variante nach Anspruch 1 oder 2, die eine SEQ ID NO: 1 umfassende variable Schwerkettendomäne und eine SEQ ID NO: 2 umfassende variable Leichtkettendomäne umfasst.

4. Pharmazeutische Zusammensetzung, die eine IgG-Variante nach einem der Ansprüche 1 bis 3 und einen pharmazeutisch annehmbaren Träger umfasst.

5. Set, umfassend eine IgG-Variante nach einem der Ansprüche 1 bis 3, in einem Behältnis, das eine sterile Zugangsöffnung, einen pharmazeutisch annehmbaren Träger und Gebrauchsanweisungen umfasst.

6. IgG-Variante nach einem der Ansprüche 2 bis 4 zur Verwendung in einem Verfahren zum Behandeln eines Tumors bei einem Individuum, wobei das Verfahren das Verabreichen einer wirksamen Menge der IgG-Variante an das Individuum umfasst.

7. IgG-Variante nach einem der Ansprüche 2 bis 4 zur Verwendung in einem Verfahren zum Hemmen der VEGF-Aktivität, wobei die VEGF-Aktivität Angiogenese ist, bei einem Individuum mit einer Krankheit oder einem Leiden, an der bzw. bei dem Angiogenese beteiligt ist, wobei das Verfahren das Verabreichen einer wirksamen Menge der IgG-Variante an das Individuum umfasst.

8. IgG-Variante nach einem der Ansprüche 2 bis 4 zur Verwendung in einem Verfahren, um das Wachstum von Krebszellen in einem Individuum zu hemmen oder diesem vorzubeugen, wobei das Verfahren das Verabreichen einer wirksamen Menge der IgG-Variante an das Individuum umfasst.

9. IgG-Variante zur Verwendung nach einem der Ansprüche 6 bis 8, wobei das Verfahren das Verabreichen der IgG-Variante an das Individuum alle 4 Wochen oder in größeren Zeitabständen umfasst.

## Revendications

1. Variant d'IgG comprenant une région Fc d'IgG1 humaine dans lequel le variant a les substitutions suivantes par rapport à une région Fc d'IgG1 humaine de type sauvage, numérotées selon l'indice EU comme dans Kabat, et a une demi-vie augmentée par comparaison à la demi-vie desdites IgG ayant la région Fc d'IgG1 humaine de type sauvage :
de la thréonine au niveau de l'acide aminé 307 de la région Fc est substituée par de la glutamine, et de l'asparagine au niveau de l'acide aminé 434 de la région Fc est substituée par de l'alanine ; ou
de la thréonine au niveau de l'acide aminé 307 de la région Fc est substituée par de la glutamine, de l'acide glutamique au niveau de l'acide aminé 380 de la région Fc est substituée par de l'alanine, et de l'asparagine au niveau de l'acide aminé 434 de la région Fc est substituée par de la sérine.

2. Variant d'IgG selon la revendication 1, dans lequel le variant d'IgG est un anticorps anti-VEGF.

3. Variant d'IgG selon la revendication 1 ou 2 comprenant le domaine variable de chaîne lourde comprenant SEQ ID NO: 1 et le domaine variable de chaîne légère comprenant SEQ ID NO: 2.

4. Composition pharmaceutique comprenant le variant d'IgG selon l'une quelconque des revendications 1 à 3 et un support pharmaceutiquement acceptable.

5. Kit comprenant le variant d'IgG selon l'une quelconque des revendications 1 à 3, dans un contenant comprenant un orifice d'accès stérile, un support pharmaceutiquement acceptable, et des instructions d'utilisation.

6. Variant d'IgG selon l'une quelconque des revendications 2 à 4, destiné à être utilisé dans un procédé de traitement d'une tumeur chez un sujet, ledit procédé comprenant l'administration au sujet d'une quantité efficace du variant d'IgG.

7. Un variant d'IgG selon l'une quelconque des revendications 2 à 4 destiné à être utilisé dans un procédé d'inhibition d'activité de VEGF, dans lequel l'activité de VEGF est l'angiogenèse, chez un sujet souffrant d'une maladie ou d'un état où l'angiogenèse est impliquée, ledit procédé comprenant l'administration audit sujet une quantité efficace du variant d'IgG.

8. Variant d'IgG selon l'une quelconque des revendications 2 à 4, destiné à être utilisé dans un procédé de traitement ou de prévention d'une tumeur chez un sujet, ledit procédé comprenant l'administration audit sujet d'une quantité efficace du variant d'IgG.

9. Variant d'IgG destiné à être utilisé selon l'une quelconque des revendications 6 à 8, dans lequel le procédé comprend l'administration du variant d'IgG au sujet toutes les 4 semaines ou plus.


### Sequence Listing

<110> GENENTECH, INC., et al.
<120> Immunoglobulin Variants and Uses Thereof
<130> P4182R1 WO
<150> US 61/105,086
   <151> 2008-10-14
<150> US 61/152,131
   <151> 2009-02-12
<150> US 61/171,768
   <151> 2009-04-22
<150> US 61/220,514
   <151> 2009-06-25
<160> 17
<210> 1
   <211> 123
   <212> PRT
   <213> Artificial sequence
<220>
   <223> sequence is synthesized
<400> 1
<210> 2
   <211> 108
   <212> PRT
   <213> Artificial sequence
<220>
   <223> sequence is synthesized
<400> 2
   Asp Ile Gln Met Thr Gln Ser Pro Ser Ser Leu Ser Ala Ser Val
   1               5                   10                  15
   Gly Asp Arg Val Thr Ile Thr Cys Ser Ala Ser Gln Asp Ile Ser
               20                  25                  30
   Asn Tyr Leu Asn Trp Tyr Gln Gln Lys Pro Gly Lys Ala Pro Lys
           35                  40                  45
   Val Leu Ile Tyr Phe Thr Ser Ser Leu His Ser Gly Val Pro Ser
       50                  55                  60
   Arg Phe Ser Gly Ser Gly Ser Gly Thr Asp Phe Thr Leu Thr Ile
   65                  70                  75
   Ser Ser Leu Gln Pro Glu Asp Phe Ala Thr Tyr Tyr Cys Gln Gln
   80                  85                  90
   Tyr Ser Thr Val Pro Trp Thr Phe Gly Gln Gly Thr Lys Val Glu
   95                  100                 105
   Ile Lys Arg
<210> 3
   <211> 118
   <212> PRT
   <213> Artificial Sequence
<220>
   <223> sequence is synthesized
<400> 3
   Glu Val Gln Leu Val Glu Ser Gly Gly Gly Leu Val Gln Pro Gly
   1               5                   10                  15
   Gly Ser Leu Arg Leu Ser Cys Ala Ala Ser Gly Tyr Asp Phe Thr
               20                  25                  30
   His Tyr Gly Met Asn Trp Val Arg Gln Ala Pro Gly Lys Gly Leu
           35                  40                  45
   Glu Trp Val Gly Trp Ile Asn Thr Tyr Thr Gly Glu Pro Thr Tyr
       50                  55                  60
   Ala Ala Asp Phe Lys Arg Arg Phe Thr Phe Ser Leu Asp Thr Ser
   65                  70                  75
   Lys Ser Thr Ala Tyr Leu Gln Met Asn Ser Leu Arg Ala Glu Asp
   80                  85                  90
   Thr Ala Val Tyr Tyr Cys Ala Lys Tyr Pro Tyr Tyr Tyr Gly Thr
   95                  100                 105
   Ser His Trp Tyr Phe Asp Val Trp Gly Gln Gly Thr Leu
   110                 115
<210> 4
   <211> 110
   <212> PRT
   <213> Artificial Sequence
<220>
   <223> sequence is synthesized
<400> 4
   Asp Ile Gln Leu Thr Gln Ser Pro Ser Ser Leu Ser Ala Ser Val
   1               5                   10                  15
   Gly Asp Arg Val Thr Ile Thr Cys Ser Ala Ser Gln Asp Ile Ser
                   20                  25                  30
   Asn Tyr Leu Asn Trp Tyr Gln Gln Lys Pro Gly Lys Ala Pro Lys
                   35                  40                  45
   Val Leu Ile Tyr Phe Thr Ser Ser Leu His Ser Gly Val Pro Ser
                   50                  55                  60
   Arg Phe Ser Gly Ser Gly Ser Gly Thr Asp Phe Thr Leu Thr Ile
                   65                  70                  75
   Ser Ser Leu Gln Pro Glu Asp Phe Ala Thr Tyr Tyr Cys Gln Gln
                   80                  85                  90
   Tyr Ser Thr Val Pro Trp Thr Phe Gly Gln Gly Thr Lys Val Glu
                   95                  100                 105
   Ile Lys Arg Thr Val
                   110
<210> 5
   <211> 118
   <212> PRT
   <213> Artificial sequence
<220>
   <223> sequence is synthesized
<400> 5 110 115
<210> 6
   <211> 111
   <212> PRT
   <213> Artificial sequence
<220>
   <223> sequence is synthesized
<400> 6
   Asp Ile Val Met Thr Gln Ser Pro Asp Ser Leu Ala Val Ser Leu
    1               5                  10                  15
   Gly Glu Arg Ala Thr Ile Asn Cys Lys Ala Ser Gln Ser Val Asp
                   20                  25                  30
   Tyr Asp Gly Asp Ser Tyr Met Asn Trp Tyr Gln Gln Lys Pro Gly
                   35                  40                  45
   Gln Pro Pro Lys Leu Leu Ile Tyr Val Ala Ser Asn Leu Glu Ser
                   50                  55                  60
   Gly Val Pro Asp Arg Phe Ser Gly Ser Gly Ser Gly Thr Asp Phe
                   65                  70                  75
   Thr Leu Thr Ile Ser Ser Leu Gln Ala Glu Asp Val Ala Val Tyr
                   80                  85                  90
   Tyr Cys Gln Gln Ser Leu Gln Asp Pro Pro Thr Phe Gly Gly Gly
                   95                 100                 105
   Thr Lys Val Glu Ile Lys
                  110
<210> 7
   <211> 449
   <212> PRT
   <213> Artificial sequence
<220>
   <223> sequence is synthesized
<400> 7
   Glu Val Gln Leu Val Glu Ser Gly Gly Gly Leu Val Gln Pro Gly
   1               5                   10                  15
   Gly Ser Leu Arg Leu Ser Cys Ala Ala Ser Gly Phe Asn Ile Lys
                   20                  25                  30
   Asp Thr Tyr Ile His Trp Val Arg Gln Ala Pro Gly Lys Gly Leu
                   35                  40                  45
   Glu Trp Val Ala Arg Ile Tyr Pro Thr Asn Gly Tyr Thr Arg Tyr
                   50                  55                  60
   Ala Asp Ser Val Lys Gly Arg Phe Thr Ile Ser Ala Asp Thr Ser
                   65                  70                  75
   Lys Asn Thr Ala Tyr Leu Gln Met Asn Ser Leu Arg Ala Glu Asp
                   80                  85                  90
   Thr Ala Val Tyr Tyr Cys Ser Arg Trp Gly Gly Asp Gly Phe Tyr
   95 100 105
   Ala Met Asp Tyr Trp Gly Gln Gly Thr Leu Val Thr Val Ser Ser
   110 115 120
   Ala Ser Thr Lys Gly Pro Ser Val Phe Pro Leu Ala Pro Ser Ser
   125 130 135
   Lys Ser Thr Ser Gly Gly Thr Ala Ala Leu Gly Cys Leu Val Lys
   140 145 150
   Asp Tyr Phe Pro Glu Pro Val Thr Val Ser Trp Asn Ser Gly Ala
   155 160 165
   Leu Thr Ser Gly Val His Thr Phe Pro Ala Val Leu Gln Ser Ser
   170 175 180
   Gly Leu Tyr Ser Leu Ser Ser Val Val Thr Val Pro Ser Ser Ser
   185 190 195
   Leu Gly Thr Gln Thr Tyr Ile Cys Asn Val Asn His Lys Pro Ser
   200 205 210
   Asn Thr Lys Val Asp Lys Lys Val Glu Pro Lys Ser Cys Asp Lys
   215 220 225
   Thr His Thr Cys Pro Pro Cys Pro Ala Pro Glu Leu Leu Gly Gly
   230 235 240
   Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser
   260 265 270
   His Glu Asp Pro Glu Val Lys Phe Asn Trp Tyr Val Asp Gly Val
   275 280 285
   Glu Val His Asn Ala Lys Thr Lys Pro Arg Glu Glu Gln Tyr Asn
   290 295 300
   Ser Thr Tyr Arg Val Val Ser Val Leu Thr Val Leu His Gln Asp
   305 310 315
   Trp Leu Asn Gly Lys Glu Tyr Lys Cys Lys Val Ser Asn Lys Ala
   320 325 330
   Leu Pro Ala Pro Ile Glu Lys Thr Ile Ser Lys Ala Lys Gly Gln
   335 340 345
   Pro Arg Glu Pro Gln Val Tyr Thr Leu Pro Pro Ser Arg Glu Glu
   350 355 360
   Met Thr Lys Asn Gln Val Ser Leu Thr Cys Leu Val Lys Gly Phe
   365 370 375
   Tyr Pro Ser Asp Ile Ala Val Glu Trp Glu Ser Asn Gly Gln Pro
   380 385 390
   Glu Asn Asn Tyr Lys Thr Thr Pro Pro Val Leu Asp Ser Asp Gly
   395 400 405
   Ser Phe Phe Leu Tyr Ser Lys Leu Thr Val Asp Lys Ser Arg Trp
   410 415 420
   Gln Gln Gly Asn Val Phe Ser Cys Ser Val Met His Glu Ala Leu
   425 430 435
   His Asn His Tyr Thr Gln Lys Ser Leu Ser Leu Ser Pro Gly
   440 445
<210> 8
   <211> 214
   <212> PRT
   <213> Artificial sequence
<220>
   <223> sequence is synthesized
<400> 8
   Asp Ile Gln Met Thr Gln Ser Pro Ser Ser Leu Ser Ala Ser Val
   1               5                   10                  15
   Gly Asp Arg Val Thr Ile Thr Cys Arg Ala Ser Gln Asp Val Asn
                   20                  25                  30
   Thr Ala Val Ala Trp Tyr Gln Gln Lys Pro Gly Lys Ala Pro Lys
                   35                  40                  45
   Leu Leu Ile Tyr Ser Ala Ser Phe Leu Tyr Ser Gly Val Pro Ser
                   50                  55                  60
   Arg Phe Ser Gly Ser Arg Ser Gly Thr Asp Phe Thr Leu Thr Ile
                   65                  70                  75
   Ser Ser Leu Gln Pro Glu Asp Phe Ala Thr Tyr Tyr Cys Gln Gln
                   80                  85                  90
   His Tyr Thr Thr Pro Pro Thr Phe Gly Gln Gly Thr Lys Val Glu
                   95                  100                 105
   Ile Lys Arg Thr Val Ala Ala Pro Ser Val Phe Ile Phe Pro Pro
                   110                 115                 120
   Ser Asp Glu Gln Leu Lys Ser Gly Thr Ala Ser Val Val Cys Leu
                   125                 130                 135
   Leu Asn Asn Phe Tyr Pro Arg Glu Ala Lys Val Gln Trp Lys Val
                   140                 145                 150
   Asp Asn Ala Leu Gln Ser Gly Asn Ser Gln Glu Ser Val Thr Glu
                   155                 160                 165
   Gln Asp Ser Lys Asp Ser Thr Tyr Ser Leu Ser Ser Thr Leu Thr
                   170                 175                 180
   Leu Ser Lys Ala Asp Tyr Glu Lys His Lys Val Tyr Ala Cys Glu
                   185                 190                 195
   Val Thr His Gln Gly Leu Ser Ser Pro Val Thr Lys Ser Phe Asn
                   200                 205                 210
   Arg Gly Glu Cys
<210> 9
   <211> 360
   <212> DNA
   <213> Artificial sequence
<220>
   <223> sequence is synthesized
<400> 9
   tcctgtgcag cctctggatt caccttcagt aacgcctgga tgagctgggt 50
   ccgccaggct ccagggaagg ggctggagtg ggtctccgct attagtggta 100
   gtggtaacac atactatgca gactccgtga agggccggtt caccatctcc 150
   agagacaatt ccaagaacac gctgtatctg caaatgaaca gcctgagagc 200
   cgaggacact gccgtgtatt actgtgcgag agcctcccac cgtatattag 250
   gttatgcttt tgatatctgg ggccagggta cactggtcac cgtgagctca 300
   gaggtgcagc tgttggagtc tgggggaggc ttggtacagc ctggggggtc 350
   cctgagactc 360
<210> 10
   <211> 327
   <212> DNA
   <213> Artificial sequence
<220>
   <223> sequence is synthesized
<400> 10
   tcttgttctg gaagccgctc caacatcggg agaaatgctg ttagttggta 50
   tcagcagctc ccaggaacgg cccccaaact cctcatctat gctaacagca 100
   atcggccctc aggggtccct gaccgattct ctggctccaa gtctggcacc 150
   tcagcctccc tggccatcag tgggctccgg tccgaggatg aggctgatta 200
   ttactgtgca gcatgggatg gcagcctgaa tggttgggtg ttcggcggag 250
   gaaccaagct gacggtccca gtctgtgctg actcagccac cctcagcgtc 300
   tgggaccccc gggcagaggg tcaccat 327
<210> 11
   <211> 227
   <212> PRT
   <213> Artificial sequence
<220>
   <223> sequence is synthesized
<400> 11
   Asp Lys Thr His Thr Cys Pro Pro Cys Pro Ala Pro Glu Leu Leu
   1               5                   10                  15
   Gly Gly Pro Ser Val Phe Leu Phe Pro Pro Lys Pro Lys Asp Thr
               20                  25                  30
   Leu Met Ile Ser Arg Thr Pro Glu Val Thr Cys Val Val Val Asp
   35 40 45
   Val Ser His Glu Asp Pro Glu Val Lys Phe Asn Trp Tyr Val Asp
   50 55 60
   Gly Val Glu Val His Asn Ala Lys Thr Lys Pro Arg Glu Glu Gln
   65 70 75
   Tyr Asn Ser Thr Tyr Arg Val Val Ser Val Leu Thr Val Leu His
   80 85 90
   Gln Asp Trp Leu Asn Gly Lys Glu Tyr Lys Cys Lys Val Ser Asn
   95 100 105
   Lys Ala Leu Pro Ala Pro Ile Glu Lys Thr Ile Ser Lys Ala Lys
   110 115 120
   Gly Gln Pro Arg Glu Pro Gln Val Tyr Thr Leu Pro Pro Ser Arg
   125 130 135
   Asp Glu Leu Thr Lys Asn Gln Val Ser Leu Thr Cys Leu Val Lys
   140 145 150
   Gly Phe Tyr Pro Ser Asp Ile Ala Val Glu Trp Glu Ser Asn Gly
   155 160 165
   Gln Pro Glu Asn Asn Tyr Lys Thr Thr Pro Pro Val Leu Asp Ser
   170 175 180
   Asp Gly Ser Phe Phe Leu Tyr Ser Lys Leu Thr Val Asp Lys Ser
   185 190 195
   Arg Trp Gln Gln Gly Asn Val Phe Ser Cys Ser Val Met His Glu
   200 205 210
   Ala Leu His Asn His Tyr Thr Gln Lys Ser Leu Ser Leu Ser Pro
   215 220 225
   Gly Lys
<210> 12
   <211> 227
   <212> PRT
   <213> Artificial sequence
<220>
   <223> sequence is synthesized
<400> 12
   Asp Lys Thr His Thr Cys Pro Pro Cys Pro Ala Pro Glu Leu Leu
   1               5                   10                  15
   Gly Gly Pro Ser Val Phe Leu Phe Pro Pro Lys Pro Lys Asp Thr
                   20                  25                  30
   Leu Met Ile Ser Arg Thr Pro Glu Val Thr Cys Val Val Val Asp
                   35                  40                  45
   Val Ser His Glu Asp Pro Glu Val Lys Phe Asn Trp Tyr Val Asp
                   50                  55                  60
   Gly Val Glu Val His Asn Ala Lys Thr Lys Pro Arg Glu Glu Gln
                   65                  70                  75
   Tyr Asn Ser Thr Tyr Arg Val Val Ser Val Leu Thr Val Leu His
                   80                  85                  90
   Gln Asp Trp Leu Asn Gly Lys Glu Tyr Lys Cys Lys Val Ser Asn
                   95                  100                 105
   Lys Ala Leu Pro Ala Pro Ile Glu Lys Thr Ile Ser Lys Ala Lys
                   110                 115                 120
   Gly Gln Pro Arg Glu Pro Gln Val Tyr Thr Leu Pro Pro Ser Arg
                   125                 130                 135
   Glu Glu Met Thr Lys Asn Gln Val Ser Leu Thr Cys Leu Val Lys
                   140                 145                 150
   Gly Phe Tyr Pro Ser Asp Ile Ala Val Glu Trp Glu Ser Asn Gly
                   155                 160                 165
   Gln Pro Glu Asn Asn Tyr Lys Thr Thr Pro Pro Val Leu Asp Ser
                   170                 175                 180
   Asp Gly Ser Phe Phe Leu Tyr Ser Lys Leu Thr Val Asp Lys Ser
                   185                 190                 195
   Arg Trp Gln Gln Gly Asn Val Phe Ser Cys Ser Val Met His Glu
                   200                 205                 210
   Ala Leu His Asn His Tyr Thr Gln Lys Ser Leu Ser Leu Ser Pro
                   215                 220                 225
   Gly Lys
<210> 13
   <211> 226
   <212> PRT
   <213> Artificial sequence
<220>
   <223> sequence is synthesized
<400> 13
   Asp Lys Thr His Thr Cys Pro Pro Cys Pro Ala Pro Glu Leu Leu
   1               5                   10                  15
   Gly Gly Pro Ser Val Phe Leu Phe Pro Pro Lys Pro Lys Asp Thr
                   20                  25                  30
   Leu Met Ile Ser Arg Thr Pro Glu Val Thr Cys Val Val Val Asp
                   35                  40                  45
   Val Ser His Glu Asp Pro Glu Val Lys Phe Asn Trp Tyr Val Asp
                   50                  55                  60
   Gly Val Glu Val His Asn Ala Lys Thr Lys Pro Arg Glu Glu Gln
                   65                  70                  75
   Tyr Asn Ser Thr Tyr Arg Val Val Ser Val Leu Thr Val Leu His
                   80                  85                  90
   Gln Asp Trp Leu Asn Gly Lys Glu Tyr Lys Cys Lys Val Ser Asn
                   95                  100                 105
   Lys Ala Leu Pro Ala Pro Ile Glu Lys Thr Ile Ser Lys Ala Lys
                   110                 115                 120
   Gly Gln Pro Arg Glu Pro Gln Val Tyr Thr Leu Pro Pro Ser Arg
                   125                 130                 135
   Asp Glu Leu Thr Lys Asn Gln Val Ser Leu Thr Cys Leu Val Lys
                   140                 145                 150
   Gly Phe Tyr Pro Ser Asp Ile Ala Val Glu Trp Glu Ser Asn Gly
                   155                 160                 165
   Gln Pro Glu Asn Asn Tyr Lys Thr Thr Pro Pro Val Leu Asp Ser
                   170                 175                 180
   Asp Gly Ser Phe Phe Leu Tyr Ser Lys Leu Thr Val Asp Lys Ser
                   185                 190                 195
   Arg Trp Gln Gln Gly Asn Val Phe Ser Cys Ser Val Met His Glu
                   200                 205                 210
   Ala Leu His Asn His Tyr Thr Gln Lys Ser Leu Ser Leu Ser Pro
                   215                 220                 225
   Gly
<210> 14
   <211> 226
   <212> PRT
   <213> Artificial sequence
<220>
   <223> sequence is synthesized
<400> 14
   Asp Lys Thr His Thr Cys Pro Pro Cys Pro Ala Pro Glu Leu Leu
   1               5                   10                  15
   Gly Gly Pro Ser Val Phe Leu Phe Pro Pro Lys Pro Lys Asp Thr
               20                  25                  30
   Leu Met Ile Ser Arg Thr Pro Glu Val Thr Cys Val Val Val Asp
           35                  40                  45
   Val Ser His Glu Asp Pro Glu Val Lys Phe Asn Trp Tyr Val Asp
       50                  55                  60
   Gly Val Glu Val His Asn Ala Lys Thr Lys Pro Arg Glu Glu Gln
   65                  70                  75
   Tyr Asn Ser Thr Tyr Arg Val Val Ser Val Leu Thr Val Leu His
                   80                  85                  90
   Gln Asp Trp Leu Asn Gly Lys Glu Tyr Lys Cys Lys Val Ser Asn
               95                  100                 105
   Lys Ala Leu Pro Ala Pro Ile Glu Lys Thr Ile Ser Lys Ala Lys
               110                 115                 120
   Gly Gln Pro Arg Glu Pro Gln Val Tyr Thr Leu Pro Pro Ser Arg
               125                 130                 135
   Glu Glu Met Thr Lys Asn Gln Val Ser Leu Thr Cys Leu Val Lys
               140                 145                 150
   Gly Phe Tyr Pro Ser Asp Ile Ala Val Glu Trp Glu Ser Asn Gly
               155                 160                 165
   Gln Pro Glu Asn Asn Tyr Lys Thr Thr Pro Pro Val Leu Asp Ser
               170                 175                 180
   Asp Gly Ser Phe Phe Leu Tyr Ser Lys Leu Thr Val Asp Lys Ser
               185                 190                 195
   Arg Trp Gln Gln Gly Asn Val Phe Ser Cys Ser Val Met His Glu
               200                 205                 210
   Ala Leu His Asn His Tyr Thr Gln Lys Ser Leu Ser Leu Ser Pro
               215                 220                 225
   Gly
<210> 15
   <211> 225
   <212> PRT
   <213> Artificial sequence
<220>
   <223> sequence is synthesized
<400> 15
   Cys Cys Val Glu Cys Pro Pro Cys Pro Ala Pro Pro Val Ala Gly
   1               5                   10                  15
   Pro Ser Val Phe Leu Phe Pro Pro Lys Pro Lys Asp Thr Leu Met
                   20                  25                  30
   Ile Ser Arg Thr Pro Glu Val Thr Cys Val Val Val Asp Val Ser
                   35                  40                  45
   His Glu Asp Pro Glu Val Gln Phe Asn Trp Tyr Val Asp Gly Val
                   50                  55                  60
   Glu Val His Asn Ala Lys Thr Lys Pro Arg Glu Glu Gln Phe Asn
                   65                  70                  75
   Ser Thr Phe Arg Val Val Ser Val Leu Thr Val Val His Gln Asp
                   80                  85                  90
   Trp Leu Asn Gly Lys Glu Tyr Lys Cys Lys Val Ser Asn Lys Gly
                   95                  100                 105
   Leu Pro Ala Pro Ile Glu Lys Thr Ile Ser Lys Thr Lys Gly Gln
                   110                 115                 120
   Pro Arg Glu Pro Gln Val Tyr Thr Leu Pro Pro Ser Arg Glu Glu
                   125                 130                 135
   Met Thr Lys Asn Gln Val Ser Leu Thr Cys Leu Val Lys Gly Phe
                   140                 145                 150
   Tyr Pro Ser Asp Ile Ala Val Glu Trp Glu Ser Asn Gly Gln Pro
                   155                 160                 165
   Glu Asn Asn Tyr Lys Thr Thr Pro Pro Met Leu Asp Ser Asp Gly
                   170                 175                 180
   Ser Phe Phe Leu Tyr Ser Lys Leu Thr Val Asp Lys Ser Arg Trp
                   185                 190                 195
   Gln Gln Gly Asn Val Phe Ser Cys Ser Val Met His Glu Ala Leu
                   200                 205                 210
   His Asn His Tyr Thr Gln Lys Ser Leu Ser Leu Ser Pro Gly Lys
                   215                 220                 225
<210> 16
   <211> 274
   <212> PRT
   <213> Artificial sequence
<220>
   <223> sequence is synthesized
<400> 16
   Leu Gly Asp Thr Thr His Thr Cys Pro Arg Cys Pro Glu Pro Lys
   1               5                   10                  15
   Ser Cys Asp Thr Pro Pro Pro Cys Pro Arg Cys Pro Glu Pro Lys
                   20                  25                  30
   Ser Cys Asp Thr Pro Pro Pro Cys Pro Arg Cys Pro Glu Pro Lys
                   35                  40                  45
   Ser Cys Asp Thr Pro Pro Pro Cys Pro Arg Cys Pro Ala Pro Glu
                   50                  55                  60
   Leu Leu Gly Gly Pro Ser Val Phe Leu Phe Pro Pro Lys Pro Lys
                   65                  70                  75
   Asp Thr Leu Met Ile Ser Arg Thr Pro Glu Val Thr Cys Val Val
                   80                  85                  90
   Val Asp Val Ser His Glu Asp Pro Glu Val Gln Phe Lys Trp Tyr
                   95                  100                 105
   Val Asp Gly Val Glu Val His Asn Ala Lys Thr Lys Pro Arg Glu
                   110                 115                 120
   Glu Gln Tyr Asn Ser Thr Phe Arg Val Val Ser Val Leu Thr Val
                   125                 130                 135
   Leu His Gln Asp Trp Leu Asn Gly Lys Glu Tyr Lys Cys Lys Val
                   140                 145                 150
   Ser Asn Lys Ala Leu Pro Ala Pro Ile Glu Lys Thr Ile Ser Lys
                   155                 160                 165
   Thr Lys Gly Gln Pro Arg Glu Pro Gln Val Tyr Thr Leu Pro Pro
                   170                 175                 180
   Ser Arg Glu Glu Met Thr Lys Asn Gln Val Ser Leu Thr Cys Leu
                   185                 190                 195
   Val Lys Gly Phe Tyr Pro Ser Asp Ile Ala Val Glu Trp Glu Ser
                   200                 205                 210
   Ser Gly Gln Pro Glu Asn Asn Tyr Lys Thr Thr Pro Pro Met Leu
                   215                 220                 225
   Asp Ser Asp Gly Ser Phe Phe Leu Tyr Ser Lys Leu Thr Val Asp
                   230                 235                 240
   Lys Ser Arg Trp Gln Gln Gly Asn Ile Phe Ser Cys Ser Val Met
                   245                 250                 255
   His Glu Ala Leu His Asn Arg Phe Thr Gln Lys Ser Leu Ser Leu
                   260                 265                 270
   Ser Pro Gly Lys
<210> 17
   <211> 224
   <212> PRT
   <213> Artificial sequence
<220>
   <223> sequence is synthesized
<400> 17
   Pro Pro Cys Pro Ser Cys Pro Ala Pro Glu Phe Leu Gly Gly Pro
   1               5                   10                  15
   Ser Val Phe Leu Phe Pro Pro Lys Pro Lys Asp Thr Leu Met Ile
                   20                  25                  30
   Ser Arg Thr Pro Glu Val Thr Cys Val Val Val Asp Val Ser Gln
                   35                  40                  45
   Glu Asp Pro Glu Val Gln Phe Asn Trp Tyr Val Asp Gly Val Glu
                   50                  55                  60
   Val His Asn Ala Lys Thr Lys Pro Arg Glu Glu Gln Phe Asn Ser
                   65                  70                  75
   Thr Tyr Arg Val Val Ser Val Leu Thr Val Leu His Gln Asp Trp
                   80                  85                  90
   Leu Asn Gly Lys Glu Tyr Lys Cys Lys Val Ser Asn Lys Gly Leu
                   95                  100                 105
   Pro Ser Ser Ile Glu Lys Thr Ile Ser Lys Ala Lys Gly Gln Pro
                   110                 115                 120
   Arg Glu Pro Gln Val Tyr Thr Leu Pro Pro Ser Gln Glu Glu Met
                   125                 130                 135
   Thr Lys Asn Gln Val Ser Leu Thr Cys Leu Val Lys Gly Phe Tyr
                   140                 145                 150
   Pro Ser Asp Ile Ala Val Glu Trp Glu Ser Asn Gly Gln Pro Glu
                   155                 160                 165
   Asn Asn Tyr Lys Thr Thr Pro Pro Val Leu Asp Ser Asp Gly Ser
                   170                 175                 180
   Phe Phe Leu Tyr Ser Arg Leu Thr Val Asp Lys Ser Arg Trp Gln
                   185                 190                 195
   Glu Gly Asn Val Phe Ser Cys Ser Val Met His Glu Ala Leu His
                   200                 205                 210
   Asn His Tyr Thr Gln Lys Ser Leu Ser Leu Ser Leu Gly Lys
                   215                 220